# EUROPEAN PATENT APPLICATION

(11) **EP 4 759 373 A2**
(43) Date of publication of application: **17.06.2026**
(21) Application number: 26174603.6
(22) Date of filing: 10.06.2022
(51) Int. Cl.: A61P 35/00

(54) **ANTI-UPAR ANTIBODIES AND USES THEREOF**

(30) Priority: 11.06.2021 US 202163209915 P
(62) Divisional of application: 22821080.3
(71) Applicant: Memorial Sloan-Kettering Cancer Center, New York, NY 10065 (US); Sloan-Kettering Institute for Cancer Research, New York, NY 10065 (US); Memorial Hospital for Cancer and Allied Diseases, New York, NY 10065 (US); Tri-institutional Therapeutics Discovery Institute, Inc., New York, NY 10021 (US)
(72) Inventor: LOWE, Scott, W, New York, NY 10065 (US); SADELAIN, Michel, New York, NY 10024 (US); AMOR VEGAS, Corina, New York, NY 11724 (US); BALDERES, Paul, New York, NY 10021 (US); LORENZ, Ivo, C., New York, NY 10021 (US)
(74) Representative: Barker Brettell LLP

(57) **Abstract**

The presently disclosed subject matter provides antibodies or antigen-binding fragments thereof that bind to uPAR and methods of using such antibodies or antigen-binding fragments thereof same.

## Description

### CROSS-REFERENCE TO RELATED APPLICATIONS

This application claims priority to U.S. Provisional Application No.: 63/209,915, filed June 11, 2021, the content of which is incorporated by reference in its entirety, and to which priority is claimed.

### SEQUENCE LISTING

This application contains a Sequence Listing which has been submitted in ASCII format via EFS-Web and is hereby incorporated by reference in its entirety. Said ASCII copy, created on June 10, 2022, is named 072734.1359_ST25.txt and is 84,771 bytes in size.

### 1. FIELD OF THE INVENTION

The presently disclosed subject matter relates to antibodies that bind to uPAR, and methods of using such antibodies.

### 2. BACKGROUND OF THE INVENTION

uPAR is associated with tumor growth or metastasis in various different types of cancers, including breast cancer, endometrial cancer, ovarian cancer, colon cancer, rectal cancer, lung cancer, stomach cancer, prostate cancer, renal cancer, pancreatic cancer, rectal cancer, acute lymphoblastic leukemia (ALL), chronic lymphocytic leukemia (CLL), and acute myeloid leukemia (AML). It also plays a role in aging, such as its association with senescence-related diseases associated with aging. It can also regulate immune response and cell-matrix interaction and promote tumor cell proliferation and emergence from dormancy.

Given the significant role for uPAR in various diseases or disorders, antibodies that recognize uPAR, and methods of using such agents, are desired.

### 3. SUMMARY OF THE INVENTION

The presently disclosed subject matter provides antibodies or antigen-binding fragments thereof that specifically bind to uPAR, and methods of using the antibodies or antigen-binding fragments thereof.

In certain embodiments, the uPAR antibody or an antigen-binding fragment thereof comprises a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 7. SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108.

In certain embodiments, the anti-uPAR antibody or an antigen-binding fragment thereof comprises a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

In certain embodiments, the anti-uPAR antibody or an antigen-binding fragment thereof comprises (a) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69. SEQ ID NO: 78, SEQ ID NO: 86. SEQ ID NO: 95, SEQ ID NO: 101, or SEQ ID NO: 108; and (b) a light chain variable region comprising an amino acid sequence that is at least about 80%, at least about 85%, at least about 90%, at least about 95%, at least about 96%, at least about 97%, at least about 98%, at least about 99% at least about 100% homologous or identical to the amino acid sequence set forth in SEQ ID NO: 8, SEQ ID NO: 16, SEQ ID NO: 24, SEQ ID NO: 32, SEQ ID NO: 42. SEQ ID NO: 51, SEQ ID NO: 60, SEQ ID NO: 70, SEQ ID NO: 79, SEQ ID NO: 87, SEQ ID NO: 96, or SEQ ID NO: 102.

In certain embodiments, the heavy chain variable region and the light chain variable region of the anti-uPAR antibody or antigen-binding fragment thereof are selected from the group consisting of:
(a) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 7, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 8;
(b) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 15, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 16;
(c) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 23, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 24;
(d) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 31, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 32;
(e) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 41, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 42;
(f) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 50, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 51;
(g) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 59, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 60;
(h) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 69, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 70;
(i) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 78, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 79;
(j) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 86, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 87.
(k) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 95, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 96; and
(l) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 101, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 102.

In certain embodiments, the anti-uPAR antibody or antigen-binding fragment thereof comprises a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108. In certain embodiments, the anti-uPAR antibody or antigen-binding fragment thereof, comprises a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

In certain embodiments, the anti-uPAR antibody or antigen-binding fragment thereof comprises a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108; and a light chain variable region comprising the amino acid sequence set forth in SEQ ID NO: 8. SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

In certain embodiments, the anti-uPAR antibody or antigen-binding fragment thereof comprises a heavy chain variable region and a light chain variable region, wherein the heavy chain variable region and the light chain variable region are selected from the group consisting of:
(a) a heavy chain variable region comprising the amino acid sequence set forth in SEQ ID NO: 7, and a light chain variable region comprising the amino acid sequence set forth in SEQ ID NO: 8;
(b) a heavy chain variable region comprising the amino acid sequence set forth in SEQ ID NO: 15, and a light chain variable region comprising the amino acid sequence set forth in SEQ ID NO: 16;
(c) a heavy chain variable region comprising the amino acid sequence set forth in SEQ ID NO: 23, and a light chain variable region comprising the amino acid sequence set forth in SEQ ID NO: 24;
(d) a heavy chain variable region comprising the amino acid sequence set forth in SEQ ID NO: 31, and a light chain variable region comprising the amino acid sequence set forth in SEQ ID NO: 32;
(e) a heavy chain variable region comprising the amino acid sequence set forth in SEQ ID NO: 41, and a light chain variable region comprising the amino acid sequence set forth in SEQ ID NO: 42;
(f) a heavy chain variable region comprising the amino acid sequence set forth in SEQ ID NO: 50, and a light chain variable region comprising the amino acid sequence set forth in SEQ ID NO: 51;
(g) a heavy chain variable region comprising the amino acid sequence set forth in SEQ ID NO: 59, and a light chain variable region comprising the amino acid sequence set forth in SEQ ID NO: 60;
(h) a heavy chain variable region comprising the amino acid sequence set forth in SEQ ID NO: 69, and a light chain variable region comprising the amino acid sequence set forth in SEQ ID NO: 70;
(i) a heavy chain variable region comprising the amino acid sequence set forth in SEQ ID NO: 78, and a light chain variable region comprising the amino acid sequence set forth in SEQ ID NO: 79;
(j) a heavy chain variable region comprising the amino acid sequence set forth in SEQ ID NO: 86, and a light chain variable region comprising the amino acid sequence set forth in SEQ ID NO: 87;
(k) a heavy chain variable region comprising the amino acid sequence set forth in SEQ ID NO: 95, and a light chain variable region comprising the amino acid sequence set forth in SEQ ID NO: 96; and
(l) a heavy chain variable region comprising the amino acid sequence set forth in SEQ ID NO: 101, and a light chain variable region comprising the amino acid sequence set forth in SEQ ID NO: 102.

In certain embodiments, the anti-uPAR antibody or antigen-binding fragment thereof comprises a heavy chain variable region that comprises CDR1, CDR2, and CDR3 domains; and a light chain variable region that comprises CDR1, CDR2, and CDR3 domains, wherein the heavy chain variable region and light chain variable region CDR3 domains are selected from the group consisting of:
(a) a heavy chain variable region CDR3 comprising the amino acid sequence set forth in SEQ ID NO: 3 and a conservative modification thereof; and a light chain variable region CDR3 comprising the amino acid sequence set forth in SEQ ID NO: 6 and a conservative modification thereof;
(b) a heavy chain variable region CDR3 comprising the amino acid sequence set forth in SEQ ID NO: 12 and a conservative modification thereof; and a light chain variable region CDR3 comprising the amino acid sequence set forth in SEQ ID NO: 14 and a conservative modification thereof;
(c) a heavy chain variable region CDR3 comprising the amino acid sequence set forth in SEQ ID NO: 20 and a conservative modification thereof; and a light chain variable region CDR3 comprising the amino acid sequence set forth in SEQ ID NO: 22 and a conservative modification thereof;
(d) a heavy chain variable region CDR3 comprising the amino acid sequence set forth in SEQ ID NO: 28 and a conservative modification thereof; and a light chain variable region CDR3 comprising the amino acid sequence set forth in SEQ ID NO: 30 and a conservative modification thereof;
(e) a heavy chain variable region CDR3 comprising the amino acid sequence set forth in SEQ ID NO: 37 and a conservative modification thereof; and a light chain variable region CDR3 comprising the amino acid sequence set forth in SEQ ID NO: 40 and a conservative modification thereof;
(f) a heavy chain variable region CDR3 comprising the amino acid sequence set forth in SEQ ID NO: 47 and a conservative modification thereof; and a light chain variable region CDR3 comprising the amino acid sequence set forth in SEQ ID NO: 49 and a conservative modification thereof;
(g) a heavy chain variable region CDR3 comprising the amino acid sequence set forth in SEQ ID NO: 56 and a conservative modification thereof; and a light chain variable region CDR3 comprising the amino acid sequence set forth in SEQ ID NO: 58 and a conservative modification thereof;
(h) a heavy chain variable region CDR3 comprising the amino acid sequence set forth in SEQ ID NO: 65 and a conservative modification thereof; and a light chain variable region CDR3 comprising the amino acid sequence set forth in SEQ ID NO: 68 and a conservative modification thereof;
(i) a heavy chain variable region CDR3 comprising the amino acid sequence set forth in SEQ ID NO: 75 and a conservative modification thereof; and a light chain variable region CDR3 comprising the amino acid sequence set forth in SEQ ID NO: 77 and a conservative modification thereof;
(j) a heavy chain variable region CDR3 comprising the amino acid sequence set forth in SEQ ID NO: 83 and a conservative modification thereof; and a light chain variable region CDR3 comprising the amino acid sequence set forth in SEQ ID NO: 85 and a conservative modification thereof;
(k) a heavy chain variable region CDR3 comprising the amino acid sequence set forth in SEQ ID NO: 92 and a conservative modification thereof; and a light chain variable region CDR3 comprising the amino acid sequence set forth in SEQ ID NO: 94 and a conservative modification thereof; and
(l) a heavy chain variable region CDR3 comprising the amino acid sequence set forth in SEQ ID NO: 99 and a conservative modification thereof; and a light chain variable region CDR3 comprising the amino acid sequence set forth in SEQ ID NO: 100 and a conservative modification thereof.

In certain embodiments, the heavy chain variable region and light chain variable region CDR2 domains of the antibody or antigen-binding fragment thereof are selected from the group consisting of:
(a) a heavy chain variable region CDR2 comprising the amino acid sequence set forth in SEQ ID NO: 2 and a conservative modification thereof; and a light chain variable region CDR2 comprising the amino acid sequence set forth in SEQ ID NO: 5 and a conservative modification thereof;
(b) a heavy chain variable region CDR2 comprising the amino acid sequence set forth in SEQ ID NO: 27 and a conservative modification thereof; and a light chain variable region CDR2 comprising the amino acid sequence set forth in SEQ ID NO: 5 and a conservative modification thereof;
(c) a heavy chain variable region CDR2 comprising the amino acid sequence set forth in SEQ ID NO: 36 and a conservative modification thereof; and a light chain variable region CDR2 comprising the amino acid sequence set forth in SEQ ID NO: 39 and a conservative modification thereof;
(d) a heavy chain variable region CDR2 comprising the amino acid sequence set forth in SEQ ID NO: 46 and a conservative modification thereof; and a light chain variable region CDR2 comprising the amino acid sequence set forth in SEQ ID NO: 5 and a conservative modification thereof;
(e) a heavy chain variable region CDR2 comprising the amino acid sequence set forth in SEQ ID NO: 55 and a conservative modification thereof; and a light chain variable region CDR2 comprising the amino acid sequence set forth in SEQ ID NO: 5 and a conservative modification thereof;
(f) a heavy chain variable region CDR2 comprising the amino acid sequence set forth in SEQ ID NO: 64 and a conservative modification thereof; and a light chain variable region CDR2 comprising the amino acid sequence set forth in SEQ ID NO: 67 and a conservative modification thereof;
(g) a heavy chain variable region CDR2 comprising the amino acid sequence set forth in SEQ ID NO: 74 and a conservative modification thereof; and a light chain variable region CDR2 comprising the amino acid sequence set forth in SEQ ID NO: 67 and a conservative modification thereof;
(h) a heavy chain variable region CDR2 comprising the amino acid sequence set forth in SEQ ID NO: 82 and a conservative modification thereof; and a light chain variable region CDR2 comprising the amino acid sequence set forth in SEQ ID NO: 67 and a conservative modification thereof;
(i) a heavy chain variable region CDR2 comprising the amino acid sequence set forth in SEQ ID NO: 91 and a conservative modification thereof; and a light chain variable region CDR2 comprising the amino acid sequence set forth in SEQ ID NO: 67 and a conservative modification thereof; and

(j) a heavy chain variable region CDR2 comprising the amino acid sequence set forth in SEQ ID NO: 46 and a conservative modification thereof; and a light chain variable region CDR2 comprising the amino acid sequence set forth in SEQ ID NO: 67 and a conservative modification thereof.

In certain embodiments, the anti-uPAR heavy chain variable region and light chain variable region CDR1 domains of the antibody or antigen-binding fragment thereof are selected from the group consisting of:
(a) a heavy chain variable region CDR1 comprising the amino acid sequence set forth in SEQ ID NO: 1 and a conservative modification thereof; and a light chain variable region CDR1 comprising the amino acid sequence set forth in SEQ ID NO: 4 and a conservative modification thereof;
(b) a heavy chain variable region CDR1 comprising the amino acid sequence set forth in SEQ ID NO: 11 and a conservative modification thereof; and a light chain variable region CDR1 comprising the amino acid sequence set forth in SEQ ID NO: 13 and a conservative modification thereof;
(c) a heavy chain variable region CDR1 comprising the amino acid sequence set forth in SEQ ID NO: 19 and a conservative modification thereof; and a light chain variable region CDR1 comprising the amino acid sequence set forth in SEQ ID NO: 21 and a conservative modification thereof;
(d) a heavy chain variable region CDR1 comprising the amino acid sequence set forth in SEQ ID NO: 19 and a conservative modification thereof; and a light chain variable region CDR 1 comprising the amino acid sequence set forth in SEQ ID NO: 29 and a conservative modification thereof;
(e) a heavy chain variable region CDR1 comprising the amino acid sequence set forth in SEQ ID NO: 35 and a conservative modification thereof; and a light chain variable region CDR1 comprising the amino acid sequence set forth in SEQ ID NO: 38 and a conservative modification thereof;
(f) a heavy chain variable region CDR1 comprising the amino acid sequence set forth in SEQ ID NO: 45 and a conservative modification thereof; and a light chain variable region CDR1 comprising the amino acid sequence set forth in SEQ ID NO: 48 and a conservative modification thereof;
(g) a heavy chain variable region CDR1 comprising the amino acid sequence set forth in SEQ ID NO: 54 and a conservative modification thereof; and a light chain variable region CDR1 comprising the amino acid sequence set forth in SEQ ID NO: 57 and a conservative modification thereof;
(h) a heavy chain variable region CDR1 comprising the amino acid sequence set forth in SEQ ID NO: 63 and a conservative modification thereof; and a light chain variable region CDR1 comprising the amino acid sequence set forth in SEQ ID NO: 66 and a conservative modification thereof;
(i) a heavy chain variable region CDR1 comprising the amino acid sequence set forth in SEQ ID NO: 73 and a conservative modification thereof; and a light chain variable region CDR1 comprising the amino acid sequence set forth in SEQ ID NO: 76 and a conservative modification thereof;
(j) a heavy chain variable region CDR1 comprising the amino acid sequence set forth in SEQ ID NO: 45 and a conservative modification thereof; and a light chain variable region CDR1 comprising the amino acid sequence set forth in SEQ ID NO: 84 and a conservative modification thereof;
(k) a heavy chain variable region CDR1 comprising the amino acid sequence set forth in SEQ ID NO: 90 and a conservative modification thereof; and a light chain variable region CDR1 comprising the amino acid sequence set forth in SEQ ID NO: 93 and a conservative modification thereof; and
(l) a heavy chain variable region CDR1 comprising the amino acid sequence set forth in SEQ ID NO: 45 and a conservative modification thereof; and a light chain variable region CDR1 comprising the amino acid sequence set forth in SEQ ID NO: 66 and a conservative modification thereof.

In certain embodiments, one or more of the CDR sequences have up to about 5 amino acid substitutions. In certain embodiments, one or more of the CDR sequences have up to about 3 amino acid substitutions.

In certain embodiments, the anti-uPAR antibody or antigen-binding fragment thereof comprises a heavy chain variable region comprising:
(a) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3:
(b) a CDRI comprising the amino acid sequence set forth in SEQ ID NO: 11, a CDR2 comprising the amino acid sequence set forth in SEQ ID NO: 2, and a CDR3 comprising the amino acid sequence set forth in SEQ ID NO: 12;
(c) a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20;
(d) a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28;
(e) a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37;
(f) a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47;
(g) a CDR1 comprising the amino acid sequence set forth in SEQ ID NO: 54, a CDR2 comprising the amino acid sequence set forth in SEQ ID NO: 55, and a CDR3 comprising the amino acid sequence set forth in SEQ ID NO: 56;
(h) a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65;
(i) a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75;
(j) a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83;
(k) a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92;
(l) a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; or
(m) a CDR1 comprising the amino acid sequence set forth in SEQ ID NO: 105, a CDR2 comprising the amino acid sequence set forth in SEQ ID NO: 106, and a CDR3 comprising the amino acid sequence set forth in SEQ ID NO: 107.

In certain embodiments, the anti-uPAR antibody or antigen-binding fragment thereof comprises a light chain variable region comprising:
(a) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6;
(b) a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14;
(c) a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22;
(d) a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30:
(e) a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40;
(f) a CDR1 comprising the amino acid sequence set forth in SEQ ID NO: 48, a CDR2 comprising the amino acid sequence set forth in SEQ ID NO: 5. and a CDR3 comprising the amino acid sequence set forth in SEQ ID NO: 49;
(g) a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58;
(h) a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68;
(i) a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77;
(j) a CDR1 comprising the amino acid sequence set forth in SEQ ID NO: 84, a CDR2 comprising the amino acid sequence set forth in SEQ ID NO: 67, and a CDR3 comprising the amino acid sequence set forth in SEQ ID NO: 85;
(k) a CDR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94; or
(l) a CDRI comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.

In certain embodiments. the anti-uPAR antibody or antigen-binding fragment thereof comprises:
(a) a heavy chain variable region comprising a COR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3: and a light chain variable region comprising a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6;
(b) a heavy chain variable region comprising a CDR1 comprising the amino acid sequence set forth in SEQ ID NO: 11, a CDR2 comprising an amino acid sequence set forth in SEQ ID NO: 2, and a CDR3 comprising the amino acid sequence set forth in SEQ ID NO: 12; and a light chain variable region comprising a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14;
(c) a heavy chain variable region comprising a COR1 comprising the amino acid sequence set forth in SEQ ID NO: 10, a CDR2 comprising the amino acid sequence set forth in SEQ ID NO: 2, and a CDR3 comprising the amino acid sequence set forth in SEQ ID NO: 20; and a light chain variable region comprising a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22;
(d) a heavy chain variable region comprising a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28; and a light chain variable region comprising a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30;
(e) a heavy chain variable region comprising a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; and a light chain variable region comprising a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40;
(f) a heavy chain variable region comprising a CDR1 comprising the amino acid sequence set forth in SEQ ID NO: 45. a CDR2 comprising the amino acid sequence set forth in SEQ ID NO: 46, and a CDR3 comprising the amino acid sequence set forth in SEQ ID NO: 47: and a light chain variable region comprising a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49;
(g) a heavy chain variable region comprising a COR1 comprising the amino acid sequence set forth in SEQ ID NO: 54, a CDR2 comprising the amino acid sequence set forth in SEQ ID NO: 55, and a CDR3 comprising the amino acid sequence set forth in SEQ ID NO: 56; and a light chain variable region comprising a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58;
(h) a heavy chain variable region comprising a CDR1 comprising the amino acid sequence set forth in SEQ ID NO: 63. a CDR2 comprising the amino acid sequence set forth in SEQ ID NO: 64, and a CDR3 comprising the amino acid sequence set forth in SEQ ID NO: 65; and a light chain variable region comprising a CDR1 comprising the amino acid sequence set forth in SEQ ID NO: 66. a CDR2 comprising the amino acid sequence set forth in SEQ ID NO: 67, and a CDR3 comprising the amino acid sequence set forth in SEQ ID NO: 68;
(i) a heavy chain variable region comprising a COR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; and a light chain variable region comprising a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77;
(j) a heavy chain variable region comprising a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83; and a light chain variable region comprising a CDR1 comprising the amino acid sequence set forth in SEQ ID NO: 84, a CDR2 comprising the amino acid sequence set forth in SEQ ID NO: 67, and a CDR3 comprising the amino acid sequence set forth in SEQ ID NO: 85;
(k) a heavy chain variable region comprising a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92; and a light chain variable region comprising a CDR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94; or
(l) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; and a light chain variable region comprising a COR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.

In certain embodiments, the sequence of the antibody is in a light-heavy variable chain orientation (V_{L}-V_{H}). In certain embodiments, the antibody or antigen-binding fragment thereof comprises a human variable region framework region. In certain embodiments, the antibody or antigen-binding fragment thereof is a fully human or an antigen-binding fragment thereof. In certain embodiments, the antibody or antigen-binding fragment thereof is a chimeric antibody or an antigen-binding fragment thereof. In certain embodiments, the antibody or antigen-binding fragment thereof is a humanized antibody or an antigen-binding fragment thereof. In certain embodiments, the antigen-binding fragment of the antibody is a Fab, Fab', F(ab')₂, variable fragment (Fv) or a single chain variable fragment (scFv).

In certain embodiments, the antigen-binding fragment of the antibody or antigen-binding fragment thereof is an scFv. In certain embodiments, the scFv of the antibody or antigen-binding fragment thereof comprises the amino acid sequence set forth in SEQ ID NO: 9, SEQ ID NO: 17, SEQ ID NO: 25, SEQ ID NO: 33, SEQ ID NO: 43. SEQ ID NO: 52. SEQ ID NO: 61, SEQ ID NO: 71, SEQ ID NO: 80, SEQ ID NO: 88, SEQ ID NO: 97, or SEQ ID NO:103.

In addition, the presently disclosed subject matter provides antibodies or antigen-binding fragments thereof, which cross-compete for binding to uPAR with any of the above-described antibody or antigen-binding fragment thereof.

The presently disclosed subject matter further provides antibodies or antigen-binding fragments thereof, which binds to the same epitope region on uPAR with any of the above-described antibody or antigen-binding fragment thereof.

The presently disclosed subject matter also provides immunoconjugates comprising the antibody or antigen-binding fragment thereof disclosed herein, linked to a therapeutic agent. In certain embodiments, the therapeutic agent is a drug, a cytotoxin, or a radioactive isotope.

Furthermore, the presently disclosed subject matter provides multi-specific molecules comprising the antibody or antigen-binding fragment thereof disclosed herein, linked to one or more functional moieties. In certain embodiments, the one or more functional moieties have a different binding specificity than the antibody or antigen binding fragment thereof.

Additionally, the presently disclosed subject matter provides compositions comprising the antibody or antigen-binding fragment thereof disclosed herein, the immunoconjugate disclosed herein, or the multi-specific molecule disclosed herein. In certain embodiments, the composition is a pharmaceutical composition that further comprises a pharmaceutically acceptable carrier.

In addition, the presently disclosed subject matter provides nucleic acids encoding the antibody or antigen-binding fragment thereof disclosed herein, vectors comprising such nucleic acid molecules, and host cells comprising such vectors.

The presently disclosed subject matter provides methods for detecting uPAR in a cell, a tissue, or a blood sample. In certain embodiments, the method comprises: contacting a cell, a tissue. or a blood sample with the antibody or antigen-binding fragment thereof disclosed herein, wherein the antibody or antigen-binding fragment thereof comprises a detectable label: and determining the amount of the labeled antibody or antigen-binding fragment thereof bound to the cell, tissue, blood sample by measuring the amount of detectable label associated with the cell, tissue, or blood sample, wherein the amount of bound antibody or antigen-binding fragment thereof indicates the amount of uPAR in the cell, tissue, or blood sample.

Furthermore, the presently disclosed subject matter provides methods of treating or ameliorating a disease or disorder in a subject. In certain embodiments, the method comprises administering to the subject an antibody or antigen-binding fragment thereof, the immunoconjugate thereof, the multi-specific molecule, or the composition disclosed herein. In certain embodiments, the disease or disorder expresses uPAR. In certain embodiments, the disease or disorder is associated with overexpression of uPAR. In certain embodiments, the disease or disorder is selected from the group consisting of tumors, senescence-associated pathologies, and tissue decline associated with aging. In certain embodiments, the disease or disorder is selected from the group consisting of lung fibrosis, cardiac fibrosis, liver fibrosis, atherosclerosis, osteoarthritis, diabetes, chronic kidney disease, Alzheimer's disease, and Parkinson disease.

In certain embodiments, the disease or disorder is a senescence-associated pathology. In certain embodiments, the senescence-associated pathology is selected from the group consisting of lung fibrosis, atherosclerosis, Alzheimer's disease, diabetes, osteoarthritis, liver fibrosis, chronic kidney disease, cardiac fibrosis, and Parkinson's disease.

In certain embodiments, the disease or disorder is a tumor. In certain embodiments, the tumor is selected from the group consisting of breast cancer (including triple negative breast cancer), endometrial cancer, ovarian cancer, colon cancer, rectal cancer, lung cancer (e.g., non-small cell lung cancer), stomach cancer, prostate cancer, gastric cancer, renal cancer, pancreatic cancer, blood cancer, cervical cancer, head and neck cancer, liver cancer (e.g., cholangiocarcinoma, hepatocellular carcinoma, and fibrolamaellar hepatocellular carcinoma), urotherial cancer, melanoma, and brain cancer (including glioblastoma multiforme). In certain embodiments, the blood cancer is selected from the group consisting of acute lymphoblastic leukemia (ALL), chronic lymphocytic leukemia (CLL), acute myeloid leukemia (AML), myelofibrosis, polycythemia vera, myelodysplastic syndrome, erythroleukemia. In certain embodiments, the tumor is cancer. Furthermore, the presently disclosed subject matter provides methods of increasing production of an immune-activating cytokine in response to a tumor cell in a subject. In certain embodiments, the method comprises administering to the subject an antibody or antigen-binding fragment thereof, the immunoconjugate thereof, the multi-specific molecule thereof, or the composition thereof disclosed herein. In certain embodiments, the immune-activating cytokine is selected from the group consisting of granulocyte macrophage colony stimulating factor (GM-CSF), IFN-α, IFN-β, IFN-γ, TNF-α, IL-1. IL-2, IL-3, IL-6, IL-11, IL-7, IL-8, IL-12, IL-15, IL-21, interferon regulatory factor 7 (IRF7), CCL1, CCL2, CCL3, CCL5, CCL7, CCL8, CCL13, CCL16, CXCL1, CXCL3, CXCL5, CXCL9, CXCL10, and combinations thereof. In certain embodiments, the subject is a human.

Furthermore, the presently disclosed subject matter provides kits for treating or ameliorating a disease or disorder in a subject, and/or increasing production of an immune-activating cytokine in response to a tumor cell in a subject, comprising the antibody or antigen-binding fragment thereof, the immunoconjugate thereof, the multi-specific molecule thereof, or the composition disclosed herein. In certain embodiments, the kit further comprises written instructions for using the antibody or antigen-binding fragment thereof, the immunoconjugate thereof, the multi-specific molecule thereof, or the composition thereof disclosed herein for treating or ameliorating a disease or disorder in a subject, and/or increasing production of an immune-activating cytokine in response to a tumor cell in a subject.

### 4. DETAILED DESCRIPTION OF EMBODIMENTS OF THE INVENTION

The presently disclosed subject matter provides anti-uPAR antibodies. Non-limiting embodiments of the present disclosure are described by the present specification and Examples.

For purposes of clarity of disclosure and not by way of limitation, the detailed description is divided into the following subsections:
4.1. Definitions;
4.2. uPAR;
4.3. Anti-uPAR Antibodies;
4.4. Nucleic Acids encoding the Antibodies or Antigen-binding Fragments;
4.5. Pharmaceutical Compositions and Methods of Treatment;
4.6. Diagnostic and Prognostic Methods;
4.7. Kits;
4.8. Exemplary Embodiments.

### 4.1. Definitions

In the description that follows, certain conventions will be followed as regards the usage of terminology. Generally, terms used herein are intended to be interpreted consistently with the meaning of those terms as they are known to those of skill in the art.

An "antigen-binding protein" is a protein or polypeptide that comprises an antigen-binding region or antigen-binding fragment, that is, has a strong affinity to another molecule to which it binds. Antigen-binding proteins encompass antibodies, chimeric antigen receptors (CARs) and fusion proteins.

"Antibody" and "antibodies" as those terms are known in the art refer to antigen binding proteins of the immune system. The term "antibody" as referred to herein includes whole, full length antibodies having an antigen-binding region, and any fragment thereof in which the "antigen-binding fragment" or "antigen-binding region" is retained, or single chains, for example, single chain variable fragment (scFv), thereof. A naturally occurring "antibody" is a glycoprotein comprising at least two heavy (H) chains and two light (L) chains inter-connected by disulfide bonds. Each heavy chain is comprised of a heavy chain variable region (abbreviated herein as V_{H}) and a heavy chain constant (CH) region. The heavy chain constant region is comprised of three domains, CH1, CH2 and CH3. Each light chain is comprised of a light chain variable region (abbreviated herein as V_{L}) and a light chain constant C_{L} region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1 q) of the classical complement system.

The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the presently disclosed subject matter may include amino acid residues not encoded by human germline immunoglobulin sequences (e.g., mutations introduced by random or site-specific mutagenesis *in vitro* or by somatic mutation *in vivo*).

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the presently disclosed subject matter may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The term "recombinant human antibody", as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo*.

The term "humanized antibody" is intended to refer to antibodies in which CDR sequences derived from the germline of another mammalian species, such as a mouse, have been grafted onto human framework sequences. Additional framework region modifications may be made within the human framework sequences.

The term "chimeric antibody" is intended to refer to antibodies in which the variable region sequences are derived from one species and the constant region sequences are derived from another species, such as an antibody in which the variable region sequences are derived from a mouse antibody and the constant region sequences are derived from a human antibody.

As used herein, an antibody that "specifically binds to uPAR" is intended to refer to an antibody that binds to uPAR (e.g., human uPAR) with a dissociation constant (K_{D}) of about 5 × 10⁻⁷ M or less, about 1 × 10⁻⁷ M or less, about 5 × 10⁻⁸ M or less, about 1 × 10⁻⁸ M or less, about 5 × 10⁻⁹ M or less, about 1 × 10⁻⁹ M or less, about 5 x 10⁻¹⁰ M or less, about 1 x 10⁻¹⁰ M or less, about 5 x 10⁻¹¹ M or less, or about 1 × 10⁻¹¹ M or less.

An "antibody that competes for binding" or "antibody that cross-competes for binding" with a reference antibody for binding to an antigen, e.g., uPAR, refers to an antibody that blocks binding of the reference antibody to the antigen (e.g., uPAR) in a competition assay by 50% or more, and conversely, the reference antibody blocks binding of the antibody to the antigen (e.g., uPAR) in a competition assay by 50% or more. An exemplary competition assay is described in "Antibodies", Harlow and Lane (Cold Spring Harbor Press, Cold Spring Harbor, NY).

As used herein, "isotype" refers to the antibody class (e.g.. IgM or IgG1) that is encoded by the heavy chain constant region genes.

The phrases "an antibody recognizing an antigen" and "an antibody specific for an antigen" are used interchangeably herein with the term" an antibody which binds specifically to an antigen (e.g., a uPAR polypeptide)."

The term "antigen-binding fragment" or "antigen-binding region" of an antibody, as used herein, refers to that region or fragment of the antibody that binds to the antigen and which confers antigen specificity to the antibody; fragments of antigen-binding proteins, for example, antibodies includes one or more fragments of an antibody that retain the ability to specifically bind to an antigen (e.g., a uPAR polypeptide). It has been shown that the antigen-binding function of an antibody can be performed by fragments of a full-length antibody. Examples of antigen-binding fragments encompassed within the term "antibody fragments" of an antibody include a Fab fragment, a monovalent fragment consisting of the V_{L}, V_{H}, C_{L} and CH1 domains; a F(ab)₂ fragment, a bivalent fragment comprising two Fab fragments linked by a disulfide bridge at the hinge region; a Fd fragment consisting of the V_{H} and CH1 domains; a Fv fragment consisting of the V_{L} and V_{H} domains of a single arm of an antibody; a dAb fragment (Ward et al., Nature 1989;341:544-546), which consists of a V_{H} domain; and an isolated complementarity determining region (CDR).

Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules. These are known as single chain Fv (scFv); see e.g., Bird et al., Science (1988);242:423-426; and Huston et al., Proc Natl Acad Sci (1998);85:5879-5883. These antibody fragments are obtained using conventional techniques known to those of skill in the art, and the fragments are screened for utility in the same manner as are intact antibodies.

An "antibody" or "antigen-binding protein" is one which has been identified and separated and/or recovered from a component of its natural environment. "Synthetic antibodies" or "recombinant antibodies" are generally generated using recombinant technology or using peptide synthetic techniques known to those of skill in the art.

As used herein, the term "single-chain variable fragment" or "scFv" is a fusion protein of the variable regions of the heavy (V_{H}) and light chains (V_{L}) of an immunoglobulin (e.g., mouse or human) covalently linked to form a V_{H}::V_{L} heterodimer. The heavy (V_{H}) and light chains (V_{L}) are either joined directly or joined by a peptide-encoding linker (e.g., 10, 15, 20, 25 amino acids), which connects the N-terminus of the V_{H} with the C-terminus of the V_{L}, or the C-terminus of the V_{H} with the N-terminus of the V_{L}. The linker is usually rich in glycine for flexibility, as well as serine or threonine for solubility. The linker can link the heavy chain variable region and the light chain variable region of the extracellular antigen-binding domain.

Non-limiting examples of linkers are disclosed in Shen et al., Anal Chem (2008);80(6):1910-1917 and WO 2014/087010, the contents of which are hereby incorporated by reference in their entireties. In certain embodiments, the linker is a G4S linker. In certain embodiments, the linker comprises or consists of the amino acid sequence set forth in SEQ ID NO: 110, which is provided below:
GGGGSGGGGSGGGSGGGGS [SEQ ID NO; 110]

In certain embodiments, the linker comprises or consists of the amino acid sequence set forth in SEQ ID NO: 111, which is provided below:
GGGGSGGGGSGGGGS [SEQ ID NO: 111]

In certain embodiments, the linker comprises or consists of the amino acid sequence set forth in SEQ ID NO: 112, which is provided below:
GGGGSGGGGSGGGGSGGGSGGGGS [SEQ ID NO: 112]

In certain embodiments, the linker comprises or consists of the amino acid sequence set forth in SEQ ID NO: 113, which is provided below:
GGGGSGGGGSGGGGSGGGSGGGSGGGGS [SEQ ID NO: 113]

In certain embodiments, the linker comprises or consists of the amino acid sequence set forth in SEQ ID NO: 114, which is provided below:
GGGGS [SEC ID NO: 114]

In certain embodiments. the linker comprises or consists of the amino acid sequence set forth in SEQ ID NO: 115, which is provided below:
GGGGSGGGGS [SEQ ID NO: 115]

Despite removal of the constant regions and the introduction of a linker, scFv proteins retain the specificity of the original immunoglobulin. Single chain Fv polypeptide antibodies can be expressed from a nucleic acid comprising V_{H} - and V_{L} -encoding sequences as described by Huston, et al. (Proc. Nat. Acad. Sci. USA, 1988;85:5879-5883). See, also, U.S. Patent Nos. 5,091,513, 5,132,405 and 4,956,778: and U.S. Patent Publication Nos. 20050196754 and 20050196754. Antagonistic scFvs having inhibitory activity have been described (see, e.g., Zhao et al., Hyrbidoma (Larchmt) 2008:27(6):455-51; Peter et al., J Cachexia Sarcopenia Muscle 2012 August 12; Shieh et al., J Imunol 2009; 183(4):2277-85; Giomarelli et al., Thromb Haemost 2007;97(6):955-63; Fife eta., J Clin Invst 2006;116(8):2252-61; Brocks et al., Immunotechnology 1997:3(3):173-84; Moosmayer et al., Ther Immunol 1995; 2(10:31-40). Agonistic scFvs having stimulatory activity have been described (see, e.g., Peter et al., J Biol Chern 2003; 25278(38):36740-7; Xie et al., Nat Biotech 1997; 15(8):768-71; Ledbetter et al., Crit Rev Immunol 1997; 17(5-6):427-55; Ho et al., BioChim Bioplys Acta 2003; 1638(3):257-66).

As used herein, "F(ab)" refers to a fragment of an antibody structure that binds to an antigen but is monovalent and does not have a Fc portion, for example, an antibody digested by the enzyme papain yields two F(ab) fragments and an Fc fragment (e.g., a heavy (H) chain constant region: Fc region that does not bind to an antigen).

As used herein, "P(ab')₂" refers to an antibody fragment generated by pepsin digestion of whole IgG antibodies, wherein this fragment has two antigen binding (ab') (bivalent) regions, wherein each (ab') region comprises two separate amino acid chains, a part of a H chain and a light (L) chain linked by an S-S bond for binding an antigen and where the remaining H chain portion are linked together. A "P(ab')₂" fragment can be split into two individual Fab' fragments.

As used herein, the term "vector" refers to any genetic element, such as a plasmid, phage, transposon. cosmid, chromosome, virus, virion. etc., which is capable of replication when associated with the proper control elements and which can transfer gene sequences into cells. Thus, the term includes cloning and expression vehicles, as well as viral vectors and plasmid vectors.

"CDRs" are defined as the complementarity determining region amino acid sequences of an antibody which are the hypervariable regions of immunoglobulin heavy and light chains. See, e. g., Kabat et al., Sequences of Proteins of Immunological Interest, 4th U. S. Department of Health and Human Services, National Institutes of Health (1987), or IMGT numbering system (Lefranc, The Immunologist (1999);7:132-136; Lefranc et al.. Dev. Comp. Immumol. (2003); 27:55-77). The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise three heavy chain and three light chain CDRs or CDR regions in the variable region. CDRs provide the majority of contact residues for the binding of the antibody to the antigen or epitope region. In certain embodiments, the CDRs are identified according to the IMGT system. In certain embodiments, the CDRs are identified using the IMGT numbering system accessible at http://www.img.org/IMGT_vquest/input.

The terms "isolated" denotes a degree of separation from original source or surroundings.

An "isolated antibody" is one which has been separated from a component of its natural environment. In certain embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatrnan et al., J. Chromatogr (2007); B 848:79-87.

An "isolated nucleic acid" refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

An "isolated nucleic acid encoding an antibody" (including references to a specific antibody, e.g. an anti-KLB antibody) refers to one or more nucleic acid molecules encoding antibody heavy and light chains (or fragments thereof), including such nucleic acid molecule(s) in a single vector separate vectors, and such nucleic acid molecule(s) present at one or more locations in a host cell.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

An "immunoconjugate" is an antibody conjugated to one or more heterologous molecule(s), including, but not limited to. a cytotoxic agent.

An "effective amount" (or, "therapeutically effective amount") is an amount sufficient to effect a beneficial or desired clinical result upon treatment. An effective amount can be administered to a subject in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically taken into account when determining an appropriate dosage to achieve an effective amount. These factors include age, sex and weight of the subject. the condition being treated, the severity of the condition, and the form and effective concentration of the cells administered.

An "individual" or "subject" herein is a vertebrate, such as a human or non-human animal, for example, a mammal. Mammals include, but are not limited to, humans, primates, farm animals, sport animals, rodents and pets. Non-limiting examples of non-human animal subjects include rodents such as mice. rats, hamsters; guinea pigs; rabbits; dogs: cats; sheep; pigs; goats: cattle; horses; and non-human primates such as apes and monkeys.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In certain embodiments, antibodies of the presently disclosed subject matter are used to delay development of a disease or to slow the progression of a disease, e.g., a tumor, e.g., a tumor associated with uPAR.

The terms "comprises", "comprising", and are intended to have the broad meaning ascribed to them in U.S. Patent Law and can mean "includes", "including" and the like.

As used herein, the term "about" or "approximately" means within an acceptable error range for the particular value as determined by one of ordinary skill in the art, which will depend in part on how the value is measured or determined, *i.e.*, the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

As described herein, any concentration range, percentage range, ratio range or integer range is to be understood to include the value of any integer within the recited range and, when appropriate, fractions thereof(such as one tenth and one hundredth of an integer), unless otherwise indicated.

Other aspects of the presently disclosed subject matter are described in the following disclosure and are within the ambit of the presently disclosed subject matter.

### 4.2. uPAR

uPAR (urokinase-type plasminogen activator receptor), also known as CD87, is a glycosylphosphatidylinositol-anchored protein. uPAR is cysteine-rich and consists of three tandem LU domains, which bind urokinase-type plasminogen activator (uPA). (Kessler et al., J. Neurochem. (2017);142: 7-18; Llinas et al., EMBO J. (2005);24(9): 1655-63; Huai et al., Science (2006);311 (5761):656-9; Chelsea et al., Human Genomics (2016); 10:10). uPAR also interacts with several other proteins, including vitronectin, the uPAR associated protein (uPARAP) and the integrin family of membrane proteins.

uPAR is associated with tumor growth or metastasis in various different types of cancers, including breast cancer (including triple negative breast cancer), endometrial cancer, ovarian cancer, colon cancer, rectal cancer. lung cancer, stomach cancer, prostate cancer, renal cancer, pancreatic cancer, rectal cancer, cervical cancer, head and neck cancer, liver cancer, gastric cancer, urotherial cancer, melanoma, brain cancer (including glioblastoma multiforme), acute lymphoblastic leukemia (ALL), chronic lymphocytic leukemia (CLL), and acute myeloid leukemia (AML). It also plays a role in aging, such as its association with senescence-related diseases associated with aging. It can also regulate immune response and cell-matrix interaction and promote tumor cell proliferation and emergence from dormancy.

uPAR is induced during the process of cellular senescence, which can be elicited by certain cancer agents and accumulates in a range of age-related and tissue damage pathologies (LIST). Elimination of senescent cells can improve the response of therapy, and ameliorate symptoms of the tissue damage pathologies including fibrosis, etc.

Soluble urokinase plasminogen activator receptor (suPAR) is found upregulated in a number of pathologies noted above, also in chronic obstructive pulmonary disease, asthma, liver failure, heart failure, cardiovascular disease. and rheumatoid arthritis. (Desmedt et al., Crit. Rev. Clin. Lab. Sci. (2017);54(2): 117-133). uPAR is found to be highly expressed on senescent cells. (Wagner et al., Nature (2020);583 (7814): 37-38, Amor et al., Nature (2020 Jul);583(7814):127-132). Thus, uPAR (e.g., suPAR) can be used as a disease stage biomarker.

Many oncogenic signaling pathways and tumor microenvironmental conditions such as hypoxia can activate transcription factors that in turn regulate uPAR. uPAR can regulate proteolysis by associating with the outer layer of the plasma membrane by a glycosyl phosphatidylinositol (GPI) anchor, but it can also be secreted or shed from the cell surface. (Harvey et al., Nat. Rev. Mol. Cell Biol (2010); 11, 23-36). uPAR expression directly correlates with the invasive potential of endometrial carcinomas. (Foca et al.. Gynecol. Oncol. (2000);79(2):244-50). uPAR is implicated in several hematological malignancies, particularly acute leukemia and multiple myeloma. (Hata et al., Blood (1993): 81: 3357-3364; MC Bone et al., Leukemia (2004); 18, 394-400). uPAR is reported to be associated with poor prognosis in breast cancer patients. (Bo et al., Oncol. Rep. (2005); 14(1):105-12; Foekens et al., Cancer Res. (2000); 60(3): 636-43).

In certain embodiments, uPAR is human uPAR comprising or consisting of the amino acid sequence with a UniProt Reference No: Q03405-1 (SEQ ID NO: 116) or a fragment thereof. SEQ ID NO: 116 is provided below. In certain embodiments, the uPAR comprises three domains: domain 1 (domain UPAR/Ly6 1), domain 2 (domain UPAR/Ly6 2), and domain 3 (domain UPAR/Ly6 3). In certain embodiments, domain 1 comprises or consists of amino acids 23 to 114 of SEQ ID NO: 116. In certain embodiments, domain 2 comprises or consists of amino acids 115 to 213 of SEQ ID NO: 116. In certain embodiments, domain 3 comprises or consists of amino acids 214 to 305 of SEQ ID NO: 116.

MGRPPLLPLL LLLHTCVPAS WGLRCMQCKT NGDCRVEECA LGQDLCRTTI VRLWEEGEEL
ELVEKSCTHS EKTNRTLSYR TGLKITSLTE VVCGLDLCNQ GNSGRAVTYS RSRYLECISC
GSSDMSCERG RHQSLQCRSP EEQCLDVVTH WIQEGEEGRP KDDRHLRGCG YLPGCPGSNG
FHNNDTFHFL KCCNTTKCNE GPILELENLP QNGRQCYSCK GNSTHGCSSE ETFLIDCRGP
MNQCLVATGT HEPKNQSYMV RGCATASMCQ HAHLGDAFSM NHIDVSCCTK SGCNHPDLDV
QYRSGAAPQP GPAHLSLTIT LLMTARLWGG TLLWT [SEQ ID NO: 116]

In certain embodiments, the uPAR comprises or consists of an amino acid sequence that is at least about 80%, at least about 85%, at least about 90%, at least about 95%, at least about 96%, at least about 97%, at least about 98%, or at least about 99%, at least about 100% identical to the amino acid sequence set forth in SEQ ID NO: 116 or a fragment thereof.

In certain embodiments, the anti-uPAR antibodies or antigen-binding fragments thereof bind to a portion of human uPAR. In certain embodiments, the anti-uPAR antibodies or antigen-binding fragments thereof bind to at least one of domain 1, domain 2, and domain 3. In certain embodiments, the anti-uPAR antibodies or antigen-binding fragments thereof bind to domain 2. In certain embodiments, the anti-uPAR antibodies or antigen-binding fragments thereof bind to domain 3. In certain embodiments, the anti-uPAR antibodies or antigen-binding fragments thereof bind to both domain 2 and domain 3. In certain embodiments, the anti-uPAR antibodies or antigen-binding fragments thereof bind to amino acids 115 to 303 of SEQ ID NO: 116. In certain embodiments, the anti-uPAR antibodies or antigen-binding fragments thereof bind to amino acids 115 to 305 of SEQ ID NO: 116.

### 4.3. Anti-uPAR Antibodies

The antibodies of the presently disclosed subject matter are characterized by particular functional features or properties of the antibodies. For example, the antibodies bind specifically to uPAR (e.g., bind to human uPAR).

In certain embodiments. a presently disclosed antibody or antigen-binding fragment binds to uPAR (e.g., human uPAR) with a binding affinity, for example with a dissociation constant (K_{D}) of 1 × 10⁻⁶ M or less, e.g., about 1 × 10⁻⁷ M or less, about 1 × 10⁻⁸ M or less, about 1 × 10⁻⁹ M or less, about 1 × 10⁻¹⁰ M or less, or about 1 × 10⁻¹¹ M or less.

The heavy and light chains of a presently disclosed antibody or antigen-binding fragment can be full-length (e.g., an antibody can include at least one (e.g., one or two) complete heavy chains, and at least one (e.g., one or two) complete light chains) or can include an antigen-binding fragment (a Fab, F(ab')₂, Fv or a single chain Fv fragment ("scFv")). In certain embodiments, the antibody heavy chain constant region is chosen from. e.g., IgG1, IgG2, IgG3, IgG4, IgM, IgA1, IgA2, IgD, and IgE, particularly chosen from, e.g., IgG1, IgG2, IgG3, and IgG4. In certain embodiments, the immunoglobulin isotype is IgG1 (e.g., human IgG1). In certain embodiments, the antibody light chain constant region is chosen from, e.g., kappa or lambda, particularly kappa.

### 4.3.1. Single-Chain Variable Fragments (scFvs)

In certain embodiments, the presently disclosed subject matter includes antibodies or antigen-binding fragments thereof that have the scFv sequence fused to one or more constant domains to form an antibody with an Fc region of a human immunoglobulin to yield a bivalent protein, increasing the overall avidity and stability of the antibody. In addition, the Fc portion allows the direct conjugation of other molecules, including but not limited to fluorescent dyes, cytotoxins, radioisotopes etc. to the antibody for example, for use in antigen quantitation studies, to immobilize the antibody for affinity measurements, for targeted delivery of a therapeutic agent, to test for Fc-mediated cytotoxicity using immune effector cells and many other applications.

The results presented here highlight the specificity, sensitivity and utility of the presently disclosed antibodies or antigen-binding fragments in targeting a uPAR polypeptide (e.g., human uPAR).

The presently disclosed molecules are based on the identification and selection of single chain variable fragments (scFvs) using phage display, the amino acid sequence of which confers the molecules' specificity for a uPAR polypeptide of interest and forms the basis of all antigen binding proteins of the disclosure. The scFv, therefore, can be used to design a diverse array of "antibody" molecules, including, for example, full length antibodies, fragments thereof, such as Fab and F(ab')₂, minibodies, fusion proteins, including scFv-Fc fusions, multivalent antibodies, that is, antibodies that have more than one specificity for the same antigen or different antigens, for example, multi-specific antibodies, tribodies, etc. (*see* Cuesta et al., Multivalent antibodies: when design surpasses evolution. Trends in Biotechnology 28:355-362 2010).

In certain embodiments, the antigen-binding protein is a full length antibody, the heavy and light chains of an antibody of the presently disclosed subject matter can be full-length (*e.g*., an antibody can include at least one, or two, complete heavy chains, and at least one, and preferably two, complete light chains) or can include an antigen-binding fragment (a Fab, F(ab')₂, Fv or scFv). In certain embodiments, the antibody heavy chain constant region is chosen from IgG1, IgG2, IgG3, IgG4, IgM, IgA1, IgA2, IgD, and IgE. etc. In certain embodiments, the immunoglobulin isotype is selected from IgG1, IgG2, IgG3, and IgG4. In certain embodiments, the immunoglobulin isotype is IgG1 (e.g., human IgGl). The choice of antibody isotype can depend on the immune effector function that the antibody is designed to elicit.

In constructing a recombinant immunoglobulin, appropriate amino acid sequences for constant regions of various immunoglobulin isotypes and methods for the production of a wide array of antibodies are known to those of skill in the art.

In certain embodiments, the anti-uPAR scFv is an scFv-Fc fusion protein or a full-length human IgG with V_{H} and V_{L} regions or CDRs selected from Table 1. In certain embodiments, the anti-uPAR scFv comprises a V_{H} comprising the amino acid sequence set forth in SEQ ID NO: 7. In certain embodiments, the anti-uPAR scFv comprises a V_{L} comprising the amino acid sequence set forth in SEQ ID NO: 8. In certain embodiments, the scFv is designated as "3-C3-A".

In certain embodiments, the anti-uPAR scFv comprises a V_{H} comprising the amino acid sequence set forth in SEQ ID NO: 7 and a V_{L} comprising the amino acid sequence set forth in SEQ ID NO: 8. In certain embodiments, the anti-uPAR scFv comprises a V_{H} comprising a CDR1 comprising the amino acid sequence set forth in SEQ ID NO: 1 or a conservative modification thereof, a CDR2 comprising the amino acid sequence set forth in SEQ ID NO: 2 or a conservative modification thereof, and a CDR3 comprising the amino acid sequence set forth in SEQ ID NO: 3 or a conservative modification thereof. SEQ ID NOs: 1-3 are provided in Table 1.

In certain embodiments, the anti-uPAR scFv comprises a V_{L} comprising a CDR1 comprising the amino acid sequence set forth in SEQ ID NO: 4 or a conservative modification thereof, a CDR2 comprising the amino acid sequence set forth in SEQ ID NO: 5 or a conservative modification thereof, and a CDR3 comprising the amino acid sequence set forth in SEQ ID NO: 6 or a conservative modification thereof. SEQ ID NOs: 4-6 are provided in Table 1.

In certain embodiments, the anti-uPAR scFv comprises a V_{H} comprising a CDR1 comprising the amino acid sequence set forth in SEQ ID NO: 1 or a conservative modification thereof, a CDR2 comprising the amino acid sequence set forth in SEQ ID NO: 2 or a conservative modification thereof, and a CDR3 comprising the amino acid sequence set forth in SEQ ID NO: 3 or a conservative modification thereof; and a V_{L} comprising a CDR1 comprising the amino acid sequence set forth in SEQ ID NO: 4 or a conservative modification thereof, a CDR2 comprising the amino acid sequence set forth in SEQ ID NO: 5 or a conservative modification, and a CDR3 comprising the amino acid sequence set forth in SEQ ID NO: 6 or a conservative modification thereof.

In certain embodiments, the anti-uPAR scFv comprises a V_{H} comprising a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; and a V_{L} comprising a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6.

In certain embodiments, the anti-uPAR scFv comprises a V_{H} comprising the amino acid sequence set forth in SEQ ID NO: 7, and a V_{L} comprising the amino acid sequence set forth in SEQ ID NO: 8. In certain embodiments, the V_{H} and V_{L} are linked via a linker. In certain embodiments, the linker comprises the amino acid sequence set forth in SEQ ID NO: 110.

In certain embodiments, the variable regions are linked one after another such that a heavy chain variable region (V_{H}) is position at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, anti-uPAR scFv comprises the amino acid sequence set forth in SEQ ID NO: 9. An exemplary nucleotide sequence encoding the amino acid sequence of SEQ ID NO: 9 is set forth in SEQ ID NO: 10. SEQ ID NOS: 9 and 10 are provided in Table 1 below.

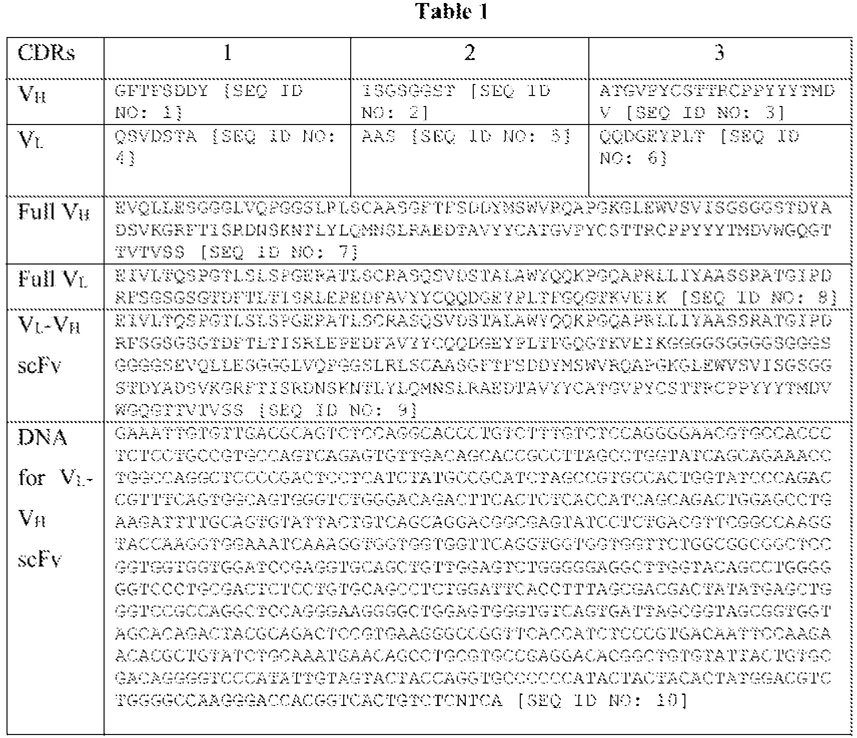

In certain embodiments, the anti-uPAR scFv comprises a V_{H} comprising the amino acid sequence set forth in SEQ ID NO: 15 and a V_{L} comprising the amino acid sequence set forth in SEQ ID NO: 16, optionally with a linker sequence, for example a linker peptide, between the heavy chain variable region and the light chain variable region. In certain embodiments, the linker comprises the amino acid sequence set forth in SEQ ID NO: 110. SEQ ID NOs: 15 and 16 are provided in Table 2 below. In certain embodiments, the scFv is designated as "3-D8-A."

In certain embodiments, the anti-uPAR scFv is an scFv-Fc fusion protein or a full-length human IgG with V_{H} and V_{L} regions or CDRs selected from Table 2. In certain embodiments, the anti-uPAR scFv comprises a V_{H} comprising the amino acid sequence set forth in SEQ ID NO: 15, as shown in Table 2. In certain embodiments, the anti-uPAR scFv comprises a V_{L} comprising the amino acid sequence set forth in SEQ ID NO: 16. In certain embodiments, the anti-uPAR scFv comprises a V_{H} comprising the amino acid sequence set forth in SEQ ID NO: 15 and a V_{L} comprising the amino acid sequence set forth in SEQ ID NO: 16.

In certain embodiments, the anti-uPAR scFv comprises a V_{H} comprising a CDR1 comprising the amino acid sequence set forth in SEQ ID NO: 11 or a conservative modification thereof, a CDR2 comprising the amino acid sequence set forth in SEQ ID NO: 2 or a conservative modification thereof, and a CDR3 comprising the amino acid sequence set forth in SEQ ID NO: 12 or a conservative modification thereof. SEQ ID NOs: 2, 11, and 12 are provided in Table 2.

In certain embodiments, the anti-uPAR scFv comprises a V_{L} comprising a CDR1 comprising the amino acid sequence set forth in SEQ ID NO: 13 or a conservative modification thereof, a CDR2 comprising the amino acid sequence set forth in SEQ ID NO: 5 or a conservative modification thereof, and a CDR3 comprising the amino acid sequence set forth in SEQ ID NO: 14 or a conservative modification thereof. SEQ ID NOs: 5, 13, and 14 are provided in Table 2.

In certain embodiments, the anti-uPAR scFv comprises a V_{H} comprising a CDR1 comprising the amino acid sequence set forth in SEQ ID NO: 11 or a conservative modification thereof, a CDR2 comprising the amino acid sequence set forth in SEQ ID NO: 2 or a conservative modification thereof, and a CDR3 comprising the amino acid sequence set forth in SEQ ID NO: 12 or a conservative modification thereof; and a V_{L} comprising CDR 1 comprising the amino acid sequence set forth in SEQ ID NO: 13 or a conservative modification thereof, a CDR2 comprising the amino acid sequence set forth in SEQ ID NO: 5 or a conservative modification thereof, and a CDR3 comprising the amino acid sequence set forth in SEQ ID NO: 14 or a conservative modification.

In certain embodiments, the anti-uPAR scFv comprises a V_{H} comprising a CDR1 comprising the amino acid sequence set forth in SEQ ID NO: 11, a CDR2 comprising the amino acid sequence set forth in SEQ ID NO: 2, and a CDR3 comprising the amino acid sequence set forth in SEQ ID NO: 12; and a V_{L} comprising a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14.

In certain embodiments, the anti-uPAR scFv comprises a V_{H} comprising the amino acid sequence set forth in SEQ ID NO: 15, and a V_{L} comprising the amino acid sequence set forth in SEQ ID NO: 16. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, the anti-uPAR scFv comprises the amino acid sequence set forth in SEQ ID NO: 17. An exemplary nucleotide sequence encoding the amino acid sequence of SEQ ID NO: 17 is set forth in SEQ ID NO: 18. SEQ ID NOS: 17 and 18 are provided in Table 2 below.

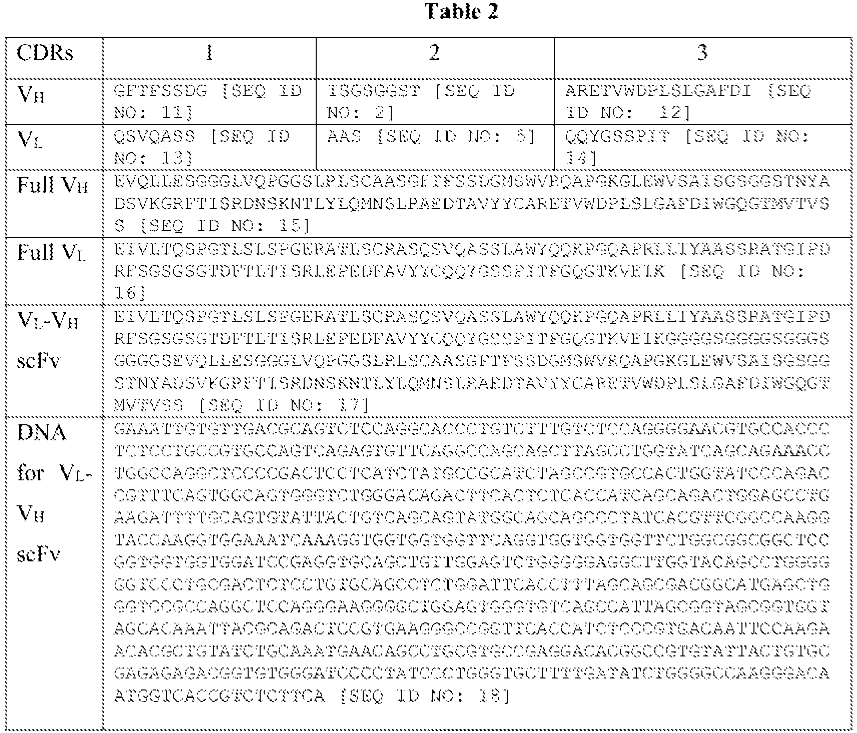

In certain embodiments, the anti-uPAR scFv comprises a V_{H} comprising the amino acid sequence set forth in SEQ ID NO: 23 and a V_{H} comprising the amino acid sequence set forth in SEQ ID NO: 24, optionally with a linker sequence, for example a linker peptide, between the heavy chain variable region and the light chain variable region. In certain embodiments, the linker comprises the amino acid sequence set forth in SEQ ID NO: 110. SEQ ID NOs: 23 and 24 are provided in Table 3 below. In certain embodiments, the anti-uPAR scFv is designated as "3-G1-A."

In certain embodiments, the anti-uPAR scFv is an scFv-Fc fusion protein or full-length human IgG with V_{H} and V_{L} regions or CDRs selected from Table 3. In certain embodiments, the anti-uPAR scFv comprises a V_{H} comprising the amino acid sequence set forth in SEQ ID NO: 23. In certain embodiments, the anti-uPAR scFv comprises a V_{L} comprising the amino acid sequence set forth in SEQ ID NO: 24.

In certain embodiments, the anti-uPAR scFv comprises a V_{H} comprising the amino acid sequence set forth in SEQ ID NO: 23 and a V_{L} comprising the amino acid sequence set forth in SEQ ID NO: 24.

In certain embodiments, the anti-uPAR scFv comprises a V_{H} comprising a CDR1 comprising the amino acid sequence set forth in SEQ ID NO: 19 or a conservative modification thereof, a CDR2 comprising the amino acid sequence set forth in SEQ ID NO: 2 or a conservative modification thereof, and a CDR3 comprising the amino acid sequence set forth in SEQ ID NO: 20 or a conservative modification thereof. SEQ ID NOs: 2. 19. and 20 are provided in Table 3.

In certain embodiments, the anti-uPAR scFv comprises a V_{L} comprising a CDR1 comprising the amino acid sequence set forth in SEQ ID NO: 21 or a conservative modification thereof, a CDR2 comprising the amino acid sequence set forth in SEQ ID NO: 5 or a conservative modification thereof, and a CDR3 comprising the amino acid sequence set forth in SEQ ID NO: 22 or a conservative modification thereof. SEQ ID NOs: 5, 21, and 22 are provided in Table 3.

In certain embodiments, the anti-uPAR scFv comprises a V_{H} comprising a CDR1 comprising the amino acid sequence set forth in SEQ ID NO: 19 or a conservative modification thereof, a CDR2 comprising the amino acid sequence set forth in SEQ ID NO: 2 or a conservative modification thereof, and a CDR3 comprising the amino acid sequence set forth in SEQ ID NO: 20 or a conservative modification thereof; and a V_{L} comprising a CDR1 comprising the amino acid sequence set forth in SEQ ID NO: 21 or a conservative modification thereof, a CDR2 comprising the amino acid sequence set forth in SEQ ID NO: 5 or a conservative modification thereof, and a CDR3 comprising the amino acid sequence set forth in SEQ ID NO: 22 or a conservative modification thereof.

In certain embodiments, the anti-uPAR scFv comprises a V_{H} comprising a CDR 1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; and a V_{L} comprising a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22.

In certain embodiments, the anti-uPAR scFv comprises a V_{H} comprising the amino acid sequence set forth in SEQ ID NO: 23, and a V_{L} comprising the amino acid sequence set forth in SEQ ID NO: 24. In certain embodiments, the V_{H} and V_{L} are linked via a linker. In certain embodiments, the linker comprises the amino acid sequence set forth in SEQ ID NO: 110.

In certain embodiments, a heavy chain variable region (Vu) is positioned at the N-terminus. In certain embodiments, the variable regions are positioned from the N- to the C-terminus: V_{B}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, anti-uPAR scFv comprises the amino acid sequence set forth in SEQ ID NO: 25. An exemplary nucleotide sequence encoding the amino acid sequence of SEQ ID NO: 25 is set forth in SEQ ID NO: 26. SEQ ID NOS: 25 and 26 are provided in Table 3 below.

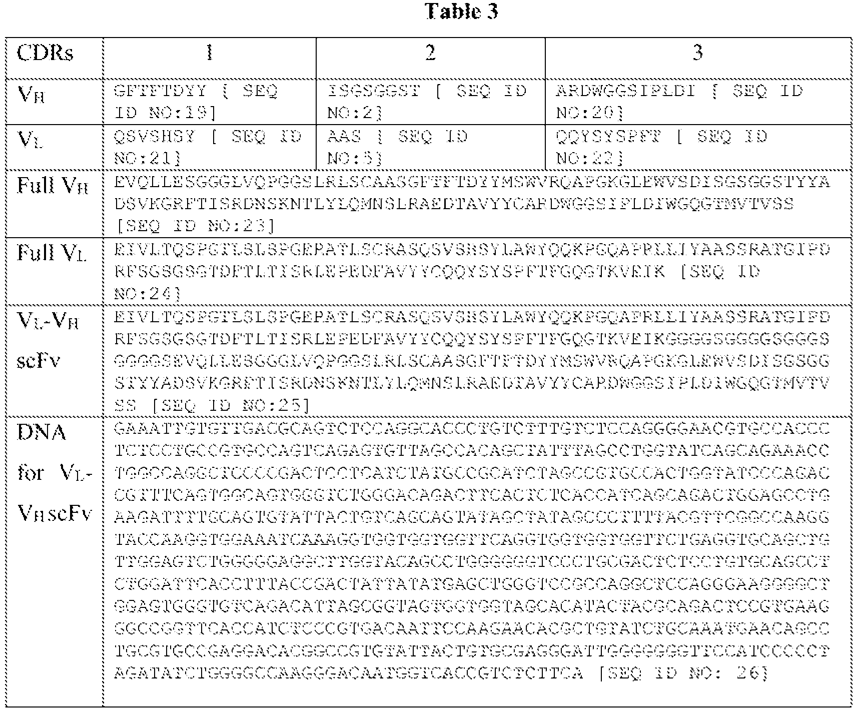

In certain embodiments, the anti-uPAR scFv comprises a V_{H} comprising the amino acid sequence set forth in SEQ ID NO: 31 and a V_{L} comprising the amino acid sequence set forth in SEQ ID NO: 32. optionally with a linker sequence. for example a linker peptide, between the heavy chain variable region and the light chain variable region. In certain embodiments, the linker comprises the amino acid sequence set forth in SEQ ID NO: 110. SEQ ID NOs: 31 and 32 are provided in Table 4 below. In certain embodiments, the anti-uPAR scFv is designated as "3-H4-A."

In certain embodiments, the anti-uPAR scFv is an scFv-Fc fusion protein or full length human IgG with V_{H} and V_{L} regions or CDRs selected from Table 4. In certain embodiments, the anti-uPAR scFv comprises a V_{H} comprising the amino acid sequence set forth in SEQ ID NO: 31. In certain embodiments, the anti-uPAR scFv comprises a V_{L} comprising the amino acid sequence set forth in SEQ ID NO: 32. In certain embodiments, the anti-uPAR scFv comprises a V_{H} comprising the amino acid sequence set forth in SEQ ID NO: 31 and a V_{L} comprising the amino acid sequence set forth in SEQ ID NO: 32. SEQ ID NOs: 31 and 32 are provided in Table 4.

In certain embodiments, the anti-uPAR scFv comprises a V_{H} comprising a CDR1 comprising the amino acid sequence set forth in SEQ ID NO: 19 or a conservative modification thereof, a CDR2 comprising the amino acid sequence set forth in SEQ ID NO: 27 or a conservative modification thereof, and a CDR3 comprising the amino acid sequence set forth in SEQ ID NO: 28 or a conservative modification thereof. SEQ ID NOs: 19, 27, and 28 are provided in Table 4.

In certain embodiments, the anti-uPAR scFv comprises a V_{L} comprising a CDR1 comprising the amino acid sequence set forth in SEQ ID NO: 29 or a conservative modification thereof, a CDR2 comprising the amino acid sequence set forth in SEQ ID NO: 5 or a conservative modification thereof, and a CDR3 comprising the amino acid sequence set forth in SEQ ID NO: 30 or a conservative modification thereof. SEQ ID NOs: 5, 29, and 30 are provided in Table 4.

In certain embodiments, the anti-uPAR scFv comprises a V_{H} comprising a CDR1 comprising the amino acid sequence set forth in SEQ ID NO: 19 or a conservative modification thereof, a CDR2 comprising the amino acid sequence set forth in SEQ ID NO: 27 or a conservative modification thereof, and a CDR3 comprising the amino acid sequence set forth in SEQ ID NO: 28 or a conservative modification thereof: and a V_{L} comprising a CDR1 comprising the amino acid sequence set forth in SEQ ID NO: 29 or a conservative modification thereof, a CDR2 comprising the amino acid sequence set forth in SEQ ID NO: 5 or a conservative modification thereof, and a CDR3 comprising the amino acid sequence set forth in SEQ ID NO: 30 or a conservative modification thereof.

In certain embodiments, the anti-uPAR scFv comprises a V_{H} comprising a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28; and a V_{L} comprising CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30.

In certain embodiments, the anti-uPAR scFv comprises a V_{H} comprising the amino acid sequence set forth in SEQ ID NO: 31, and a V_{L} comprising the amino acid sequence set forth in SEQ ID NO: 32. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, scFv comprises the amino acid sequence set forth in SEQ ID NO: 33. An exemplary nucleotide sequence encoding the amino acid sequence of SEQ ID NO: 33 is set forth in SEQ ID NO: 34. SEQ ID NOS: 33 and 34 are provided in Table 4 below.

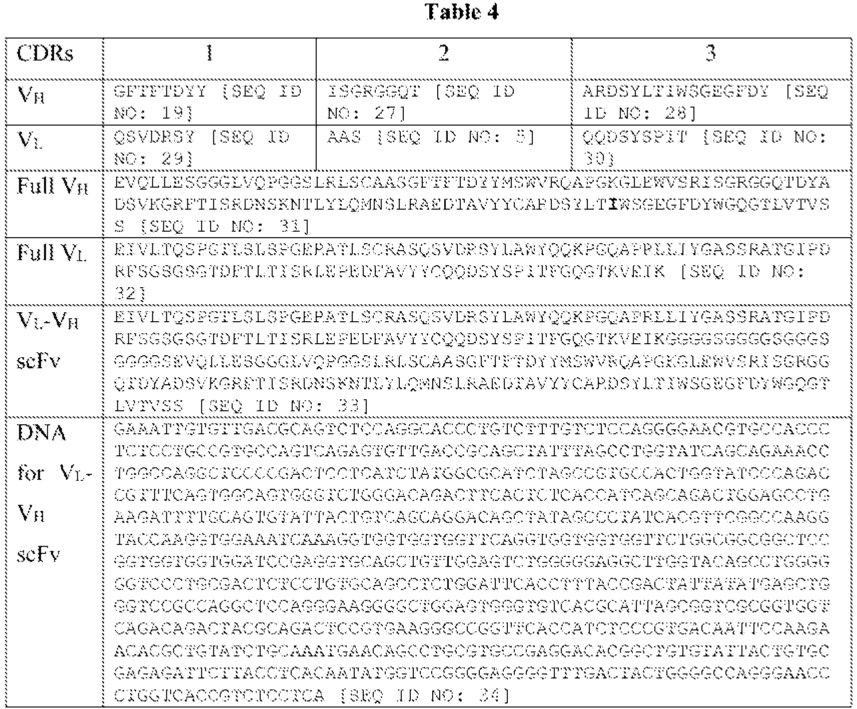

In certain embodiments, the anti-uPAR scFv comprises a V_{H} comprising the amino acid sequence set forth in SEQ ID NO: 41 and a V_{L} comprising the amino acid sequence set forth in SEQ ID NO: 42. optionally with a linker sequence. for example a linker peptide, between the heavy chain variable region and the light chain variable region. In certain embodiments, the linker comprises the amino acid sequence set forth in SEQ ID NO: 110. SEQ ID NOs: 41 and 42 are provided Table 5. In certain embodiments, the anti-uPAR scFv is designated as "4-F5-A."

In certain embodiments. the anti-uPAR scFv is an scFv-Fe fusion protein or a full-length human IgG with V_{H} and V_{L} regions or CDRs selected from Table 5. In certain embodiments, the anti-uPAR scFv comprises a V_{H} comprising the amino acid sequence set forth in SEQ ID NO: 41. In certain embodiments, the anti-uPAR scFv comprises a V_{L} comprising the amino acid sequence set forth in SEQ ID NO: 42. In certain embodiments, the anti-uPAR scFv comprises a V_{H} comprising the amino acid sequence set forth in SEQ ID NO: 41 and a V_{L} comprising the amino acid sequence set forth in SEQ ID NO: 42.

In certain embodiments, the anti-uPAR scFv comprises a V_{H} comprising a CDR1 comprising the amino acid sequence set forth in SEQ ID NO: 35 or a conservative modification thereof, a CDR2 comprising the amino acid sequence set forth in SEQ ID NO: 36 or a conservative modification thereof, and a CDR3 comprising the amino acid sequence set forth in SEQ ID NO: 37 or a conservative modification thereof. SEQ ID NOs: 35-37 are provided in Table 5.

In certain embodiments, the anti-uPAR scFv comprises a V_{L} comprising a CDR1 comprising the amino acid sequence set forth in SEQ ID NO: 38 or a conservative modification thereof, a CDR2 comprising the amino acid sequence set forth in SEQ ID NO: 39 or a conservative modification thereof, and a CDR3 comprising the amino acid sequence set forth in SEQ ID NO: 40 or a conservative modification thereof. SEQ ID NOs: 38-40 are provided in Table 5.

In certain embodiments, the anti-uPAR scFv comprises a V_{H} comprising a CDR1 comprising the amino acid sequence set forth in SEQ ID NO: 35 or a conservative modification thereof, a CDR2 comprising the amino acid sequence set forth in SEQ ID NO: 36 or a conservative modification thereof, and a CDR3 comprising the amino acid sequence set forth in SEQ ID NO: 37 or a conservative modification thereof, and a V_{L} comprising a CDR1 comprising the amino acid sequence set forth in SEQ ID NO: 38 or a conservative modification thereof, a CDR2 comprising the amino acid sequence set forth in SEQ ID NO: 39 or a conservative modification thereof, and a CDR3 comprising the amino acid sequence set forth in SEQ ID NO: 40 or a conservative modification thereof.

In certain embodiments, the anti-uPAR scFv comprises a V_{H} comprising a CDR1 comprising the amino acid sequence set forth in SEQ ID NO: 35, a CDR2 comprising the amino acid sequence set forth in SEQ ID NO: 36, a CDR3 comprising the amino acid sequence set forth in SEQ ID NO: 37; and a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40.

In certain embodiments, the anti-uPAR scFv comprises a V_{H} comprising the amino acid sequence set forth in SEQ ID NO: 41, and a V_{L} comprising the amino acid sequence set forth in SEQ ID NO: 42. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, the anti-uPAR scFv comprises the amino acid sequence set forth in SEQ ID NO: 43. An exemplary nucleotide sequence encoding the amino acid sequence of SEQ ID NO: 43 is set forth in SEQ ID NO: 44. SEQ ID NOS: 43 and 44 are provided in Table 5 below.

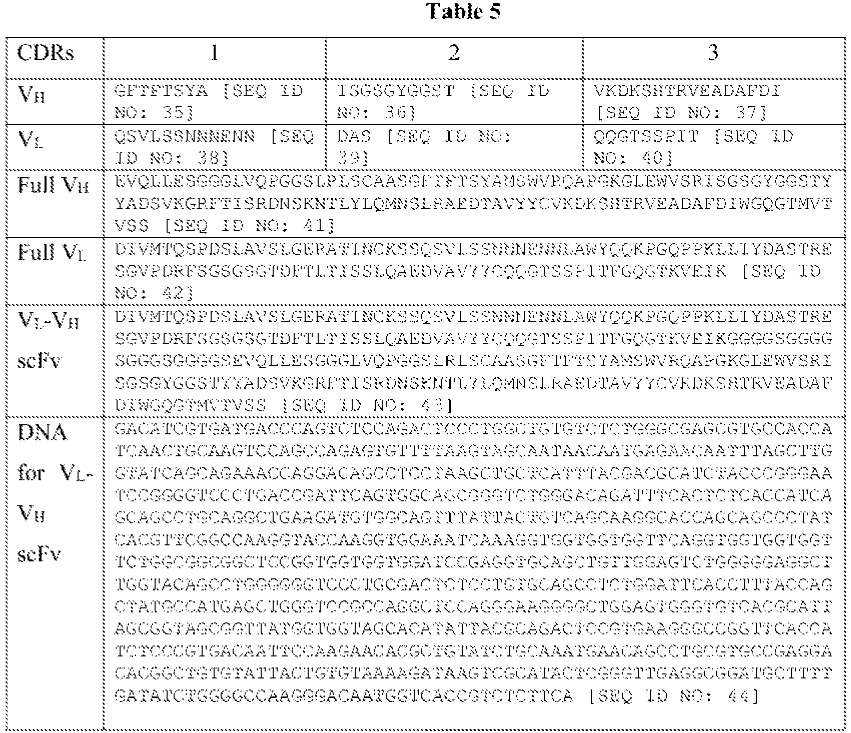

In certain embodiments, the anti-uPAR scFv comprises a V_{H} comprising the amino acid sequence set forth in SEQ ID NO: 50 and a V_{L} comprising the amino acid sequence set forth in SEQ ID NO: 51, optionally with a linker sequence, for example a linker peptide. between the heavy chain variable region and the light chain variable region. In certain embodiments, the linker comprises the amino acid sequence set forth in SEQ ID NO: 110. SEQ ID NOs: 50 and 51 are provided in Table 6 below. In certain embodiments, the anti-uPAR scFv is designated as "4-F12-A."

In certain embodiments, the anti-uPAR scFv is an scFv-Fc fusion protein or a full-length human IgG with V_{H} and V_{L} regions or CDRs selected from Table 6. In certain embodiments, the anti-uPAR scFv comprises a V_{H} comprising the amino acid sequence set forth in SEQ ID NO: 50. In certain embodiments, the anti-uPAR scFv comprises a V_{L} comprising the amino acid sequence set forth in SEQ ID NO: 51. In certain embodiments. the anti-uPAR scFv comprises a V_{H} comprising the amino acid sequence set forth in SEQ ID NO: 50 and a V_{L} comprising the amino acid sequence set forth in SEQ ID NO: 51.

In certain embodiments, the anti-uPAR scFv comprises a V_{H} comprising a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 47 or a conservative modification thereof. SEQ ID NOs: 45-47 are provided in Table 6.

In certain embodiments, the anti-uPAR scFv comprises a V_{L} comprising a CDR1 comprising the amino acid sequence set forth in SEQ ID NO: 48 or a conservative modification thereof, a CDR2 comprising the amino acid sequence set forth in SEQ ID NO: 5 or a conservative modification thereof, and a CDR3 comprising the amino acid sequence set forth in SEQ ID NO: 49 or a conservative modification thereof. SEQ ID NOs: 5, 48, and 49 are provided in Table 6.

In certain embodiments, the anti-uPAR scFv comprises a V_{H} comprising a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 47 or a conservative modification thereof, and a V_{L} comprising a CDR1 comprising the amino acid sequence set forth in SEQ ID NO: 48 or a conservative modification thereof, a CDR2 comprising the amino acid sequence set forth in SEQ ID NO: 5 or a conservative modification thereof, and a CDR3 comprising the amino acid sequence set forth in SEQ ID NO: 49 or a conservative modification thereof.

In certain embodiments, the anti-uPAR scFv comprises a V_{H} comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; and a V_{L} comprising a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49.

In certain embodiments, the anti-uPAR scFv comprises a V_{H} comprising the amino acid sequence set forth in SEQ ID NO: 50, and a V_{L} comprising the amino acid sequence set forth in SEQ ID NO: 51. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, the anti-uPAR scFv comprises the amino acid sequence set forth in SEQ ID NO: 52. An exemplary nucleotide sequence encoding the amino acid sequence of SEQ ID NO: 52 is set forth in SEQ ID NO: 53. SEQ ID NOS: 52 and 53 are provided in Table 6 below.

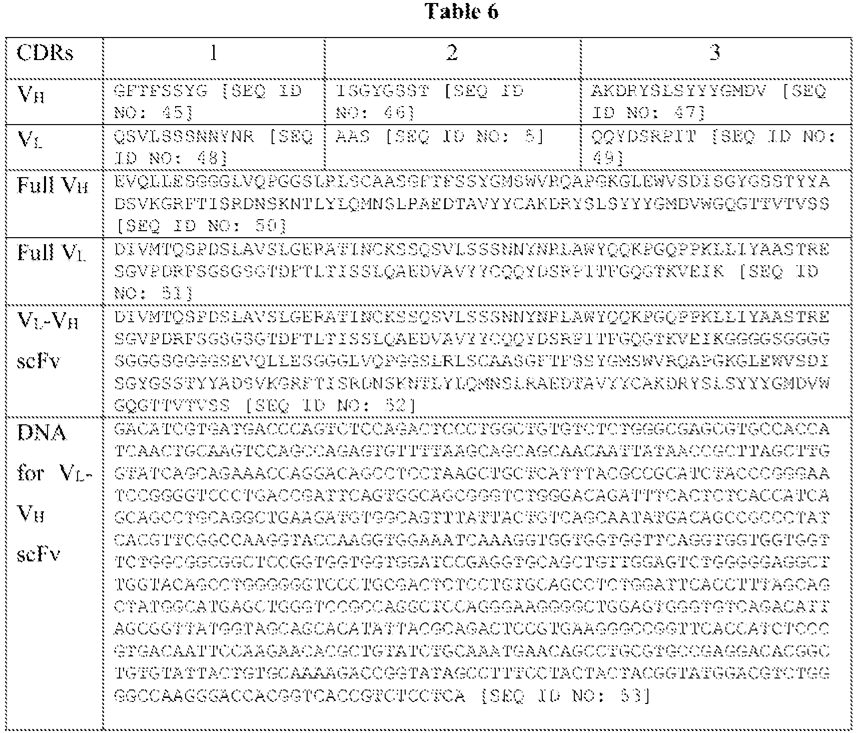

In certain embodiments, the anti-uPAR scFv comprises a V_{H} comprising the amino acid sequence set forth in SEQ ID NO: 59 and a V_{L} comprising the amino acid sequence set forth in SEQ ID NO: 60, optionally with a linker sequence, for example a linker peptide, between the heavy chain variable region and the light chain variable region. In certain embodiments, the linker comprises the amino acid sequence set forth in SEQ ID NO: 110. SEQ ID NOs: 59 and 60 are provided in Table 7 below. In certain embodiments, the anti-uPAR scFv is designated as "4-A5-B."

In certain embodiments, the anti-uPAR scFv is an scFv-Fc fusion protein or a full-length human IgG with V_{H} and V_{L} regions or CDRs selected from Table 7. In certain embodiments, the anti-uPAR scFv comprises a V_{H} comprising the amino acid sequence set forth in SEQ ID NO: 59. In certain embodiments, the anti-uPAR scFv comprises a V_{L} comprising the amino acid sequence set forth in SEQ ID NO: 60. In certain embodiments, the anti-uPAR scFv comprises a V_{H} comprising the amino acid sequence set forth in SEQ ID NO: 59 and a V_{L} comprising the amino acid sequence set forth in SEQ ID NO: 60.

In certain embodiments, the anti-uPAR scFv comprises a V_{H} comprising a CDR1 comprising the amino acid sequence set forth in SEQ ID NO: 54 or a conservative modification thereof, a CDR2 comprising the amino acid sequence set forth in SEQ ID NO: 55 or a conservative modification thereof, and a CDR3 comprising the amino acid sequence set forth in SEQ ID NO: 56 or a conservative modification thereof. SEQ ID NOs: 54-56 are provided in Table 7.

In certain embodiments, the anti-uPAR scFv comprises a V_{L} comprising a CDR1 comprising the amino acid sequence set forth in SEQ ID NO: 57 or a conservative modification thereof, a CDR2 comprising the amino acid sequence set forth in SEQ ID NO: 5 or a conservative modification thereof, and a CDR3 comprising the amino acid sequence set forth in SEQ ID NO: 58 or a conservative modification thereof. SEQ ID NOs: 5, 57, and 58 are provided in Table 7.

In certain embodiments, the anti-uPAR scFv comprises a V_{H} comprising a CDR1 comprising the amino acid sequence set forth in SEQ ID NO: 54 or a conservative modification thereof, a CDR2 comprising the amino acid sequence set forth in SEQ ID NO: 55 or a conservative modification thereof, a CDR3 comprising the amino acid sequence set forth in SEQ ID NO: 56 or a conservative modification thereof; and a V_{L} comprising a CDR1 comprising the amino acid sequence set forth in SEQ ID NO: 57 or a conservative modification thereof, a CDR2 comprising the amino acid sequence set forth in SEQ ID NO: 5 or a conservative modification thereof, and a CDR3 comprising the amino acid sequence set forth in SEQ ID NO: 58 or a conservative modification thereof.

In certain embodiments, the anti-uPAR scFv comprises a V_{H} comprising a CDR1 comprising the amino acid sequence set forth in SEQ ID NO: 54, a CDR2 comprising the amino acid sequence set forth in SEQ ID NO: 55, a CDR3 comprising the amino acid sequence set forth in SEQ ID NO: 56; and a V_{L} comprising a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58.

In certain embodiments, the anti-uPAR scFv comprises a V_{H} comprising the amino acid sequence set forth in SEQ ID NO: 59, and a V_{L} comprising the amino acid sequence set forth in SEQ ID NO: 60. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, the anti-uPAR scFv comprises the amino acid sequence set forth in SEQ ID NO: 61. An exemplary nucleotide sequence encoding the amino acid sequence of SEQ ID NO: 61 is set forth in SEQ ID NO: 62. SEQ ID NOS: 61 and 62 are provided in Table 7 below.

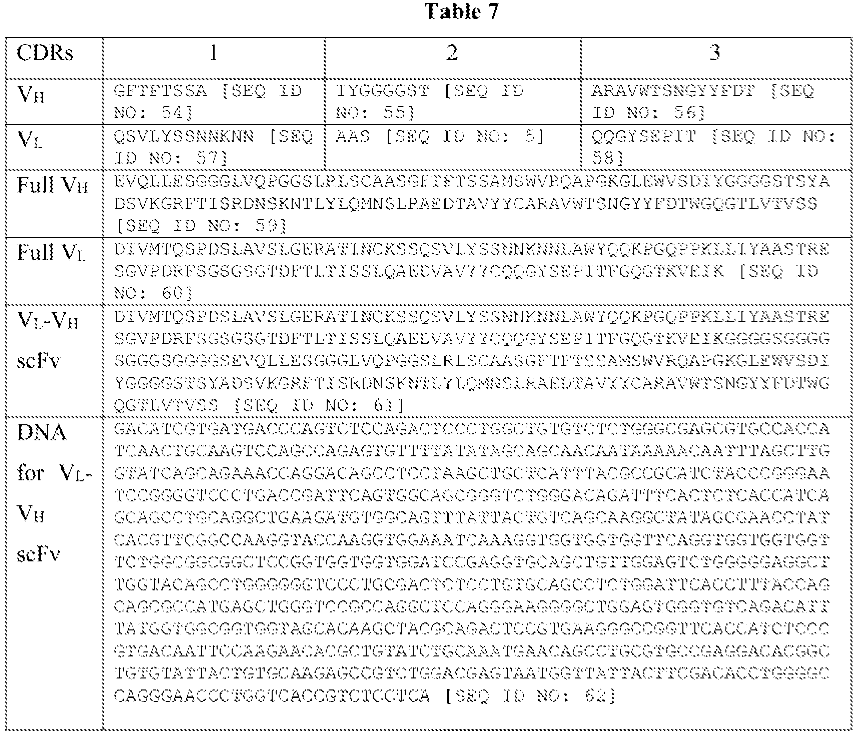

In certain embodiments, the anti-uPAR scFv comprises a V_{H} comprising the amino acid sequence set forth in SEQ ID NO: 69 and a V_{L} comprising the amino acid sequence set forth in SEQ ID NO: 70, optionally with a linker sequence, for example a linker peptide, between the heavy chain variable region and the light chain variable region. In certain embodiments, the linker comprises the amino acid sequence set forth in SEQ ID NO: 110. SEQ ID NOs: 69 and 70 are provided in Table 8 below. In certain embodiments, the anti-uPAR scFv is designated as "05G9."

In certain embodiments, the anti-uPAR scFv is an scFv-Fc fusion protein or a full-length human IgG with V_{H} and V_{L} regions or CDRs selected from Table 8. In certain embodiments, the anti-uPAR scFv comprises a V_{H} comprising the amino acid sequence set forth in SEQ ID NO: 69. In certain embodiments, the anti-uPAR scFv comprises a V_{L} comprising the amino acid sequence set forth in SEQ ID NO: 70. In certain embodiments, the anti-uPAR scFv comprises a V_{H} comprising the amino acid sequence set forth in SEQ ID NO: 69 and a V_{L} comprising the amino acid sequence set forth in SEQ ID NO: 70.

In certain embodiments, the anti-uPAR scFv comprises a V_{H} comprising a CDR1 comprising the amino acid sequence set forth in SEQ ID NO: 63 or a conservative modification thereof, a CDR2 comprising the amino acid sequence set forth in SEQ ID NO: 64 or a conservative modification thereof, and a CDR3 comprising the amino acid sequence set forth in SEQ ID NO: 65 or a conservative modification thereof. SEQ ID NOs: 63-65 are provided in Table 8.

In certain embodiments, the anti-uPAR scFv comprises a V_{L} comprising a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 68 or a conservative modification thereof. SEQ ID NOs: 66-68 are provided in Table 8.

In certain embodiments, the anti-uPAR scFv comprises a V_{H} comprising a CDR1 comprising the amino acid sequence set forth in SEQ ID NO: 63 or a conservative modification thereof, a CDR2 comprising the amino acid sequence set forth in SEQ ID NO: 64 or a conservative modification thereof, and a CDR3 comprising the amino acid sequence set forth in SEQ ID NO: 65 or a conservative modification thereof; and a V_{L} comprising a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 68 or a conservative modification thereof.

In certain embodiments, the anti-uPAR scFv comprises a V_{H} comprising a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65: and a V_{L} comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68.

In certain embodiments, the anti-uPAR scFv comprises a V_{H} comprising the amino acid sequence set forth in SEQ ID NO: 69, and a V_{L} comprising the amino acid sequence set forth in SEQ ID NO: 70. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, the anti-uPAR. scFv comprises the amino acid sequence set forth in SEQ ID NO: 71. An exemplary nucleotide sequence encoding the amino acid sequence of SEQ ID NO: 71 is set forth in SEQ ID NO: 72. SEQ ID NOS: 71 and 72 are provided in Table 8 below.

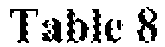

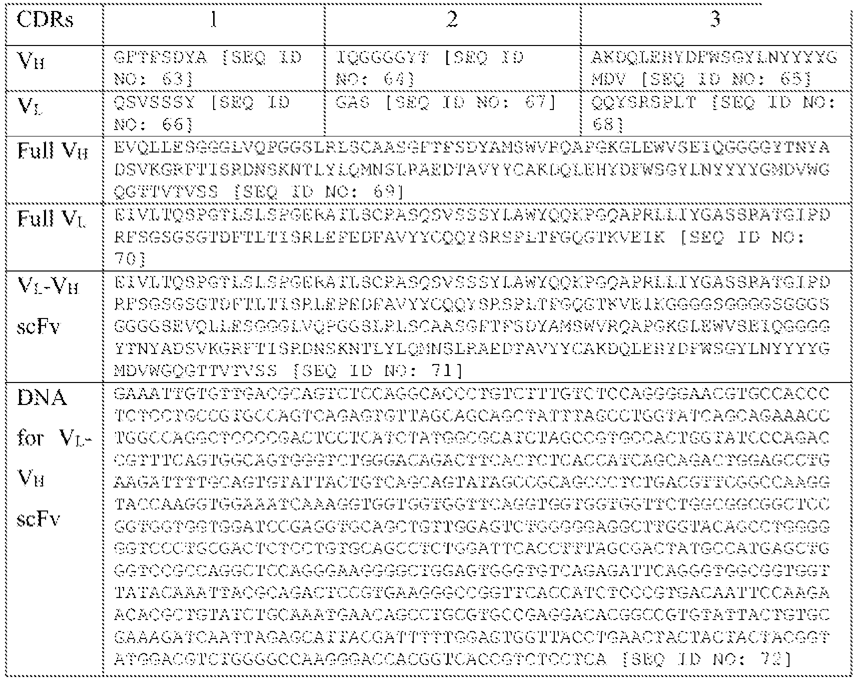

In certain embodiments, the anti-uPAR scFv comprises a V_{H} comprising the amino acid sequence set forth in SEQ ID NO: 78 and a V_{L} comprising the amino acid sequence set forth in SEQ ID NO: 79, optionally with a linker sequence, for example a linker peptide, between the heavy chain variable region and the light chain variable region. In certain embodiments, the linker comprises the amino acid sequence set forth in SEQ ID NO: 110. SEQ ID NOs: 78 and 79 are provided in Table 9 below. In certain embodiments, the anti-uPAR scFv is designated as "05A6."

In certain embodiments, the anti-uPAR scFv is an scFv-Fc fusion protein or a full-length human IgG with Vu and V_{L} regions or CDRs selected from Table 9. In certain embodiments, the anti-uPAR scFv comprises a V_{H} comprising the amino acid sequence set forth in SEQ ID NO: 78. In certain embodiments, the anti-uPAR scFv comprises a V_{L} comprising the amino acid sequence set forth in SEQ ID NO: 79. In certain embodiments, the anti-uPAR scFv comprises a V_{H} comprising the amino acid sequence set forth in SEQ ID NO: 78 and a V_{L} comprising the amino acid sequence set forth in SEQ ID NO: 79.

In certain embodiments, the anti-uPAR scFv comprises a V_{H} comprising a CDR1 comprising the amino acid sequence set forth in SEQ ID NO: 73 or a conservative modification thereof, a CDR2 comprising the amino acid sequence set forth in SEQ ID NO: 74 or a conservative modification thereof, and a CDR3 comprising the amino acid sequence set forth in SEQ ID NO: 75 or a conservative modification thereof. SEQ ID NOs: 73-75 are provided in Table 9.

In certain embodiments, the anti-uPAR scFv comprises a V_{L} comprising a CDR1 comprising the amino acid sequence set forth in SEQ ID NO: 76 or a conservative modification thereof, a CDR2 comprising the amino acid sequence set forth in SEQ ID NO: 67 or a conservative modification thereof, and a CDR3 comprising the amino acid sequence set forth in SEQ ID NO: 77 or a conservative modification thereof: SEQ ID NOs: 76. 67, and 77 are provided in Table 9.

In certain embodiments, the anti-uPAR scFv comprises a V_{H} comprising a CDR1 comprising the amino acid sequence set forth in SEQ ID NO: 73 or a conservative modification thereof, a CDR2 comprising the amino acid sequence set forth in SEQ ID NO: 74 or a conservative modification thereof, and a CDR3 comprising the amino acid sequence set forth in SEQ ID NO: 75 or a conservative modification thereof; and a V_{L} comprising a CDR1 comprising the amino acid sequence set forth in SEQ ID NO: 76 or a conservative modification thereof, a CDR2 comprising the amino acid sequence set forth in SEQ ID NO: 67 or a conservative modification thereof, and a CDR3 comprising the amino acid sequence set forth in SEQ ID NO: 77 or a conservative modification thereof.

In certain embodiments, the anti-uPAR scFv comprises a V_{H} comprising a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; and a V_{L} comprising a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77.

In certain embodiments, the anti-uPAR. scFv comprises a V_{H} comprising the amino acid sequence set forth in SEQ ID NO: 78, and a V_{L} comprising the amino acid sequence set forth in SEQ ID NO: 79. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, the anti-uPAR scFv comprises the amino acid sequence set forth in SEQ ID NO: 80. An exemplary nucleotide sequence encoding the amino acid sequence of SEQ ID NO: 80 is set forth in SEQ ID NO: 81. SEQ ID NOS: 80 and 81 are provided in Table 9 below.

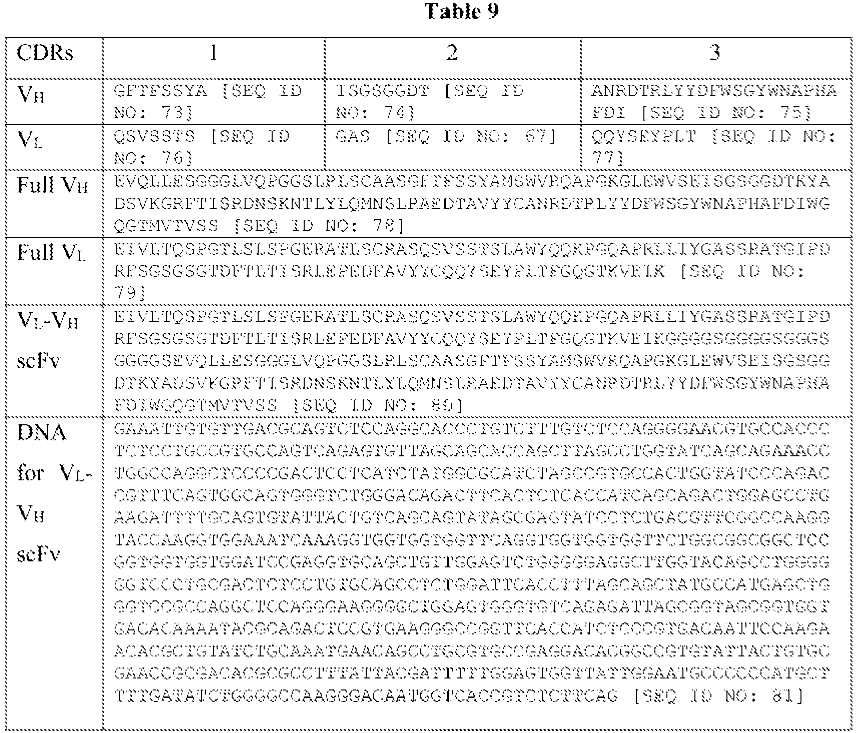

In certain embodiments, the anti-uPAR scFv comprises a V_{H} comprising the amino acid sequence set forth in SEQ ID NO: 86 and a V_{L} comprising the amino acid sequence set forth in SEQ ID NO: 87, optionally with a linker sequence, for example a linker peptide, between the heavy chain variable region and the light chain variable region. In certain embodiments, the linker comprises the amino acid sequence set forth in SEQ ID NO: 110. SEQ ID NOs: 86 and 87 are provided in Table 10 below. In certain embodiments, the anti-uPAR scFv is designated as "05B2."

In certain embodiments, the anti-uPAR scFv is an scFv-Fc fusion protein or a full-length human IgG with V_{H} and V_{L} regions or CDRs selected from Table 10. In certain embodiments, the anti-uPAR scFv comprises a V_{H} comprising the amino acid sequence set forth in SEQ ID NO: 86. In certain embodiments, the anti-uPAR scFv comprises a V_{L} comprising the amino acid sequence set forth in SEQ ID NO: 87. In certain embodiments, the anti-uPAR scFv comprises a V_{H} comprising the amino acid sequence set forth in SEQ ID NO: 86 and a V_{L} comprising the amino acid sequence set forth in SEQ ID NO: 87.

In certain embodiments, the anti-uPAR scFv comprises a V_{H} comprising a CDR1 comprising the amino acid sequence set forth in SEQ ID NO: 45 or a conservative modification thereof, a CDR2 comprising the amino acid sequence set forth in SEQ ID NO: 82 or a conservative modification thereof, and a CDR3 comprising the amino acid sequence set forth in SEQ ID NO: 83 or a conservative modification thereof: SEQ ID NOs: 45, 82, and 83 are provided in Table 10.

In certain embodiments, the anti-uPAR scFv comprises a V_{L} comprising a CDR1 comprising the amino acid sequence set forth in SEQ ID NO: 84 or a conservative modification thereof, a CDR2 comprising the amino acid sequence set forth in SEQ ID NO: 67 or a conservative modification thereof, and a CDR3 comprising the amino acid sequence set forth in SEQ ID NO: 85 or a conservative modification thereof. SEQ ID NOs: 84. 67, and 85 are provided in Table 10.

In certain embodiments, the anti-uPAR scFv comprises a V_{H} comprising a CDR1 comprising the amino acid sequence set forth in SEQ ID NO: 45 or a conservative modification thereof, a CDR2 comprising the amino acid sequence set forth in SEQ ID NO: 82 or a conservative modification thereof, a CDR3 comprising the amino acid sequence set forth in SEQ ID NO: 83 or a conservative modification thereof, and a V_{L} comprising a CDR1 comprising the amino acid sequence set forth in SEQ ID NO: 84 or a conservative modification thereof, a CDR2 comprising the amino acid sequence set forth in SEQ ID NO: 67 or a conservative modification thereof, and a CDR3 comprising the amino acid sequence set forth in SEQ ID NO: 85 or a conservative modification thereof.

In certain embodiments, the anti-uPAR scFv comprises a V_{H} comprising a CDR1 comprising the amino acid sequence set forth in SEQ ID NO: 45, a CDR2 comprising the amino acid sequence set forth in SEQ ID NO: 82, a CDR3 comprising the amino acid sequence set forth in SEQ ID NO: 83; and a V₁. comprising a CDR1 comprising the amino acid sequence set forth in SEQ ID NO: 84, a CDR2 comprising the amino acid sequence set forth in SEQ ID NO: 67. and a CDR3 comprising the amino acid sequence set forth in SEQ ID NO: 85.

In certain embodiments, the anti-uPAR scFv comprises a V_{H} comprising the amino acid sequence set forth in SEQ ID NO: 86, and a V_{L} comprising the amino acid sequence set forth in SEQ ID NO: 87. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments. the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, the anti-uPAR scFv comprises the amino acid sequence set forth in SEQ ID NO: 88. An exemplary nucleotide sequence encoding the amino acid sequence of SEQ ID NO: 88 is set forth in SEQ ID NO: 89. SEQ ID NOS: 88 and 89 are provided in Table 10 below.

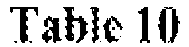

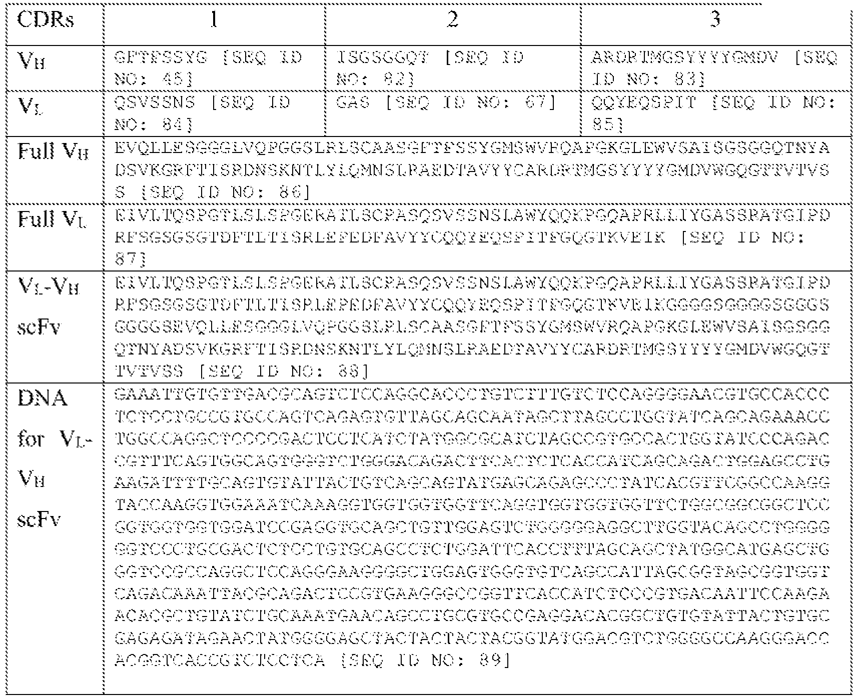

In certain embodiments, the anti-uPAR scFv comprises a V_{H} comprising the amino acid sequence set forth in SEQ ID NO: 95 and a V_{L} comprising the amino acid sequence set forth in SEQ ID NO: 96, optionally with a linker sequence, for example a linker peptide, between the heavy chain variable region and the light chain variable region. In certain embodiments, the linker comprises the amino acid sequence set forth in SEQ ID NO: 110. SEQ ID NOs: 95 and 96 are provided in Table 11 below. In certain embodiments, the anti-uPAR scFv is designated as "05F5."

In certain embodiments, the anti-uPAR scFv is an scFv-Fc fusion protein or a full-length human IgG with V_{H} and V_{L} regions or CDRs selected from Table 11. In certain embodiments, the anti-uPAR scFv comprises a V_{H} comprising the amino acid sequence set forth in SEQ ID NO: 95. In certain embodiments, the anti-uPAR scFv comprises a V_{L} comprising the amino acid sequence set forth in SEQ ID NO: 96. In certain embodiments, the anti-uPAR scFv comprises a V_{H} comprising the amino acid sequence set forth in SEQ ID NO: 95 and a V_{L} comprising the amino acid sequence set forth in SEQ ID NO: 96.

In certain embodiments, the anti-uPAR scFv comprises a V_{H} comprising a CDR1 comprising the amino acid sequence set forth in SEQ ID NO: 90 or a conservative modification thereof, a CDR2 comprising the amino acid sequence set forth in SEQ ID NO: 91 or a conservative modification thereof, and a CDR3 comprising the amino acid sequence set forth in SEQ ID NO: 92 or a conservative modification thereof. SEQ ID NOs: 90-92 are provided in Table 11.

In certain embodiments, the anti-uPAR scFv comprises a V_{L} comprising a CDR1 comprising the amino acid sequence set forth in SEQ ID NO: 93 or a conservative modification thereof, a CDR2 comprising the amino acid sequence set forth in SEQ ID NO: 67 or a conservative modification thereof, and a CDR3 comprising the amino acid sequence set forth in SEQ ID NO: 94 or a conservative modification thereof. SEQ ID NOs: 93. 67, and 94 are provided in Table 11.

In certain embodiments, the anti-uPAR scFv comprises a V_{H} comprising a CDR1 comprising the amino acid sequence set forth in SEQ ID NO: 90 or a conservative modification thereof, a CDR2 comprising the amino acid sequence set forth in SEQ ID NO: 91 or a conservative modification thereof, and a CDR3 comprising the amino acid sequence set forth in SEQ ID NO: 92 or a conservative modification thereof; and a V_{L} comprising a CDR1 comprising the amino acid sequence set forth in SEQ ID NO: 93 or a conservative modification thereof, a CDR2 comprising the amino acid sequence set forth in SEQ ID NO: 67 or a conservative modification thereof, and a CDR3 comprising the amino acid sequence set forth in SEQ ID NO: 94 or a conservative modification thereof.

In certain embodiments, the anti-uPAR scFv comprises a V_{H} comprising a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92: a V_{L} comprising a CDR1 comprising the amino acid sequence set forth in SEQ ID NO: 93, a CDR2 comprising the amino acid sequence set forth in SEQ ID NO: 67. and a CDR3 comprising the amino acid sequence set forth in SEQ ID NO: 94.

In certain embodiments, the anti-uPAR scFv comprises a V_{H} comprising the amino acid sequence set forth in SEQ ID NO: 95, and a V_{L} comprising the amino acid sequence set forth in SEQ ID NO: 96. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, the anti-uPAR scFv comprises the amino acid sequence set forth in SEQ ID NO: 97. An exemplary nucleotide sequence encoding the amino acid sequence of SEQ ID NO: 97 is set forth in SEQ ID NO: 98. SEQ ID NOS: 97 and 98 are provided in Table 11 below.

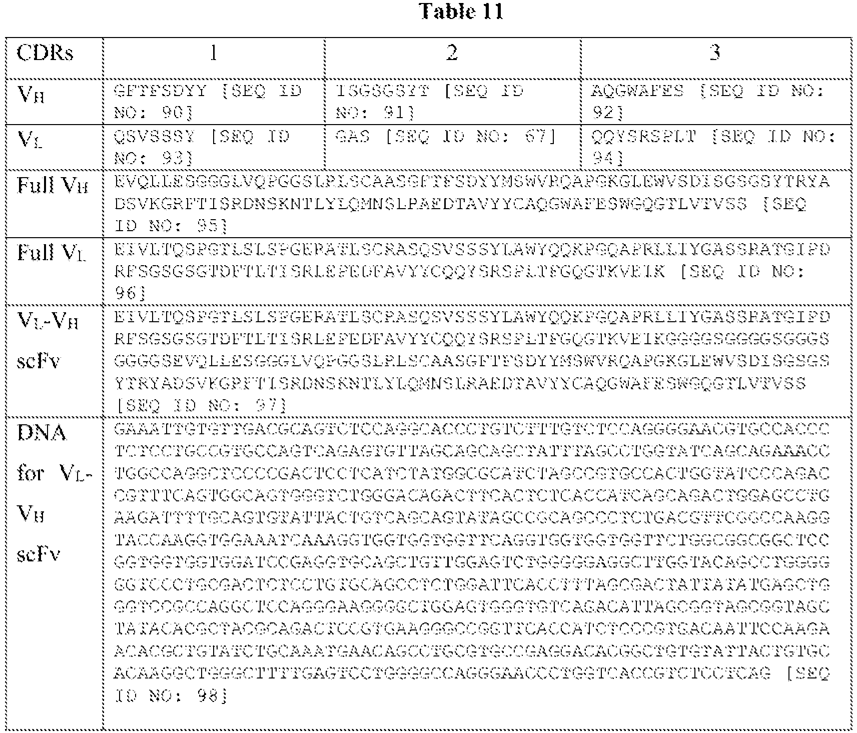

In certain embodiments, the anti-uPAR scFv comprises a V_{H} comprising the amino acid sequence set forth in SEQ ID NO: 101 and a V_{L} comprising the amino acid sequence set forth in SEQ ID NO: 102, optionally with a linker sequence, for example a linker peptide, between the heavy chain variable region and the light chain variable region. In certain embodiments, the linker comprises the amino acid sequence set forth in SEQ ID NO: 110. SEQ ID NOs: 101 and 102 are provided in Table 12 below. In certain embodiments, the anti-uPAR scFv is designated as "05G5."

In certain embodiments, the anti-uPAR scFv is an scFv-Fc fusion protein or a full-length human IgG with V_{H} and V_{L} regions or CDRs selected from Table 12. In certain embodiments, the anti-uPAR scFv comprises a V_{H} comprising the amino acid sequence set forth in SEQ ID NO: 101. In certain embodiments, the anti-uPAR scFv comprises a V_{L} comprising the amino acid sequence set forth in SEQ ID NO: 102. In certain embodiments, the anti-uPAR scFv comprises a V_{H} comprising the amino acid sequence set forth in SEQ ID NO: 101 and a V_{L} comprising the amino acid sequence set forth in SEQ ID NO: 102.

In certain embodiments, the anti-uPAR scFv comprises a V_{H} comprising a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 99 or a conservative modification thereof. SEQ ID NOs: 45, 46, and 99 are provided in Table 12.

In certain embodiments, the anti-uPAR scFv comprises a V_{L} comprising a CDR1 comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 100 or a conservative modification thereof. SEQ ID NOs: 66, 67, and 100 are provided in Table 12.

In certain embodiments, the anti-uPAR scFv comprises a V_{H} comprising a CDR1 comprising the amino acid sequence set forth in SEQ ID NO: 45 or a conservative modification thereof, a CDR2 comprising the amino acid sequence set forth in SEQ ID NO: 46 or a conservative modification thereof, and a CDR3 comprising the amino acid sequence set forth in SEQ ID NO: 99 or a conservative modification thereof; and a V_{L} comprising a CDRI comprising the amino acid sequence set forth in SEQ ID NO: 66 or a conservative modification thereof, a CDR2 comprising the amino acid sequence set forth in SEQ ID NO: 67 or a conservative modification thereof, and a CDR3 comprising the amino acid sequence set forth in SEQ ID NO: 100 or a conservative modification thereof.

In certain embodiments, the anti-uPAR scFv comprises a V_{H} comprising a CDRI comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; and a V_{L} comprising a CDR I comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.

In certain embodiments, the anti-uPAR scFv comprises a V_{H} comprising the amino acid sequence set forth in SEQ ID NO: 101, and a V_{L} comprising the amino acid sequence set forth in SEQ ID NO: 102. In certain embodiments, the V_{H} and V_{L} are linked via a linker. In certain embodiments, the linker comprises the amino acid sequence set forth in SEQ ID NO: 110.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-terminus. In certain embodiments, the variable regions are positioned from the N- to the C-terminus: V_{L}-V_{H}. In certain embodiments, the anti-uPAR scFv comprises the amino acid sequence set forth in SEQ ID NO: 103. An exemplary nucleotide sequence encoding the amino acid sequence of SEQ ID NO: 103 is set forth in SEQ ID NO: 104. SEQ ID NOS: 103 and 104 are provided in Table 12 below.

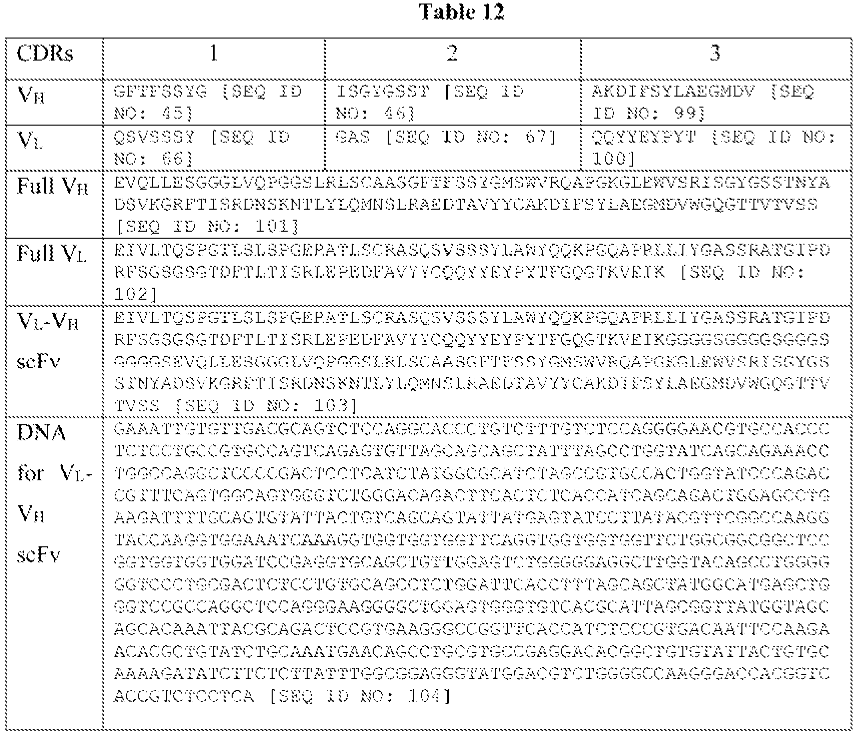

In certain embodiments, the anti-uPAR scFv comprises a V_{H} comprising the amino acid sequence set forth in SEQ ID NO: 108 and a V_{L}, optionally with a linker sequence. for example a linker peptide, between the heavy chain variable region and the light chain variable region. SEQ ID NOs: 108 are provided in Table 13 below. In certain embodiments, the anti-uPAR scFv is designated as "07B3."

In certain embodiments, the anti-uPAR scFv is an scFv-Fc fusion protein or a full-length human IgG with V_{H} and V_{L} regions or CDRs selected from Table 13. In certain embodiments, the anti-uPAR scFv comprises a V_{H} comprising the amino acid sequence set forth in SEQ ID NO: 108.

In certain embodiments, the anti-uPAR scFv comprises a V_{H} comprising a CDR1 comprising the amino acid sequence set forth in SEQ ID NO: 105 or a conservative modification thereof, a CDR2 comprising the amino acid sequence set forth in SEQ ID NO: 106 or a conservative modification thereof, and a CDR3 comprising the amino acid sequence set forth in SEQ ID NO: 107 or a conservative modification thereof. SEQ ID NOs: 105-107 are provided in Table 13.

In certain embodiments, the anti-uPAR scFv comprises a V_{H} comprising a CDR1 comprising the amino acid sequence set forth in SEQ ID NO: 105 or a conservative modification thereof, a CDR2 comprising the amino acid sequence set forth in SEQ ID NO: 106 or a conservative modification thereof, a CDR3 comprising the amino acid sequence set forth in SEQ ID NO: 107 or a conservative modification thereof.

In certain embodiments, the anti-uPAR scFv comprises a V_{H} comprising a CDR1 comprising the amino acid sequence set forth in SEQ ID NO: 105, a CDR2 comprising the amino acid sequence set forth in SEQ ID NO: 106, and a CDR3 comprising the amino acid sequence set forth in SEQ ID NO: 107.

In certain embodiments, the anti-uPAR scFv comprises a V_{H} comprising the amino acid sequence set forth in SEQ ID NO: 108, and a V_{L}. In certain embodiments, the V_{H} and V_{L} are linked via a linker. An exemplary nucleotide sequence encoding the amino acid sequence of SEQ ID NO: 108 is set forth in SEQ ID NO: 109.

In certain embodiments, a heavy chain variable region (V_{H}) is positioned at the N-terminus. In certain embodiments, the variable regions are positioned from the N- to the C-terminus: V_{H}-V_{L}. In certain embodiments, a light chain variable region (V_{L}) is positioned at the N-teminus. In certain embodiments, the variable regions are positioned from the N- to the C-terminus: V_{L}-V_{H}.

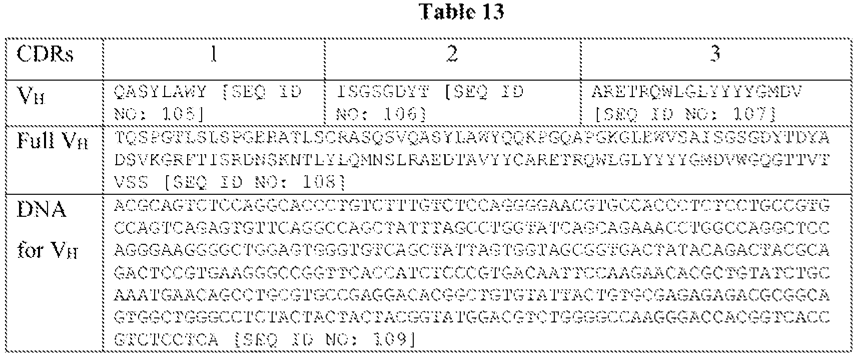

### 4.3.2. Monoclonal Antibodies

The presently disclosed subject matter provides antibodies (e.g., human antibodies, e.g., human monoclonal antibodies) that specifically bind to uPAR (e.g., human uPAR). The V_{H} amino acid sequences of anti-uPAR antibodies 3-C3-A, 3-D8-A, 3-G1-A, 3-H4-A, 4-F5-A, 4-F12-A, 4-AS-B, 05G9, 05A6, 05B2, 05F5, 05G5, and 07B3 are set forth in SEQ ID NOs: 7, 15, 23, 31, 41, 50, 59, 69, 78, 86, 95, 101, and 108, respectively. The V_{L} amino acid sequences of 3-C3-A, 3-D8-A, 3-G1-A, 3-H4-A, 4-F5-A, 4-F12-A, 4-A5-B, , 05G9, 05A6, 05B2, 05F5. and 05G5 are set forth in SEQ ID NOs: 8, 16, 24, 32, 42, 51, 60, 70, 79, 87. 96, and 102, respectively.

Given that each of 3-C3-A, 3-D8-A, 3-G1-A, 3-H4-A, 4-F5-A, 4-F12-A, 4-A5-B, 05G9, 05A6, 05B2, 05F5, 05G5, and 07B3 antibodies can bind to uPAR, the V_{H} and V sequences can be "mixed and matched" to create other anti-uPAR binding molecules. uPAR binding of such "mixed and matched" antibodies can be tested using the binding assays known in the art, including for example, ELISAs, Western blots. RIAs, Biacore analysis. Preferably, when V_{H} and V_{L} chains are mixed and matched, a V_{H} sequence from a particular V_{H}/V_{L} pairing is replaced with a structurally similar V_{H} sequence. Likewise, a V_{L} sequence from a particular V_{H}/V_{L} pairing is replaced with a structurally similar V_{L} sequence.

In certain embodiments, the presently disclosed subject matter provides an antibody or an antigen-binding fragment thereof comprising: (a) a heavy chain variable region (V_{H}) comprising an amino acid sequence selected from SEQ ID NOs: 7, 15, 23. 31, 41, 50, 59, 69, 78, 86, 95, 101, and 108; and (b) a light chain variable region (V_{L}) comprising an amino acid sequence selected from SEQ ID NOs: 8, 16, 24, 32, 42, 51, 60, 70, 79, 87, 96, and 102; wherein the antibody or antigen-binding fragment specifically binds to uPAR, e.g., human uPAR. In certain embodiments, the V_{H} and V_{L} are selected from the group consisting of:
(a) a heavy chain variable region comprising the amino acid sequence set forth in SEQ ID NO: 7, and a light chain variable region comprising the amino acid sequence set forth in SEQ ID NO: 8;
(b) a heavy chain variable region comprising the amino acid sequence set forth in SEQ ID NO: 15, and a light chain variable region comprising the amino acid sequence set forth in SEQ ID NO: 16;
(c) a heavy chain variable region comprising the amino acid sequence set forth in SEQ ID NO: 23, and a light chain variable region comprising the amino acid sequence set forth in SEQ ID NO: 24;
(d) a heavy chain variable region comprising the amino acid sequence set forth in SEQ ID NO: 31, and a light chain variable region comprising the amino acid sequence set forth in SEQ ID NO: 32;
(e) a heavy chain variable region comprising the amino acid sequence set forth in SEQ ID NO: 41, and a light chain variable region comprising the amino acid sequence set forth in SEQ ID NO: 42;
(f) a heavy chain variable region comprising the amino acid sequence set forth in SEQ ID NO: 50, and a light chain variable region comprising the amino acid sequence set forth in SEQ ID NO: 51;
(g) a heavy chain variable region comprising the amino acid sequence set forth in SEQ ID NO: 59, and a light chain variable region comprising the amino acid sequence set forth in SEQ ID NO: 60;
(h) a heavy chain variable region comprising the amino acid sequence set forth in SEQ ID NO: 69, and a light chain variable region comprising the amino acid sequence set forth in SEQ ID NO: 70;
(i) a heavy chain variable region comprising the amino acid sequence set forth in SEQ ID NO: 78, and a light chain variable region comprising the amino acid sequence set forth in SEQ ID NO: 79;
(j) a heavy chain variable region comprising the amino acid sequence set forth in SEQ ID NO: 86, and a light chain variable region comprising the amino acid sequence set forth in SEQ ID NO: 87;
(k) a heavy chain variable region comprising the amino acid sequence set forth in SEQ ID NO: 95, and a light chain variable region comprising the amino acid sequence set forth in SEQ ID NO: 96; and
(l) a heavy chain variable region comprising the amino acid sequence set forth in SEQ ID NO: 101, and a light chain variable region comprising the amino acid sequence set forth in SEQ ID NO: 102.

In certain embodiments, the presently disclosed subject matter provides antibodies or antigen-binding fragments thereof that comprise the heavy chain and light chain CDR1s, CDR2s and CDR3s of 3-C3-A, 3-D8-A, 3-G1-A, 3-H4-A, 4-F5-A, 4-F12-A, 4-A5-B, 05G9, 05A6, 05B2, 05F5, 05G5, and 07B3.

The amino acid sequences of the V_{H} CDR1s of 3-C3-A, 3-D8-A, 3-G1-A, 3-H4-A, 4-F5-A, 4-F12-A, 4-A5-B, 05G9, 05A6, 05B2, 05F5, 05G5, and 07B3 are shown in SEQ ID NOs: 1, 11, 19, 19, 35, 45, 54, 63, 73, 45, 90, 45, and 105, respectively. The amino acid sequences of the V_{H} CDR2s of 3-C3-A, 3-D8-A, 3-G1-A, 3-H4-A, 4-F5-A, 4-F12-A, 4-A5-B, 05G9, 05A6, 05B2, 05F5, 05G5, and 07B3 antibodies set forth in SEQ ID NOs: 2, 2, 2, 27, 36, 46, 55, 64, 74, 82, 91, 46, and 106, respectively. The amino acid sequences of the V_{H} CDR3s of 3-C3-A, 3-D8-A, 3-G1-A, 3-H4-A, 4-F5-A, 4-F12-A, 4-A5-B, 05G9, 05A6, 05B2, 05F5, 05G5, and 07B3 set forth in SEQ ID NOs: 3, 12, 20, 28, 37, 47, 56, 65, 75, 83, 92, 99, and 107, respectively.

The amino acid sequences of the V_{L} (CDR1s of 3-C3-A, 3-D8-A, 3-C1-A, 3-H4-A, 4-FS-A, 4-F12-A, 4-A5-B, 05G9, 05A6, 05B2, 05F5, and 05G5 are set forth in SEQ ID NOs: 4, 13, 21, 29, 38, 48, 57, 66, 76, 84, 93, and 66 respectively. The amino acid sequences of the V_{L} CDR2s of 3-C3-A, 3-D8-A, 3-G1-A, 3-H4-A, 4-FS-A, 4-F12-A, 4-A5-B, 05G9, 05A6, 05B2, 05F5. and 05G5 are set forth in SEQ ID NOs: 5, 5, 5, 5, 39, 5, 5, 67, 67, 67, 67, and 67, respectively. The amino acid sequences of the V_{L} CDR3s of 3-C3-A. 3-DS-A, 3-G1-A, 3-H4-A, 4-F5-A, 4-F12-A, 4-A5-B, 05G9, 05A6, 05B2, 05F5. and 05G5 are set forth in SEQ ID NOs: 6, 14, 22, 30, 40, 49, 58, 68, 77, 85, 94, and 100, respectively. The CDR regions are delineated using the IMGT system. In certain embodiments, the CDR regions are delineated using the IMGT numbering system accessible at http://www.imgt.org/IMGT_vquest/input.

Given that each of these antibodies or antigen-binding fragments thereof can bind to uPAR and that antigen-binding specificity is provided primarily by the CDR1. CDR2, and CDR3 regions, the V_{H} CDR1, CDR2, and CDR3 sequences and V_{L} CDR1, CDR2, and CDR3 sequences can be "mixed and matched" (i.e., CDRs from different antibodies can be mixed and match, although each antibody must contain a V_{H} CDR1, CDR2, and CDR3 and a V_{L} CDR1, CDR2, and CDR3) to create other anti-uPAR binding molecules. uPAR binding of such "mixed and matched" antibodies can be tested using the binding assays described above. When V_{H} CDR sequences are mixed and matched, the CDR1, CDR2 and/or CDR3 sequence from a particular V_{H} sequence is replaced with a structurally similar CDR sequence(s). Likewise, when V_{L} CDR sequence are mixed and matched, the CDR1, CDR2 and/or CDR3 sequence from a particular V_{L} sequence preferably is replaced with a structurally similar CDR sequence(s). It will be readily apparent to the ordinarily skilled artisan that novel V_{H} and V_{L} sequences can be created by substituting one or more V_{H} and/or V_{L} CDR region sequences with structurally similar sequences from the CDR sequences of the antibodies or antigen-binding fragments thereof disclosed herein 3-C3-A, 3-D8-A, 3-G1-A, 3-H4-A, 4-F5-A, 4-F12-A, 4-A5-B, 0509, 05A6, 05B2, 05F5. 05G5. and 07B3.

In certain embodiments, the presently disclosed subject matter provides an antibody or an antigen-binding fragment thereof comprising:
(a) a heavy chain variable region CDR1 comprising an amino acid sequence selected from SEQ ID NO: 1, SEQ ID NO: 11, SEQ ID NO: 19, SEQ ID NO: 35, SEQ ID NO: 45, SEQ ID NO: 54, SEQ ID NO: 63, SEQ ID NO: 73, SEQ ID NO: 90. or SEQ ID NO: 105;
(b) a heavy chain variable region CDR2 comprising an amino acid sequence selected from SEQ ID NO: 2, SEQ ID NO: 27. SEQ ID NO: 36, SEQ ID NO: 46, SEQ ID NO: 55, SEQ ID NO: 64, SEQ ID NO: 74, SEQ ID NO: 82, SEQ ID NO: 91, or SEQ ID NO: 106;
(c) a heavy chain variable region CDR3 comprising an amino acid sequence selected from SEQ ID NO: 3, SEQ ID NO: 12, SEQ ID NO: 20, SEQ ID NO: 28, SEQ ID NO: 37, SEQ ID NO: 47, SEQ ID NO: 56, SEQ ID NO: 65, SEQ ID NO: 75, SEQ ID NO: 83, SEQ ID NO: 92, SEQ ID NO: 99, or SEQ ID NO: 107;
(d) a light chain variable region CDR1 comprising an amino acid sequence selected from SEQ ID NO: 4, SEQ ID NO: 13, SEQ ID NO: 21, SEQ ID NO: 29, SEQ ID NO: 38, SEQ ID NO: 48, SEQ ID NO: 57, SEQ ID NO: 66, SEQ ID NO: 76, SEQ ID NO: 84, or SEQ ID NO: 93;
(e) a light chain variable region CDR2 comprising an amino acid sequence selected from SEQ ID NO: 5, SEQ ID NO: 39, or SEQ ID NO: 67; and
(f) a light chain variable region CDR3 comprising an amino acid sequence selected from SEQ ID NO: 6, SEQ ID NO: 14, SEQ ID NO: 22, SEQ ID NO: 30, SEQ ID NO: 40, SEQ ID NO: 49, SEQ ID NO: 58, SEQ ID NO: 68, SEQ ID NO: 77, SEQ ID NO: 85, SEQ ID NO: 94, or SEQ ID NO: 100.

In certain embodiments, the antibody or antigen-binding fragment thereof comprises:
(a) a heavy chain variable region CDR1 comprising the amino acid sequence set forth in SEQ ID NO: 1;
(b) a heavy chain variable region CDR2 comprising the amino acid sequence set forth in SEQ ID NO: 2;
(c) a heavy chain variable region CDR3 comprising the amino acid sequence set forth in SEQ ID NO: 3;
(d) a light chain variable region CDR1 comprising the amino acid sequence set forth in SEQ ID NO: 4;
(e) a light chain variable region CDR2 comprising the amino acid sequence set forth in SEQ ID NO: 5; and
(f) a light chain variable region CDR3 comprising the amino acid sequence set forth in SEQ ID NO: 6.

In certain embodiments, the antibody or antigen-binding fragment comprises:
(a) a heavy chain variable region CDR1 comprising the amino acid sequence set forth in SEQ ID NO: 11;
(b) a heavy chain variable region CDR2 comprising the amino acid sequence set forth in SEQ ID NO: 2;
(c) a heavy chain variable region CDR3 comprising the amino acid sequence set forth in SEQ ID NO: 12;
(d) a light chain variable region CDR1 comprising the amino acid sequence set forth in SEQ ID NO: 13;
(e) a light chain variable region CDR2 comprising the amino acid sequence set forth in SEQ ID NO: 5; and
(f) a light chain variable region CDR3 comprising the amino acid sequence set forth in SEQ ID NO: 14.

In certain embodiments, the antibody or antigen-binding fragment thereof comprises:
(a) a heavy chain variable region COR1 comprising the amino acid sequence set forth in SEQ ID NO: 19;
(b) a heavy chain variable region CDR2 comprising the amino acid sequence set forth in SEQ ID NO: 2;
(c) a heavy chain variable region CDR3 comprising the amino acid sequence set forth in SEQ ID NO: 20;
(d) a light chain variable region CDR1 comprising the amino acid sequence set forth in SEQ ID NO: 21;
(e) a light chain variable region CDR2 comprising the amino acid sequence set forth in SEQ ID NO: 5; and
(f) a light chain variable region CDR3 comprising the amino acid sequence set forth in SEQ ID NO: 22.

In certain embodiments, the antibody or antigen-binding fragment thereof comprises:
(a) a heavy chain variable region CDR1 comprising the amino acid sequence set forth in SEQ ID NO: 19;
(b) a heavy chain variable region CDR2 comprising the amino acid sequence set forth in SEQ ID NO: 27;
(c) a heavy chain variable region CDR3 comprising the amino acid sequence set forth in SEQ ID NO: 28;
(d) a light chain variable region CDR1 comprising the amino acid sequence set forth in SEQ ID NO: 29;
(e) a light chain variable region CDR2 comprising the amino acid sequence set forth in SEQ ID NO: 5; and
(f) a light chain variable region CDR3 comprising the amino acid sequence set forth in SEQ ID NO: 30.

In certain embodiments, the antibody or antigen-binding fragment thereof comprises:
(a) a heavy chain variable region CDR1 comprising the amino acid sequence set forth in SEQ ID NO: 35;
(b) a heavy chain variable region CDR2 comprising the amino acid sequence set forth in SEQ ID NO: 36;
(c) a heavy chain variable region CDR3 comprising the amino acid sequence set forth in SEQ ID NO: 37;
(d) a light chain variable region CDR1 comprising the amino acid sequence set forth in SEQ ID NO: 38;
(e) a light chain variable region CDR2 comprising the amino acid sequence set forth in SEQ ID NO: 39; and
(f) a light chain variable region CDR3 comprising the amino acid sequence set forth in SEQ ID NO: 40.

In certain embodiments, the antibody or antigen-binding fragment thereof comprises:
(a) a heavy chain variable region CDR1 comprising the amino acid sequence set forth in SEQ ID NO: 45;
(b) a heavy chain variable region CDR2 comprising the amino acid sequence set forth in SEQ ID NO: 46;
(c) a heavy chain variable region CDR3 comprising the amino acid sequence set forth in SEQ ID NO: 47;
(d) a light chain variable region CDR1 comprising the amino acid sequence set forth in SEQ ID NO: 48;
(e) a light chain variable region CDR2 comprising the amino acid sequence set forth in SEQ ID NO: 5; and
(f) a light chain variable region CDR3 comprising the amino acid sequence set forth in SEQ ID NO: 49.

In certain embodiments, the antibody or antigen-binding fragment thereof comprises:
(a) a heavy chain variable region CDR1 comprising the amino acid sequence set forth in SEQ ID NO: 54;
(b) a heavy chain variable region CDR2 comprising the amino acid sequence set forth in SEQ ID NO: 55;
(c) a heavy chain variable region CDR3 comprising the amino acid sequence set forth in SEQ ID NO: 56;
(d) a light chain variable region CDR1 comprising the amino acid sequence set forth in SEQ ID NO: 57;
(e) a light chain variable region CDR2 comprising the amino acid sequence set forth in SEQ ID NO: 5; and
(f) a light chain variable region CDR3 comprising the amino acid sequence set forth in SEQ ID NO: 58.

In certain embodiments, the antibody or antigen-binding fragment thereof comprises:
(a) a heavy chain variable region CDR1 comprising the amino acid sequence set forth in SEQ ID NO: 63;
(b) a heavy chain variable region CDR2 comprising the amino acid sequence set forth in SEQ ID NO: 64;
(c) a heavy chain variable region CDR3 comprising the amino acid sequence set forth in SEQ ID NO: 65;
(d) a light chain variable region CDR1 comprising the amino acid sequence set forth in SEQ ID NO: 66;
(e) a light chain variable region CDR2 comprising the amino acid sequence set forth in SEQ ID NO: 67; and
(f) a light chain variable region CDR3 comprising the amino acid sequence set forth in SEQ ID NO: 68.

In certain embodiments, the antibody or antigen-binding fragment thereof comprises:
(a) a heavy chain variable region CDR1 comprising the amino acid sequence set forth in SEQ ID NO: 73;
(b) a heavy chain variable region CDR2 comprising the amino acid sequence set forth in SEQ ID NO: 74;
(c) a heavy chain variable region CDR3 comprising the amino acid sequence set forth in SEQ ID NO: 75;
(d) a light chain variable region CDR1 comprising the amino acid sequence set forth in SEQ ID NO: 76;
(e) a light chain variable region CDR2 comprising the amino acid sequence set forth in SEQ ID NO: 67; and
(f) a light chain variable region CDR3 comprising the amino acid sequence set forth in SEQ ID NO: 77.

In certain embodiments, the antibody or antigen-binding fragment thereof comprises:
(a) a heavy chain variable region CDR1 comprising the amino acid sequence set forth in SEQ ID NO: 45;
(b) a heavy chain variable region CDR2 comprising the amino acid sequence set forth in SEQ ID NO: 82;
(c) a heavy chain variable region CDR3 comprising the amino acid sequence set forth in SEQ ID NO: 83;
(d) a light chain variable region CDR1 comprising the amino acid sequence set forth in SEQ ID NO: 84;
(e) a light chain variable region CDR2 comprising the amino acid sequence set forth in SEQ ID NO: 67; and
(f) a light chain variable region CDR3 comprising the amino acid sequence set forth in SEQ ID NO: 85.

In certain embodiments, the antibody or antigen-binding fragment thereof comprises:
(a) a heavy chain variable region CDR1 comprising the amino acid sequence set forth in SEQ ID NO: 90;
(b) a heavy chain variable region CDR2 comprising the amino acid sequence set forth in SEQ ID NO: 91;
(c) a heavy chain variable region CDR3 comprising the amino acid sequence set forth in SEQ ID NO: 92;
(d) a light chain variable region CDR1 comprising the amino acid sequence set forth in SEQ ID NO: 93;
(e) a light chain variable region CDR2 comprising the amino acid sequence set forth in SEQ ID NO: 67; and
(f) a light chain variable region CDR3 comprising the amino acid sequence set forth in SEQ ID NO: 94.

In certain embodiments, the antibody or antigen-binding fragment thereof comprises:
(a) a heavy chain variable region CDR1 comprising the amino acid sequence set forth in SEQ ID NO: 45;
(b) a heavy chain variable region CDR2 comprising the amino acid sequence set forth in SEQ ID NO: 46;
(c) a heavy chain variable region CDR3 comprising the amino acid sequence set forth in SEQ ID NO: 99;
(d) a light chain variable region CDR1 comprising the amino acid sequence set forth in SEQ ID NO: 66;
(e) a light chain variable region CDR2 comprising the amino acid sequence set forth in SEQ ID NO: 67; and
(f) a light chain variable region CDR3 comprising the amino acid sequence set forth in SEQ ID NO: 100.

The constant region/framework region of the anti-uPAR antibodies disclosed herein can be altered, for example, by amino acid substitution, to modify the properties of the antibody (e.g., to increase or decrease one or more of: antigen binding affinity, Fc receptor binding, antibody carbohydrate, for example, glycosylation, fucosylation etc., the number of cysteine residues, effector cell function, effector cell function, complement function or introduction of a conjugation site).

In certain embodiments, a presently disclosed anti-uPAR antibody is a fully-human antibody, e.g., any one of 3-C3-A, 3-D8-A, 3-G1-A, 3-H4-A, 4-F5-A, 4-F12-A, 4-A5-B, 05G9, 05A6, 05B2, 05F5, 05G5, and 07B3. Fully-human mAbs, when administered to humans, causing serious side effects, including anaphylaxis and hypersensitivity reactions.

The use of phage display libraries has made it possible to select large numbers of antibody repertoires for unique and rare Abs against very defined epitopes (for more details on phage display see McCafferty et al., Phage antibodies: filamentous phage displaying antibody variable domains. Nature, 348: 552-554.) The rapid identification of human Fab or single chain Fv (scFv) fragments highly specific for tumor antigen-derived peptide-MHC complex molecules has thus become possible. In addition, by engineering full-length monoclonal antibody (mAb) using the Fab fragments, it is possible to directly generate a therapeutic human mAb, bypassing months of time-consuming work, normally needed for developing therapeutic mAbs. The presently disclosed subject matter involves the development of a fully human mAb that recognizes, for example, a human uPAR polypeptide (e.g., a polypeptide having the amino acid sequence set forth in SEQ ID NO: 116) for cancer therapy.

### 4.3.3. Homologous Antibodies

In certain embodiments, a presently disclosed antibody or antigen-binding fragment thereof comprises heavy and light chain variable regions comprising amino acid sequences that are homologous or identical to the amino acid sequences of the antibodies described herein (e.g., 3-C3-A, 3-D8-A, 3-G1-A, 3-H4-A, 4-F5-A, 4-F12-A, 4-A5-B, 05G9, 05A6, 05B2, 05F5, 05G5. and 07B3 antibodies), and wherein the antibodies or antigen-binding fragments thereof retain the desired functional properties of the anti-uPAR antibodies or antigen-binding fragments thereof of the presently disclosed subject matter.

For example, the presently disclosed subject matter provides an antibody or an antigen-binding fragment thereof, comprising a heavy chain variable region and a light chain variable region, wherein:
(a) the heavy chain variable region comprises an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108;
(b) the light chain variable region comprises an amino acid sequence that is at least about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98% or about 99% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

In certain embodiments, the V_{H} and or V_{L} amino acid sequences can be at least about 80%, about 81%, about 82%, about 83%, about 94%, about 85%, about 86%, about 87%, about 88%, about 89%, about 90%, about 91%, about 92%, about 93%, about 94%, about 95%, about 96%, about 97%, about 98% or about 99% homologous or identical to the sequences set forth above. An antibody having V_{H} and V_{L} regions having high (i.e., 80% or greater) homology or identity to the V_{H} and V_{L} regions of the sequences set forth above, can be obtained by mutagenesis (e.g., site-directed or PCR-mediated mutagenesis), followed by testing of the encoded altered antibody for retained function (i.e., the binding affinity) using the binding assays described herein.

As used herein, the percent homology between two amino acid sequences is equivalent to the percent identity between the two sequences. The percent identity or homology between the two sequences is a function of the number of identical positions shared by the sequences (i.e., % homology = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent homology or identity between two amino acid sequences can be determined using the algorithm of E. Meyers and W. Miller (Comput Appl Biosci (1988);14:11-17) which has been incorporated into the ALIGN program (version 2.0), using a PAM120 weight residue table, a gap length penalty of 12 and a gap penalty of 4. In addition, the percent homology between two amino acid sequences can be determined using the Needleman and Wunsch (J Mol Biol (1970);48:444-453) algorithm which has been incorporated into the GAP program in the GCG software package (available at www.gcg.com), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the presently disclosed subject matter can further be used as a "query sequence" to perform a search against public databases to, for example, identify related sequences. Such searches can be performed using the XBLAST program (version 2.0) of Altschul et al., J Mol Biol ( 1990);215:403-10. BLAST protein searches can be performed with the XBLAST program, score = 50, word length = 3 to obtain amino acid sequences homologous to the antibody molecules of the invention. To obtain gapped alignments for comparison purposes, Gapped BLAST can be utilized as described in Altschul et al., Nucleic Acids Res (1997);25(17):3389-3402. When utilizing BLAST and Gapped BLAST programs. the default parameters of the respective programs (e.g., XBLAST and NBLAST) can be used.

### 4.3.4. Antibodies with Conservative Modifications

In certain embodiments, a presently disclosed antibody or an antigen-binding fragment thereof comprises a heavy chain variable region comprising CDR1, CDR2 and CDR3 sequences and a light chain variable region comprising CDR1, CDR2 and CDR3 sequences, wherein one or more of these CDR sequences comprise specified amino acid sequences based on the preferred antibodies described herein (e.g., 3-C3-A, 3-D8-A, 3-G1-A, 3-H4-A, 4-F5-A, 4-F12-A, 4-A5-B, 05G9, 05A6, 05B2, 05F5, 05G5, and 07B3 antibodies). or a conservative modification thereof, and wherein the antibodies retain the desired functional properties of the anti-uPAR antibodies or antigen-binding fragments thereof of the presently disclosed subject matter. The presently disclosed subject matter provides an antibody or an antigen-binding fragment thereof, comprising a heavy chain variable region comprising CDR1, CDR2, and CDR3 sequences and a light chain variable region comprising CDR1, CDR2, and CDR3 sequences, wherein:
(a) the heavy chain variable region CDR3 sequence comprises an amino acid sequence selected from the amino acid sequences of SEQ ID NOs: 3, 12, 20, 28. 37, 47, 56, 65, 75, 83. 92, 99, and 107, and conservative modifications thereof;
(b) the light chain variable region CDR3 sequence comprises an amino acid sequence selected from the amino acid sequence of SEQ ID NOs: 6, 14, 22, 30, 40, 49, 58, 68, 77, 85, 94, and 100, and conservative modifications thereof.

In certain embodiments, the heavy chain variable region CDR3 sequence comprises an amino acid sequence selected from the amino acid sequences of SEQ ID NOs: 3. 12, 20. 28, 37, 47. 56, 65, 75, 83, 92. 99, and 107, and conservative modifications thereof; and the light chain variable region CDR3 sequence comprises an amino acid sequence selected from the amino acid sequences of SEQ ID NOs: 6. 14, 22, 30, 40, 49, 58, 68, 77, 85, 94. and 100, and conservative modifications thereof.

In certain embodiments, the heavy chain variable region CDR2 sequence comprises an amino acid sequence selected from the amino acid sequences of SEQ ID NOs: 2, 27, 36, 46, 55, 64, 74, 82, 91, and 106, and conservative modifications thereof, and the light chain variable region CDR2 sequence comprises an amino acid sequence selected from the amino acid sequences of SEQ ID NOs: 5, 39, and 67, and conservative modifications thereof.

In certain embodiments, the heavy chain variable region CDR1 sequence comprises an amino acid sequence selected from the amino acid sequences of SEQ ID NOs: 1, 11, 19, 19, 35, 45, 54, 63, 73, 90, and 105, and conservative modifications thereof; and the light chain variable region CDR1 sequence comprises an amino acid sequence selected from the amino acid sequences of SEQ ID NOs: 4, 13, 21, 29, 38, 48, 57, 66, 76. 84, and 93, and conservative modifications thereof.

As used herein, the term "conservative sequence modifications" is intended to refer to amino acid modifications that do not significantly affect or alter the binding characteristics of the antibody containing the amino acid sequence. Such conservative modifications include amino acid substitutions, additions and deletions. Modifications can be introduced into an antibody of the present disclosure by standard techniques known in the art, such as site-directed mutagenesis and PCR-mediated mutagenesis.

Conservative amino acid substitutions are ones in which the amino acid residue is replaced with an amino acid residue having a similar side chain. Families of amino acid residues having similar side chains have been defined in the art. Exemplary conservative amino acid substitutions are shown in Table 14. Amino acid substitutions may be introduced into an antibody of interest and the products screened for a desired activity, e.g., retained/improved antigen binding, decreased immunogenicity, or improved ADCC or CDC. In certain embodiments, a sequence disclosed herein, e.g., a CDR sequence, a V_{H} sequence or a V_{L} sequence, can have up to about one, up to about two, up to about three, up to about four, up to about five, up to about six, up to about seven, up to about eight, up to about nine or up to about ten amino acid residues that are modified and/or substituted.

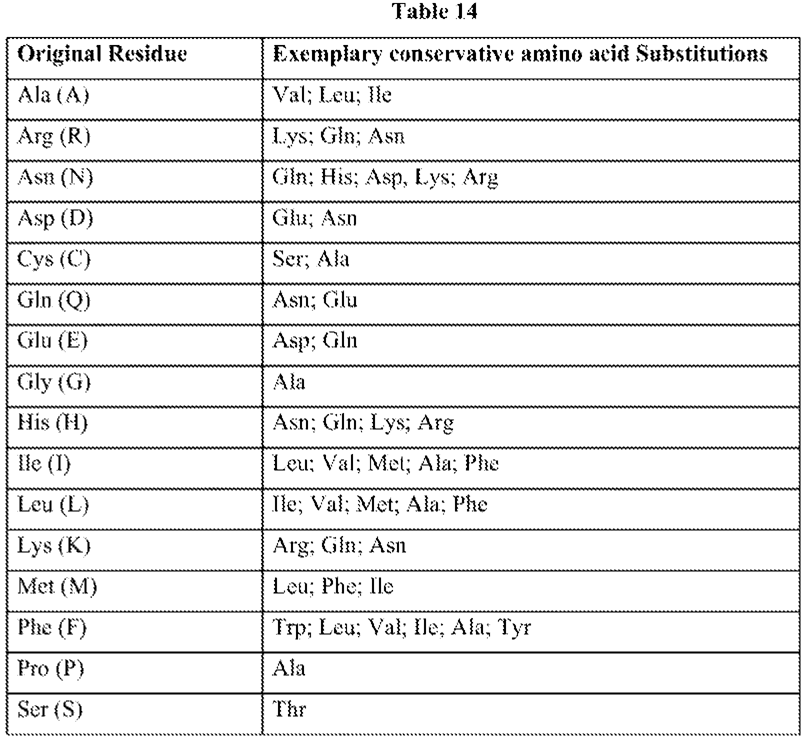

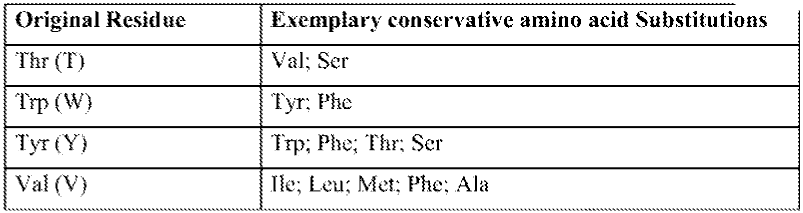

Amino acids may be grouped according to common side-chain properties:
- hydrophobic: Norleucine, Met, Ala, Val. Leu, Ile;
- neutral hydrophilic: Cys, Ser, Thr, Asn, Gln;
- acidic: Asp, Glu;
- basic: His. Lys. Arg:
- residues that influence chain orientation: Gly. Pro;
- aromatic: Trp. Tyr, Phe.

Non-conservative substitutions will entail exchanging a member of one of these classes for another class.

### 4.3.5. Anti-uPAR Antibodies that Cross-compete for Binding to uPAR with Anti-uPAR Antibodies of the Invention

The presently disclosed subject matter provides antibodies or antigen-binding fragments thereof that cross-compete with any of the disclosed anti-uPAR antibodies for binding to uPAR (e.g., human uPAR). For example, and not by way of limitation, the cross-competing antibodies can bind to the same epitope region, e.g., same epitope, adjacent epitope, or overlapping as any of the anti- uPAR antibodies or antigen-binding fragments thereof of the presently disclosed subject matter. In certain embodiments, the reference antibody or reference antigen-binding fragments thereof for cross-competition studies can be any one of the anti-uPAR antibodies or antigen-binding fragments thereof disclosed herein, e.g., 3-C3-A, 3-D8-A. 3-G1-A, 3-H4-A, 4-F5-A, 4-F12-A, 4-A5-B, 05G9, 05A6, 05B2, 05F5, 05G5, and 07B3 antibodies.

Such cross-competing antibodies can be identified based on their ability to cross-compete with any one of the presently disclosed anti- uPAR antibodies or antigen-binding fragments thereof in standard uPAR binding assays. For example, Biacore analysis, ELISA assays or flow cytometry can be used to demonstrate cross-competition with the antibodies of the presently disclosed subject matter. The ability of a test antibody to inhibit the binding of, for example, any one of the presently disclosed anti-uPAR antibodies (e.g., 3-C3-A, 3-D8-A, 3-G1-A, 3-H4-A, 4-F5-A, 4-F12-A, 4-A5-B, 05G9, 05A6, 05B2, 05F5, 05G5, and 07B3 antibodies) to uPAR (e.g., human uPAR) demonstrates that the test antibody can compete with any one of the presently disclosed anti-uPAR antibodies or antigen-binding fragments thereof for binding to uPAR (e.g., human uPAR) and thus binds to the same epitope region on uPAR (e.g., human uPAR) as any one of the presently disclosed anti-uPAR antibodies or antigen-binding fragments thereof. In certain embodiments, the cross-competing antibody or antigen-binding fragment thereof binds to the same epitope on uPAR (e.g., human uPAR) as any one of the presently disclosed anti-uPAR antibodies or antigen-binding fragments thereof.

### 4.3.6. Characterization of Antibody Binding to Antigen

Antibodies or antigen-binding fragments thereof of the presently disclosed subject can be tested for binding to uPAR by, for example, standard ELISA. To determine if the selected anti-uPAR antibodies bind to unique epitopes, each antibody can be biotinylated using commercially available reagents (Pierce, Rockford, IL). Competition studies using unlabeled monoclonal antibodies and biotinylated monoclonal antibodies can be performed using uPAR coated-ELISA plates as described above. Biotinylated mAb binding can be detected with a strep-avidin-alkaline phosphatase probe.

To determine the isotype of purified antibodies, isotype ELISAs can be performed using reagents specific for antibodies of a particular isotype. Anti-uPAR human IgGs can be further tested for reactivity with uPAR antigen by Western blotting.

In certain embodiments, the K_{D} is measured by a radiolabeled antigen binding assay (RIA). In certain embodiments, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J Mol Biol (1999);293:865-881).

In certain embodiments, the K_{D} is measured using a BIACORE^{®} surface plasmon resonance assay. For example, an assay using a BIACORE^{®}-2000 or a BIACORE ^{®}-3000 (BIAcore, Inc., Piscataway, NJ)

### 4.3.7. Immunoconjugates

The presently disclosed subject provides an anti-uPAR antibody or an antigen-binding fragment thereof, conjugated to a therapeutic moiety, such as a cytotoxin, a drug (e.g., an immunosuppressant) or a radiotoxin. Such conjugates are referred to herein as "immunoconjugates". Immunoconjugates that include one or more cytotoxins are referred to as "immunotoxins." A cytotoxin or cytotoxic agent includes any agent that is detrimental to (e.g., kills) cells. Non-limiting Examples of cytotoxins include taxol (such as ricin, diphtheria, gelonin), cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, calecheamicin, aureastatin, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C. and cis- dichlorodiamine platinum (11) (DDP) cisplatin), anthracyclines (e.g., daunorubicin (formerly daunomycin) and doxorubicin), antibiotics (e.g., dactinomycin (formerly actinomycin), bleomycin, mithramycin, anthramycin (AMC)), hyponicthylating agents (azacytidine and decitabine), and anti-mitotic agents (e.g., vincristine and vinblastine).

Other examples of therapeutic cytotoxins that can be conjugated to an anti-uPAR antibody disclosed herein include duocarmycins, calichcamicins, maytansines and auristatins, and derivatives thereof. Cytotoxins can be conjugated to an anti-uPAR antibody or an antigen-binding fragment thereof disclosed herein using linker technology available in the art. Examples of linker types that have been used to conjugate a cytotoxin to an antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (e.g., cathepsins B, C, D). For further discussion of types of cytotoxins, linkers and methods for conjugating therapeutic agents to antibodies, see also Saito, G. et al. (2003) Adv. Drug Deliv. Rev. 55:199-215; Trail, P.A. et al. (2003) Cancer Immunol. Immunother. 52:328-337; Payne, G. (2003) Cancer Cell 3:207-212; Allen, T.M. (2002) Nat. Rev. Cancer 2:750-763; Pastan, I. and Kreitman, R. J. (2002) Curr. Opin. Investig. Drugs 3:1089-1091; Senter, P.D. and Springer, C.J. (2001) Adv. Drug Deliv. Rev. 53:247-264.

In addition, anti-uPAR antibodies or antigen-binding fragments thereof of the presently disclosed subject matter can be conjugated to an agent that induces senescence. In certain embodiments, the agent that induces senescence is a setiogenic agent. Non-limiting examples of senescence-inducing agents include Cdk4/6 inhibitors. Cdk2 inhibitors, MEK inhibitors, inhibitors of CDC7 and chemotherapy drugs. Non-limiting examples of MEK inhibitors include trametinib, cobimetinib, binimetinib, selumctinib. PD-325901, TAK-733, CI-1040 (PD 184352), PD0325901, MEK 162, AZD8330, GDC-0623, refametinib, pimasertib, R04987655, R05126766, WX-554, HL-085, CInQ-03, G-573, PD184161, PD318088, PD98059, R05068760, U0126, and SL327. Non-limiting examples of CDK4/6 inhibitors include palbociclib, ribociclib, and abemaciclib. Non-limiting examples of chemotherapy drugs include cisplatin, doxorubicin, cyclophosphamide, and etoposide.

Anti-uPAR antibodies or antigen-binding fragments thereof of the presently disclosed subject matter also can be conjugated to a radioactive isotope to generate cytotoxic radiopharmaceuticals, also referred to as radioimmunoconjugates. Non-limiting examples of radioactive isotopes that can be conjugated to antibodies for use diagnostically or therapeutically include ⁹⁰Y, ¹³¹I, ²²⁵Ac, ²¹³Bi, ²²³Ra and ²²⁷Th. Methods for preparing radioimmunconjugates are established in the art. Examples of radioimmunoconjugates are commercially available, including Zevalin^{™} (IDEC Pharmaceuticals) and Bexxar^{™} (Corixa Pharmaceuticals), and similar methods can be used to prepare radioimmunoconjugates using the antibodies of the invention.

Anti-uPAR antibodies **or** antigen-binding fragments thereof of the presently disclosed subject matter also can be conjugated to an imagining agent or probe, e.g., for use in imagining techniques, e.g., ImmunoPET. In certain embodiments, a presently disclosed anti-uPAR antibody or antigen-binding fragment thereof is conjugated to an immunoPET probe, e.g., ⁹Zr-Df, and ⁸⁹Zr.

The antibody conjugates of the presently disclosed subject matter can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor (TNF) or interferon-y; or, biological response moditiers such as, for example, lymphokines, interleukin-1 (IL-1), interleukin-2 (IL-2), interleukin-6 (IL-6), granulocyte macrophage colony stimulating factor (GM-CSF), granulocyte colony stimulating factor (G-CSF), or other growth factors.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy. Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications. Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

### 4.3.8. Multi-specific Molecules

The presently disclosed subject matter provides multi-specific molecules comprising an anti-uPAR antibody, or a fragment thereof, disclosed herein. A presently disclosed or an antigen-binding fragment thereof can be derivatized or linked to one more functional molecules, e.g., one or more peptides or proteins (e.g., one or more antibodies or ligands for a receptor) to generate a multi-specific molecule that binds to two or more different binding sites or target molecules. The presently disclosed anti-uPAR antibody or antigen-binding fragment thereof can in fact be derivatized or linked to more than one other functional molecules to generate multi-specific molecules that bind to more than two different binding sites and/or target molecules. To create a multi-specific molecule, a presently disclosed anti-uPAR antibody or an antigen-binding fragment thereof can be functionally linked (e.g., by chemical coupling, genetic fusion, noncovalent association or otherwise) to one or more other binding molecules. such as another antibody, antibody fragment, peptide or binding mimetic, such that a bispecific molecule.

In certain embodiments, the multi-specific molecule is a bispecific molecule. In certain embodiments, the bispecific molecules comprises at least a first binding specificity for uPAR and a second binding specificity for a second target epitope region. The second target epitope region can be a uPAR epitope, or a non-uPAR epitope, e.g., a different antigen. In certain embodiments, the multi-specific molecule comprises a first binding specificity for uPAR, a second binding specificity for a second target, and a third binding specificity for a third target. In certain embodiments, In certain embodiments, the second target is an antigen expressed on the surface of an immune cell (e.g., a T cell, or a human immune effector cell). In certain embodiments, the multi-specific molecule is capable of recruiting the activity of that immune effector cell by specifically binding to the effector antigen on the human immune effector cell, thereby enhancing effector function. In certain embodiments, the third target is an antigen expressed on a senescent cell.

The multi-specific molecules of the presently disclosed subject matter can be prepared by conjugating the constituent binding specificities using methods known in the art. For example, each binding specificity of the multi-specific molecule can be generated separately and then conjugated to one another. When the binding specificities are proteins or peptides, a variety of coupling or cross-linking agents can be used for covalent conjugation. Non-limiting examples of cross-linking agents include protein A, carbodiimide, N-succinimidyl-S-acetyl-thioacetate (SATA), 5, 5'-dithiobis(2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl) cyclohaxane-1-carboxylate (sulfo-SMCC) (see e.g., Karpovsky et al. (1984) J. Exp. Med. 160:1686; Liu, MA et al. (1985) Proc. Natl. Acad. Sci. USA 82:8648). Other methods include those described in Paulus (1985) Behring Ins. Mitt. No. 78, 118-132; Brennan et al. (1985) Science 229:81-83), and Glennie et al. (1987) J. Immunol. 139: 2367-2375). Conjugating agents can be SATA and sulfo-SMCC, both available from Pierce Chemical Co. (Rockford, IL).

When the binding specificities are antibodies, they can be conjugated via sulthydryl bonding of the C-terminus hinge regions of the two heavy chains. In certain embodiments, the hinge region is modified to contain an odd number of sulfhydryl residues, preferably one, prior to conjugation.

Alternatively, both binding specificities can be encoded in the same vector and expressed and assembled in the same host cell. This method is particularly useful where the multi-specific molecule is a mAb x mAb, mAb x Fab, Fab x F(ab'): or ligand x Fab fusion protein.

Binding of the multi-specific molecules to their specific targets can be confirmed by, for example, enzyme-linked immunosorbent assay (ELISA), radioimmunoassay (RIA), FACS analysis, bioassay (e.g., growth inhibition), or Western Blot assay. Each of these assays generally detects the presence of protein-antibody complexes of particular interest by employing a labeled reagent (e.g., an antibody) specific for the complex of interest. Alternatively, the complexes can be detected using any of a variety of other immunoassays. For example, the antibody can be radioactively labeled and used in a radioimmunoassay (RIA) (see, for example, Weintraub. B., Principles of Radioimmunoassays, Seventh Training Course on Radioligand Assay Techniques, The Endocrine Society. March. 1986, which is incorporated by reference herein). The radioactive isotope can be detected by such means as the use of a y counter or a scintillation counter or by autoradiography.

### 4.3.9. Selecting a high affinity ScFv against a uPAR polypeptide

The next step is to select a phage that binds to the target antigen of interest (e.g., uPAR) with a high binding affinity in a phage display library (e.g., a human phage display library) that either does not bind or that binds with a lower binding affinity. This can be accomplished by iterative binding of phage to the antigen, which is bound to a solid support, for example, beads or mammalian cells followed by removal of non-bound phage and by elution of specifically bound phage. In certain embodiments, antigens (e.g., uPAR) are immobilized on a surface (e.g., a polystyrene surface). The phage library is incubated with the cells, beads or other solid support and nonbinding phage is removed by washing. Clones that bind are selected and tested.

Once selected, positive scFv clones are tested for their binding to uPAR (e.g., human uPAR) on cell surfaces by flow cytometry. Briefly, phage clones are incubated with NALM6 cells over-expressing uPAR. The cells are washed and then incubated with a M13 coat protein mAb. Cells are washed again and labeled with a PE-labeled anti-mouse Fab₂ prior to flow cytometry.

In other embodiments, the anti-uPAR antibodies can comprise one or more framework region amino acid substitutions designed to improve protein stability, antibody binding, expression levels or to introduce a site for conjugation of therapeutic agents. These scFv are then used to produce recombinant human monoclonal Igs in accordance with methods known to those of skill in the art.

### 4.3.10. Engineering full length mAb using the selected ScFv fragments

Phage display technology allows for the rapid selection and production of antigen-specific scFv and Fab fragments, which are useful in and of themselves, or which can be further developed to provide complete antibodies, antigen binding proteins or antigen binding fragments thereof. Complete mAbs with Fc domains have a number of advantages over the scFv and Fab antibodies. First, only full-length Abs exert immunological function such as CDC and ADCC mediated via Fc domain. Second, bivalent mAbs offer stronger antigen-binding affinity than monomeric Fab Abs. Third, plasma half-life and renal clearance will be different with the Fab and bivalent mAb. The particular features and advantages of each can be matched to the planned effector strategy. Fourth, bivalent mAb may be internalized at different rates than scFv and Fab, altering immune function or carrier function. Alpha emitters, for example, do not need to be internalized to kill the targets, but many drugs and toxins will benefit from internalization of the immune complex. In certain embodiments, therefore, once scFv clones specific for uPAR were obtained from phage display libraries, a full-length IgG mAb using the scFv fragments was produced.

To produce recombinant human monoclonal IgG in Chinese hamster ovary (CHO) cells, a full length IgG mAb can be engineered based on a method known to those of skill in the art (Tomomatsu et al., Production of human monoclonal antibodies against FceRIa by a method combining in vitro immunization with phage display. Biosci Biotechnol Biochem 73(7): 1465-1469 2009). Briefly, antibody variable regions can be subcloned into mammalian expression vectors, with matching Lambda or Kappa light chain constant sequences and IgG1 subclass Fc (for example) (Lidija P, et al. An integrated vector system for die eukaryotic expression of antibodies or their fragments after selection from phage display libraries. Gene 1997; 187(1): 9-18; Lisa JH, et al. Crystallographic structure of an intact IgG1monoclonal antibody. Journal of Molecular Biology 1998; 275 (5): 861-872). Kinetic binding analysis (Yasmina NA, et al. Probing the binding mechanism and affinity of tanezumab, a recombinant humanized anti-NGF monoclonal antibody, using a repertoire of biosensors. Protein Science 2008; 17(8): 1326-1335) can be used to confirm specific binding of full-length IgG to uPAR, with a K_{D} in a range of subnanomolar to micromolar.

### 4.4. Nucleic Acids encoding the Antibodies or Antigen-binding Fragments

The presently disclosed subject matter provides nucleic acids encoding the anti-uPAR antibodies or antigen-binding fragments thereof disclosed herein. In certain embodiments, the nucleic acid comprises or consists of the nucleotide sequence set forth in SEQ ID NO: 10, SEQ ID NO: 18, SEQ ID NO: 26, SEQ ID NO: 34, SEQ ID NO: 44, SEQ ID NO: 53, or SEQ ID NO: 62.

Further provided are vectors comprising the presently disclosed nucleic acids. In certain embodiments, the vector is an expression vector. The presently disclosed subject matter further provides host cells comprising the vectors disclosed herein. In certain embodiments, the host cells are T cells.

### 4.5. Pharmaceutical Compositions and Methods of Treatment

The presently disclosed subject matter provides compositions comprising a presently disclosed anti-uPAR antibody or an antigen-binding fragment thereof, a presently disclosed immunoconjugate, or a presently disclosed multi-specific molecule. In certain embodiments, the composition is a pharmaceutical composition further comprising a pharmaceutically acceptable carrier.

The anti-uPAR antibodies or antigen-binding fragments thereof of the presently disclosed subject matter can be administered in the form of a composition additionally comprising a pharmaceutically acceptable carrier. Suitable pharmaceutically acceptable carriers include, for example, one or more of water, saline, phosphate buffered saline, dextrose, glycerol, ethanol and the like, as well as combinations thereof.

Pharmaceutically acceptable carriers may further comprise minor amounts of auxiliary substances such as wetting or emulsifying agents, preservatives or buffers, which enhance the shelf life or effectiveness of the binding proteins. The compositions of the injection can, as is well known in the art, be formulated so as to provide quick, sustained or delayed release of the active ingredient after administration to the mammal.

The presently disclosed subject matter provides various methods of using the anti-uPAR antibodies or antigen-binding fragments thereof, the immunoconjugate, the multi-specific molecule, and the composition disclosed herein in a therapy. The presently disclosed subject matter provides methods for treating or ameliorating a disease or disorder in a subject. In certain embodiments, the disease or disorder is associated with uPAR. In certain embodiments, the disease or disorder is associated with overexpression of uPAR. In certain embodiments, the disease or disorder is selected from the group consisting of tumors, senescence-associated pathologies, and tissue decline associated with aging. Non-limiting examples of senescence-associated pathologies include lung fibrosis, atherosclerosis, Alzheimer's disease, diabetes, osteoarthritis, liver fibrosis, chronic kidney disease, cardiac fibrosis, and Parkinson's disease.

In certain embodiments, the method comprises administering to a subject in need thereof the presently disclosed anti-uPAR antibody or antigen-binding fragment thereof, immunoconjugate, multi-specific molecule, or composition.

For treatnient, the amount of the anti-uPAR antibodies or antigen-binding fragments thereof, the immunoconjugate, or the multi-specific molecule provided herein administered is an amount effective in producing the desired effect, for example, treatment or amelioration of the diseases or disorders (e.g., tumors and senescence-associated pathologies). An effective amount can be provided in one or a series of administrations of the anti-uPAR antibodies or antigen-binding fragments thereof, the immunoconjugate, or the multi-specific molecule disclosed herein.

The anti-uPAR antibodies or antigen-binding fragments thereof, the immunoconjugate, and the multi-specific molecule of the presently disclosed subject matter can be administered by any methods known in the art, including, but not limited to, pleural administration, intravenous administration, subcutaneous administration, intranodal administration, intratumoral administration, intrathecal administration, intravitreal administration, intrapleural administration, intraperitoneal administration, and direct administration to the thymus.

In certain embodiments, the disease or disorder is a tumor. In certain embodiments, the presently disclosed anti-uPAR antibodies or antigen-binding fragments thereof, immunoconjugates, or multi-specific molecules can reduce tumor burden, reduce the number of tumor cells, reduce tumor size, and/or eradicate the tumor in the subject, and/or increase or lengthen survival of the subject.

Non-limiting examples of tumors include breast cancer (including triple negative breast cancer), endometrial cancer, ovarian cancer, colon cancer, rectal cancer, lung cancer (e.g., non-small cell lung cancer), stomach cancer, prostate cancer, gastric cancer, renal cancer, pancreatic cancer, blood cancer, cervical cancer, head and neck cancer, liver cancer (e.g., cholangiocarcinoma, hepatocellular carcinoma, and fibrolamaellar hepatocellular carcinoma), urotherial cancer, melanoma, and brain cancer (including glioblastoma multiforme). In certain embodiments, the blood cancer is selected from the group consisting of acute lymphoblastic leukemia (ALL), chronic lymphocytic leukemia (CLL), acute myeloid leukemia (AML), myelofibrosis, polycythemia vera, myelodysplastic syndrome, and erythroleukemia. In certain embodiments, the tumor is cancer. In certain embodiments, the cancer is a relapsed or refractory cancer. In certain embodiments, the cancer is resistant to a cancer therapy, e.g., chemotherapy.

Furthermore, the presently disclosed subject matter provides methods of increasing production of an immune-activating cytokine in response to a tumor cell in a subject. In certain embodiments, the method comprises administering to the subject the presently disclosed anti-uPAR antibody or antigen-binding fragment thereof, immunoconjugate, or multi-specific molecule. Non-limiting examples of immune-activating cytokine include granulocyte macrophage colony stimulating factor (GM-CSF), IFN-α, IFN-β, IFN-γ, TNF-α, IL-1, IL-2, IL-3, IL-6, IL-1 1, IL-7, IL-8, IL-12, IL-15, IL-21, interferon regulatory factor 7 (IRF7), CCL1, CCL2, CCL3, CCL5, CCL7, CCL8, CCL13, CCL16, CXCL1, CXCL3, CXCL5, CXCL9, CXCL10, and combinations thereof.

In certain embodiments, the disease or disorder is a senescence-associated pathology. In certain embodiments, the subject exhibits an increased accumulation of senescent cells compared to that observed in a healthy control subject. In certain embodiments, the senescence-associated pathology is selected from the group consisting of lung fibrosis, atherosclerosis, Alzheimer's disease, diabetes, liver fibrosis, chronic kidney disease, osteoarthritis, cardiac fibrosis, and Parkinson's disease. In certain embodiments, the senescent cells exhibit a Senescence-Associated Secretory Phenotype (SASP). The Senescence-Associated Secretory Phenotype may be induced by replication, an oncogene (e.g., HRASG12D, NRAsG12D NRAsG12D, etc.), radiation, chemotherapy, or a drug (e.g., Cdk4/6 inhibitors, MEK inhibitors, chemotherapy drugs, etc.). Non-limiting examples of MEK inhibitors include trametinib, cobimetinib, binimetinib, selumetinib, PD-325901, TAK-733, CI-1040 (PD 184352), PD0325901, MEK162, AZD8330, GDC-0623, refametinib, pimasertib, R04987655, R05126766, WX-554, HL-085, CInQ-03, G-573, PD184161, PD318088, PD98059, R05068760, U0126, and SL327. Non-limiting examples of CDK4/6 inhibitors include palbociclib, ribociclib, and abemaciclib, Non-limiting examples of chemotherapy drugs include cisplatin, doxorubicin, cyclophosphamide, and etoposide.

In certain embodiments, the presently disclosed methods for treating or ameliorating a disease or disorder further comprise administering to the subject a tumor specific monoclonal antibody, wherein the subject is receiving/has received a senescence-inducing therapy (e.g., chemotherapy). In certain embodiments, the tumor specific monoclonal antibody is administered subsequent to the administration of the anti-uPAR antibody or antigen-binding fragment thereof, immunoconjugate, or multi-specific molecule. Non-limiting examples of senescence-inducing therapies include doxorubicin, ionizing radiation therapy, combination therapy with MEK inhibitors and CDK4/6 inhibitors, combination therapy with CDC7 inhibitors and mTOR inhibitors, and the like. Examples of CDK4/6 inhibitors include palbociclib, ribociclib, and abemaciclib. Non-limiting examples of MEK inhibitors include trametinib, cobimetinib, binimetinib, selumetinib, PD-325901, TAK-733, CI-1040 (PD184352), PD0325901, MEK162, AZD8330, GDC-0623, refametinib, pimasertib, R04987655, R05126766, WX-554, HL-085, CInQ-03, G-573, PD184161. PD318088, PD98059. R05068760, U0126, and SL327. Non-limiting examples of mTOR inhibitors include rapamycin, sertraline, sirolimus, everolimus, temsirolimus, ridaforolimus, and deforolimus. Examples of CDC7 inhibitors include TAK-931, PHA-767491, XL413, 1H-pyrrolo[2,3-b]pyridines, 2,3-dihydrothienol[3,2-d]pyrimidin-4(1H)-ones, furanone derivatives, trisubstituted thiazoles, pyrrolopyridinones, and the like.

In certain embodiments, the tumor specific monoclonal antibody is administered subsequent to the administration of the anti-uPAR antibody or antigen-binding fragment thereof, immunoconjugate, or multi-specific molecule.

In certain embodiments, the subject is human.

In certain embodiments, the presently disclosed methods for treating or ameliorating a disease or disorder further comprise administering to the subject a cancer therapy. In certain embodiments, the cancer therapy is selected from the group consisting of chemotherapy, radiation therapy, immunotherapy, monoclonal antibodies, anti-cancer nucleic acids or proteins, anti-cancer viruses or microorganisms, and any combinations thereof.

In certain embodiments, the presently disclosed methods for treating or ameliorating a disease or disorder further comprise fadministering to the subject a cytokine. In certain embodiments, the cytokine is administered prior to, during, or subsequent to the administration of the anti-uPAR antibody or antigen-binding fragment thereof, immunoconjugate, or multi-specific molecule. In certain embodiments, the cytokine is selected from the group consisting of interferon a, interferon (3, interferon y, complement C5a, IL-2, TNF-α, CD4OL, IL12, IL-23. IL15, IL17, CCL1, CCL11, CCL12, CCL13, CCL14-1, CCL14-2, CCL14-3, CCL15-1, CCL15-2, CCL16, CCL17, CCL18, CCL19, CCL19, CCL2, CCL20, CCL21. CCL22, CCL23-1, CCL23-2, CCL24, CCL25-1, CCL25-2, CCL26, CCL27, CCL28, CCL3, CCL3L1, CCL4, CCL4L1, CCL5, CCL6, CCL7, CCL8, CCL9, CCR10, CCR2, CCR5, CCR6, CCR7, CCR8, CCRL1, CCRL2, CX3CL1, CX3CR, CXCL1, CXCL10, CXCL11, CXCL12, CXCL13, CXCL14, CXCL15, CXCL16, CXCL2, CXCL3, CXCL4, CXCL5, CXCL6, CXCL7, CXCL8, CXCL9, CXCL9, CXCR1, CXCR2, CXCR4, CXCR5, CXCR6, CXCR7 and XCL2.

In certain embodiments, the chemotherapy comprises administering to the subject a chemotherapeutic agent. Non-limiting examples of chemotherapeutic agents include nitrogen mustards, ethylenimine derivatives, alkyl sulfonates, nitrosoureas, gemcitabine, triazenes, folic acid analogs, anthracyclines, taxanes, COX-2 inhibitors, pyrimidine analogs, purine analogs, antibiotics, enzyme inhibitors, epipodophyllotoxins. platinum coordination complexes, vinca alkaloids, substituted ureas, methyl hydrazine derivatives, adrenocortical suppressants, hormone antagonists, endostatin, taxols, camptothecins, SN-38, doxorubicin, doxorubicin analogs, antimetabolites, alkylating agents, antimitotics, anti-angiogenic agents, tyrosine kinase inhibitors, mTOR inhibitors, heat shock protein (HSP90) inhibitors, proteosome inhibitors, HDAC inhibitors, pro-apoptotic agents, methotrexate and CPT-11.

In certain embodiments, the disease or disorder is lung fibrosis, and the method further comprises sequentially, separately, or simultaneously administering to the subject at least one therapy selected from the group consisting of pirfenidone, nintedanib, oxygen therapy, corticosteroids (e.g., prednisone), mycophenolate mofetil/mycophenolic acid, and azathioprine.

In certain embodiments, the disease or disorder is atherosclerosis, and the method further comprises sequentially, separately, or simultaneously administering to the subject at least one therapy selected from the group consisting of statins (e.g., Atorvastatin, Fluvastatin, Lovastatin, Pitavastatin, Pravastatin, Rosuvastatin calcium, Simvastatin), fibrates (e.g., Gemfibrozil, Fenofibrate), niacin, ezetimibe, bile acid sequestrants (e.g.. cholestyramine, colestipol, colesevelam), proprotein convertase subtilisin kexin type 9 (PCSK9) inhibitors, anti-platelet medications (e.g., aspirin, Clopidogrel, Ticagrelor, warfarin, prasugral), beta blockers, Angiotensin-converting enzyme (ACE) inhibitors, calcium channel blockers, and diuretics.

In certain embodiments, the disease or disorder is Alzheimer's disease, and the method further comprises sequentially, separately, or simultaneously administering to the subject at least one therapy selected from the group consisting of donepezil, galantamine, memantine, rivastigmine, memantine extended-release and donepezil (Namzaric), aducanumab, solanezumab, insulin, verubecestat, AADvac1, CSP-1103, and intepirdine.

In certain embodiments, the disease or disorder is diabetes, and the method further comprises sequentially, separately, or simultaneously administering to the subject at least one therapy selected from the group consisting of insulin, metformin, amylin analogs, glucagon, sulfonylureas (e.g., glimepiride, glipizide, glyburide, chlorpropamide, tolazamide, tolbutamide), meglitinides (e.g., nateglinide, repaglinide), thiazolidinediones (e.g., pioglitazone, rosiglitazone), alpha-glucosidase inhibitors (e.g.. acarbose, miglitol), dipeptidyl peptidase (DPP-4) inhibitors (e.g., alogliptin, linagliptin, sitagliptin, saxagliptin), sodium-glucose co-transporter 2 (SGLT2) inhibitors (e.g., canagliflozin, dapagliflozin, empagliflozin, ertugliflozin), and incretin mimetics (e.g., exenatide, liraglutide, dulaglutide, lixisenatide, semaglutide).

In certain embodiments, the disease or disorder is osteoarthritis, and the method further comprises sequentially, separately, or simultaneously administering to the subject at least one therapy selected from the group consisting of analgesics (e.g., acetaminophen, tramadol, oxycodone, hydrocodone), nonsteroidal anti-inflammatory drugs (e.g., aspirin, ibuprofen, naproxen, celecoxib), cyclooxygetiase-2 inhibitors, corticosteroids, and hyaluronic acid.

In certain embodiments, the disease or disorder is liver fibrosis, and the method further comprises sequentially, separately, or simultaneously administering to the subject at least one therapy selected from the group consisting of ACE inhibitors (e.g., benazepril, Lisinopril, Ramipril), a-Tocopherol, interferon-a, PPAR-antagonists, colchicine, corticosteroids, endothelin inhibitors, interleukin-10, pentoxifylline, phosphatidylcholine, S-adenosyl-methionine, and TGF-131 inhibitors.

In certain embodiments, the disease or disorder is chronic kidney disease, and the method further comprises sequentially, separately, or simultaneously administering to the subject at least one therapy selected from the group consisting of ACE inhibitors (e.g., benazepril, Lisinopril, Ramipril), statins (e.g., Atorvastatin, Fluvastatin, Lovastatin, Pitavastatin, Pravastatin, Rosuvastatin calcium, Simvastatin), furosemide, erythropoietin, phosphate binders (e.g., calcium acetate, calcium carbonate), colecalciferol, ergocalciferol, and cyclophosphamide.

### 4.6. Diagnostic and Prognostic Methods

The presently disclosed anti-uPAR antibodies, antigen-binding fragments thereof, multi-specific molecules, and nucleic acids encode thereof can be used for diagnostic and prognostic applications as well as use as research tools for detection of uPAR in a biological sample, in a cell, a tissue, or a blood sample. The presently disclosed subject matter provides methods for detecting uPAR in a cell. a tissue, or a blood sample. In certain embodiments, the method comprises: contacting a cell. a tissue, or a blood sample with the antibody. antigen-binding fragment thereof, or multi-specific molecule disclosed herein, wherein the antibody, antigen-binding fragment thereof or multi-specific molecule comprises a detectable label; and determining the amount of the labeled antibody, antigen-binding fragment thereof, or multi-specific molecule bound to the cell, tissue, or blood sample by measuring the amount of detectable label associated with the cell or tissue, wherein the amount of bound antibody, antigen-binding fragment thereof, or multi-specific molecule indicates the amount of uPAR in the cell, tissue, or a blood sample. The cell or tissue can be any cell or tissue, including any normal, healthy, or cancerous cells and tissues. In certain embodiments, the blood sample is a peripheral blood sample.

uPAR may be used as a marker for detecting the senescent cell burden of a subject. Thus, the presently disclosed antibody, antigen-binding fragment thereof, or bispecific molecule can be used for detecting senescent cells in a biological sample obtained from a subject. The presently disclosed subject matter provides methods for detecting senescent cells in a biological sample obtained from a subject. In certain embodiments, the method comprises a) contacting the biological sample with the antibody, antigen-binding fragment thereof, or multi-specific molecule disclosed herein, wherein the antibody, antigen-binding fragment thereof or multi-specific molecule comprises a detectable label; b) determining the amount of the labeled antibody, antigen-binding fragment thereof, or multi-specific molecule in the biological sample by measuring the amount of detectable label in the biological sample, wherein the amount of bound antibody, antigen-binding fragment thereof, or multi-specific molecule indicates the amount of uPAR in the biological sample; and c) detecting the presence of senescent cells in the biological sample by detecting uPAR in the biological sample that are i) increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 100% compared to that observed in a reference sample; and/or ii) increased by at least 0.5-fold, at least 1.0 fold, at least 1.5-fold, at least 2.0 fold, at least 2.5-fold, at least 3.0 fold, at least 3.5-fold, at least 4.0 fold, at least 4.5-fold, at least 5.0 fold, at least 5.5-fold, at least 6.0 fold, at least 6.5-fold, at least 7.0 fold, at least 7.5-fold, at least 8.0 fold, at least 8.5-fold, at least 9.0 fold, at least 9.5-fold, or at least 10.0 fold compared to that observed in a reference sample. The detectable label can be an immunoPET probe. The reference sample may be obtained from a healthy control subject or may contain a predetermined level of the uPAR and/or suPAR polypeptide. Non-limiting examples of biological samples include mucus, saliva, bronchial alveolar lavage (BAL), bronchial wash (BW), whole blood, cerebrospinal fluid (CSF), urine, plasma, serum, lymph, semen, synovial fluid, tears, amniotic fluid, bile, aqueous humor, and a bodily fluid. In certain embodiments, measuring the amount of detectable label in the biological sample comprises Western Blotting, flow cytometry, Enzyme-linked immunosorbent assay (ELISA), immunoprecipitation, immunoelectrophoresis, immunostaining, imaging techniques (e.g., immunoPET), isoelectric focusing, High-performance liquid chromatography (HPLC), or mass-spectrometry.

### 4.7. Kits

The presently disclosed subject matter provides kits for treatment or ameliorating a disease or disorder, and/or detecting uPAR. In certain embodiments, the kit comprises the anti-uPAR antibodies or antigen-binding fragments thereof, the immunoconjugate, the multi-specific molecule, or the composition disclosed herein. In certain embodiments, the kit comprises a sterile container which contains a therapeutic or prophylactic vaccine; such containers can be boxes, ampules, bottles, vials, tubes, bags, pouches, blister-packs, or other suitable container forms known in the art. Such containers can be made of plastic, glass, laminated paper, metal foil, or other materials suitable for holding medicaments.

In certain embodiments, the kit further comprises instructions for administering the anti-uPAR antibodies or antigen-binding fragments thereof, the immunoconjugate, the multi-specific molecule, or the composition disclosed herein to a subject in need the treatment. The instructions can generally include information about the use of the anti-uPAR antibodies or antigen-binding fragments thereof, the immunoconjugate, the multi-specific molecule, and the composition disclosed herein for the treatment or ameliorating a disease or disorder. In certain embodiments, the instructions include at least one of the following: description of the therapeutic agent; dosage schedule and administration for treatment and/or prevention of a tumor or neoplasm or symptoms thereof; precautions; warnings; indications: counter-indications; overdosage information; adverse reactions; animal pharmacology; clinical studies; and/or references. The instructions may be printed directly on the container (when present), or as a label applied to the container, or as a separate sheet, pamphlet, card, or folder supplied in or with the container.

### 4.8. Exemplary Embodiments

A. In certain non-limiting embodiments, the presently disclosed subject matter provides an anti-uPAR antibody or an antigen-binding fragment thereof, comprising a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59. SEQ ID NO: 69, SEQ ID NO: 78, SEQ ID NO: 86, SEQ ID NO: 95, SEQ ID NO: 101, or SEQ ID NO: 108.

Al. In certain non-limiting embodiments, the presently disclosed subject matter provides an anti-uPAR antibody or an antigen-binding fragment thereof, comprising a light chain variable region comprising an amino acid sequence that is at least about 80%. at least about 85%, at least about 90%, at least about 95%, at least about 96%, at least about 97%, at least about 98%, at least about 99%, at least about 100% homologous or identical to the amino acid sequence set forth in SEQ ID NO: 8. SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

A2. In certain non-limiting embodiments, the presently disclosed subject matter provides an anti-uPAR antibody or an antigen-binding fragment thereof, comprising: (a) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108; and (b) a light chain variable region comprising an amino acid sequence that is at least about 80%, at least about 85%, at least about 90%, at least about 95%, at least about 96%, at least about 97%, at least about 98%, at least about 99% at least about 100% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

A3. In certain non-limiting embodiments, the presently disclosed subject matter provides an anti-uPAR antibody or an antigen-binding fragment thereof, comprising a heavy chain variable region and a light chain variable region, wherein the heavy chain variable region and the light chain variable region are selected from the group consisting of: (a) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO:7, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 8; (b) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 15, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 16; (c) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 23, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 24; (d) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 31, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 32; (e) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 41, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 42; (f) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 50. and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 51; (g) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 59. and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 60; (h) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 69, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 70; (i) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 78, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 79; (j) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 86, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 87; (k) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 95, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 96; and (l) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 101, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 102.

A4. In certain non-limiting embodiments, the presently disclosed subject matter provides an anti-uPAR antibody or an antigen-binding fragment thereof, comprising a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108.

A5. In certain non-limiting embodiments, the presently disclosed subject matter provides an anti-uPAR antibody or an antigen-binding fragment thereof, comprising a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

A6. In certain non-limiting embodiments, the presently disclosed subject matter provides an anti-uPAR antibody or an antigen-binding fragment thereof, comprising: (a) a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108; and (b) a light chain variable region comprising the amino acid sequence set forth in SEQ ID NO: 8, SEQ ID NO: 16, SEQ ID NO: 24, SEQ ID NO: 32. SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.

A7. The foregoing antibody or antigen-binding fragment thereof of any one of A-A6, wherein (a) the heavy chain variable region comprises the amino acid sequence set forth in SEQ ID NO: 7, and the light chain variable region comprises the amino acid sequence set forth in SEQ ID NO: 8; (b) the heavy chain variable region comprises the amino acid sequence set forth in SEQ ID NO: 15, and the light chain variable region comprises the amino acid sequence set forth in SEQ ID NO: 16; (c) the heavy chain variable region comprises the amino acid sequence set forth in SEQ ID NO: 23, and the light chain variable region comprises the amino acid sequence set forth in SEQ ID NO: 24; (d) the heavy chain variable region comprises the amino acid sequence set forth in SEQ ID NO: 31, and the light chain variable region comprises the amino acid sequence set forth in SEQ ID NO: 32; (e) the heavy chain variable region comprises the amino acid sequence set forth in SEQ ID NO: 41, and the light chain variable region comprises the amino acid sequence set forth in SEQ ID NO: 42; (t) the heavy chain variable region comprises the amino acid sequence set forth in SEQ ID NO: 50, and the light chain variable region comprises the amino acid sequence set forth in SEQ ID NO: 51; (g) the heavy chain variable region comprises the amino acid sequence set forth in SEQ ID NO: 59, and the light chain variable region comprises the amino acid sequence set forth in SEQ ID NO: 60: (h) the heavy chain variable region comprises the amino acid sequence set forth in SEQ ID NO: 69, and the light chain variable region comprises the amino acid sequence set forth in SEQ ID NO: 70; (i) the heavy chain variable region comprises the amino acid sequence set forth in SEQ ID NO: 78, and the light chain variable region comprises the amino acid sequence set forth in SEQ ID NO: 79: (j) the heavy chain variable region comprises the amino acid sequence set forth in SEQ ID NO: 86, and the light chain variable region comprises the amino acid sequence set forth in SEQ ID NO: 87; (k) the heavy chain variable region comprises the amino acid sequence set forth in SEQ ID NO: 95, and the light chain variable region comprises the amino acid sequence set forth in SEQ ID NO: 96; and (l) the heavy chain variable region comprises the amino acid sequence set forth in SEQ ID NO: 101, and the light chain variable region comprises the amino acid sequence set forth in SEQ ID NO: 102.

A8. In certain non-limiting embodiments, the presently disclosed subject matter provides an anti-uPAR antibody or an antigen-binding fragment thereof, comprising a heavy chain variable region that comprises CDR 1, CDR2, and CDR3 domains; and a light chain variable region that comprises CDR1, CDR2, and CDR3 domains, wherein the heavy chain variable region and light chain variable region CDR3 domains are selected from: (a) a heavy chain variable region CDR3 comprising the amino acid sequence set forth in SEQ ID NO: 3 and a conservative modification thereof; and a light chain variable region CDR3 comprising the amino acid sequence set forth in SEQ ID NO: 6 and a conservative modification thereof; (b) a heavy chain variable region CDR3 comprising the amino acid sequence set forth in SEQ ID NO: 12 and a conservative modification thereof, and a light chain variable region CDR3 comprising the amino acid sequence set forth in SEQ ID NO: 14 and a conservative modification thereof; (c) a heavy chain variable region CDR3 comprising the amino acid sequence set forth in SEQ ID NO: 20 and a conservative modification thereof; and a light chain variable region CDR3 comprising the amino acid sequence set forth in SEQ ID NO: 22 and a conservative modification thereof; (d) a heavy chain variable region CDR3 comprising the amino acid sequence set forth in SEQ ID NO: 28 and a conservative modification thereof; and a light chain variable region CDR3 comprising the amino acid sequence set forth in SEQ ID NO: 30 and a conservative modification thereof; (c) a heavy chain variable region CDR3 comprising the amino acid sequence set forth in SEQ ID NO: 37 and a conservative modification thereof; and a light chain variable region CDR3 comprising the amino acid sequence set forth in SEQ ID NO: 40 and a conservative modification thereof; (f) a heavy chain variable region CDR3 comprising the amino acid sequence set forth in SEQ ID NO: 47 and a conservative modification thereof; and a light chain variable region CDR3 comprising the amino acid sequence set forth in SEQ ID NO: 49 and a conservative modification thereof, (g) a heavy chain variable region CDR3 comprising the amino acid sequence set forth in SEQ ID NO: 56 and a conservative modification thereof; and a light chain variable region CDR3 comprising the amino acid sequence set forth in SEQ ID NO: 58 and a conservative modification thereof; (h) a heavy chain variable region CDR3 comprising the amino acid sequence set forth in SEQ ID NO: 65 and a conservative modification thereof; and a light chain variable region CDR3 comprising the amino acid sequence set forth in SEQ ID NO: 68 and a conservative modification thereof; (i) a heavy chain variable region CDR3 comprising the amino acid sequence set forth in SEQ ID NO: 75 and a conservative modification thereof; and a light chain variable region CDR3 comprising the amino acid sequence set forth in SEQ ID NO: 77 and a conservative modification thereof; (j) a heavy chain variable region CDR3 comprising the amino acid sequence set forth in SEQ ID NO: 83 and a conservative modification thereof; and a light chain variable region CDR3 comprising the amino acid sequence set forth in SEQ ID NO: 85 and a conservative modification thereof; (k) a heavy chain variable region CDR3 comprising the amino acid sequence set forth in SEQ ID NO: 92 and a conservative modification thereof; and a light chain variable region CDR3 comprising the amino acid sequence set forth in SEQ ID NO: 94 and a conservative modification thereof; and (l) a heavy chain variable region CDR3 comprising the amino acid sequence set forth in SEQ ID NO: 99 and a conservative modification thereof; and a light chain variable region CDR3 comprising the amino acid sequence set forth in SEQ ID NO: 100 and a conservative modification thereof.

A9. The foregoing antibody or antigen-binding fragment thereof of A8, wherein the heavy chain variable region and light chain variable region CDR2 domains are selected from: (a) a heavy chain variable region CDR2 comprising the amino acid sequence set forth in SEQ ID NO: 2 and a conservative modification thereof, and a light chain variable region CDR2 comprising the amino acid sequence set forth in SEQ ID NO: 5 and a conservative modification thereof; (b) a heavy chain variable region CDR2 comprising the amino acid sequence set forth in SEQ ID NO: 27 and a conservative modification thereof; and a light chain variable region CDR2 comprising the amino acid sequence set forth in SEQ ID NO: 5 and a conservative modification thereof; (c) a heavy chain variable region CDR2 comprising the amino acid sequence set forth in SEQ ID NO: 36 and a conservative modification thereof; and a light chain variable region CDR2 comprising the amino acid sequence set forth in SEQ ID NO: 39 and a conservative modification thereof; (d) a heavy chain variable region CDR2 comprising the amino acid sequence set forth in SEQ ID NO: 46 and a conservative modification thereof, and a light chain variable region CDR2 comprising the amino acid sequence set forth in SEQ ID NO: 5 and a conservative modification thereof; (e) a heavy chain variable region CDR2 comprising the amino acid sequence set forth in SEQ ID NO: 55 and a conservative modification thereof, and a light chain variable region CDR2 comprising the amino acid sequence set forth in SEQ ID NO: 5 and a conservative modification thereof; (f) a heavy chain variable region CDR2 comprising the amino acid sequence set forth in SEQ ID NO: 64 and a conservative modification thereof; and a light chain variable region CDR2 comprising the amino acid sequence set forth in SEQ ID NO: 67 and a conservative modification thereof; (g) a heavy chain variable region CDR2 comprising the amino acid sequence set forth in SEQ ID NO: 74 and a conservative modification thereof; and a light chain variable region CDR2 comprising the amino acid sequence set forth in SEQ ID NO: 67 and a conservative modification thereof, (h) a heavy chain variable region CDR2 comprising the amino acid sequence set forth in SEQ ID NO: 82 and a conservative modification thereof; and a light chain variable region CDR2 comprising the amino acid sequence set forth in SEQ ID NO: 67 and a conservative modification thereof; (i) a heavy chain variable region CDR2 comprising the amino acid sequence set forth in SEQ ID NO: 91 and a conservative modification thereof; and a light chain variable region CDR2 comprising the amino acid sequence set forth in SEQ ID NO: 67 and a conservative modification thereof; and (j) a heavy chain variable region CDR2 comprising the amino acid sequence set forth in SEQ ID NO: 46 and a conservative modification thereof; and a light chain variable region CDR2 comprising the amino acid sequence set forth in SEQ ID NO: 67 and a conservative modification thereof.

A10. The foregoing antibody or antigen-binding fragment thereof of A8 or A9, wherein the heavy chain variable region and light chain variable region CDR1 domains are selected from: (a) a heavy chain variable region CDR1 comprising the amino acid sequence set forth in SEQ ID NO: 1 and a conservative modification thereof; and a light chain variable region CDR1 comprising the amino acid sequence set forth in SEQ ID NO: 4 and a conservative modification thereof; (b) a heavy chain variable region CDR1 comprising the amino acid sequence set forth in SEQ ID NO: 11 and a conservative modification thereof; and a light chain variable region CDR 1 comprising the amino acid sequence set forth in SEQ ID NO: 13 and a conservative modification thereof: (c) a heavy chain variable region CDR1 comprising the amino acid sequence set forth in SEQ ID NO: 19 and a conservative modification thereof: and a light chain variable region CDR1 comprising the amino acid sequence set forth in SEQ ID NO: 21 and a conservative modification thereof; (d) a heavy chain variable region CDR1 comprising the amino acid sequence set forth in SEQ ID NO: 19 and a conservative modification thereof; and a light chain variable region CDR1 comprising the amino acid sequence set forth in SEQ ID NO: 29 and a conservative modification thereof; (e) a heavy chain variable region COR1 comprising the amino acid sequence set forth in SEQ ID NO: 35 and a conservative modification thereof; and a light chain variable region CDR1 comprising the amino acid sequence set forth in SEQ ID NO: 38 and a conservative modification thereof; (f) a heavy chain variable region CDR1 comprising the amino acid sequence set forth in SEQ ID NO: 45 and a conservative modification thereof, and a light chain variable region CDR1 comprising the amino acid sequence set forth in SEQ ID NO: 48 and a conservative modification thereof; (g) a heavy chain variable region CDR1 comprising the amino acid sequence set forth in SEQ ID NO: 54 and a conservative modification thereof; and a light chain variable region CDR1 comprising the amino acid sequence set forth in SEQ ID NO: 57 and a conservative modification thereof; (h) a heavy chain variable region CDR1 comprising the amino acid sequence set forth in SEQ ID NO: 63 and a conservative modification thereof; and a light chain variable region CDR1 comprising the amino acid sequence set forth in SEQ ID NO: 66 and a conservative modification thereof, (i) a heavy chain variable region CDR1 comprising the amino acid sequence set forth in SEQ ID NO: 73 and a conservative modification thereof, and a light chain variable region CDR1 comprising the amino acid sequence set forth in SEQ ID NO: 76 and a conservative modification thereof; (j) a heavy chain variable region CDR1 comprising the amino acid sequence set forth in SEQ ID NO: 45 and a conservative modification thereof; and a light chain variable region CDR1 comprising the amino acid sequence set forth in SEQ ID NO: 84 and a conservative modification thereof; (k) a heavy chain variable region CDR1 comprising the amino acid sequence set forth in SEQ ID NO: 90 and a conservative modification thereof; and a light chain variable region CDR1 comprising the amino acid sequence set forth in SEQ ID NO: 93 and a conservative modification thereof; and (l) a heavy chain variable region CDR1 comprising the amino acid sequence set forth in SEQ ID NO: 45 and a conservative modification thereof; and a light chain variable region CDR1 comprising the amino acid sequence set forth in SEQ ID NO: 66 and a conservative modification thereof.

A11. The foregoing antibody or antigen-binding fragment thereof of any one of A8-A10, wherein one or more of the CDR sequences have up to about 5 amino acid substitutions.

A12. The foregoing antibody or antigen-binding fragment thereof of any one of A8-A10, wherein one or more of the CDR sequences have up to about 3 amino acid substitutions.

A13. In certain non-limiting embodiments, the presently disclosed subject matter provides an anti-uPAR antibody or an antigen-binding fragment thereof, comprising a heavy chain variable region comprising: (a) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; (b) a CDR1 comprising the amino acid sequence set forth in SEQ ID NO: 11, a CDR2 comprising the amino acid sequence set forth in SEQ ID NO: 2. and a CDR3 comprising the amino acid sequence set forth in SEQ ID NO: 12; (c) a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; (d) a CDR 1 comprising the amino acid sequence set forth in SEQ ID NO: 19; a heavy chain variable region CDR2 comprising the amino acid sequence set forth in SEQ ID NO: 27; and a heavy chain variable region CDR3 comprising the amino acid sequence set forth in SEQ ID NO: 28; (e) a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; (f) a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; (g) a CDR1 comprising the amino acid sequence set forth in SEQ ID NO: 54, a CDR2 comprising the amino acid sequence set forth in SEQ ID NO: 55, and a CDR3 comprising the amino acid sequence set forth in SEQ ID NO: 56; (h) a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65; (i) a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; (j) a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83; (k) a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92; (l) a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; or (m) a CDR1 comprising the amino acid sequence set forth in SEQ ID NO: 105, a CDR2 comprising the amino acid sequence set forth in SEQ ID NO: 106, and a CDR3 comprising the amino acid sequence set forth in SEQ ID NO: 107.

A14. In certain non-limiting embodiments, the presently disclosed subject matter provides an n anti-uPAR antibody or an antigen-binding fragment thereof, comprising a light chain variable region comprising: (a) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6; (b) a CDR1 comprising the amino acid sequence set forth in SEQ ID NO: 13, a CDR2 comprising the amino acid sequence set forth in SEQ ID NO: 5. and a CDR3 comprising the amino acid sequence set forth in SEQ ID NO: 14; (c) a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22; (d) a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30; (e) a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40; (f) a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49; (g) a CDR1 comprising the amino acid sequence set forth in SEQ ID NO: 57, a CDR2 comprising the amino acid sequence set forth in SEQ ID NO: 5. and a CDR3 comprising the amino acid sequence set forth in SEQ ID NO: 58; (h) a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68; (i) a COR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77; (j) a CDR1 comprising the amino acid sequence set forth in SEQ ID NO: 84, a CDR2 comprising the amino acid sequence set forth in SEQ ID NO: 67, and a CDR3 comprising the amino acid sequence set forth in SEQ ID NO: 85; (k) a CDR1 comprising the amino acid sequence set forth in SEQ ID NO: 93, a CDR2 comprising the amino acid sequence set forth in SEQ ID NO: 67. and a CDR3 comprising the amino acid sequence set forth in SEQ ID NO: 94; or (l) a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.

A15. In certain non-limiting embodiments, the presently disclosed subject matter provides an anti-uPAR antibody or an antigen-binding fragment thereof, comprising: (a) a heavy chain variable region comprising a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; and a light chain variable region comprising a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6; (b) a heavy chain variable region comprising a COR1 comprising the amino acid sequence set forth in SEQ ID NO: 11, a CDR2 comprising an amino acid sequence set forth in SEQ ID NO: 2, and a CDR3 comprising the amino acid sequence set forth in SEQ ID NO: 12; and a light chain variable region comprising a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14; (c) a heavy chain variable region comprising a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; and a light chain variable region comprising a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22; (d) a heavy chain variable region comprising a CDR1 comprising the amino acid sequence set forth in SEQ ID NO: 19. a CDR2 comprising the amino acid sequence set forth in SEQ ID NO: 27, and a CDR3 comprising the amino acid sequence set forth in SEQ ID NO: 28; and a light chain variable region comprising a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30; (e) a heavy chain variable region comprising a COR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; and a light chain variable region comprising a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40; (f) heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; and a light chain variable region comprising a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49; (g) heavy chain variable region comprising a CDR1 comprising the amino acid sequence set forth in SEQ ID NO: 54, a comprising the amino acid sequence set forth in SEQ ID NO: 55, and a CDR3 comprising the amino acid sequence set forth in SEQ ID NO: 56; and a light chain variable region comprising a CDR1 comprising the amino acid sequence set forth in SEQ ID NO: 57, a CDR2 comprising the amino acid sequence set forth in SEQ ID NO: 5. and a CDR3 comprising the amino acid sequence set forth in SEQ ID NO: 58: (h) heavy chain variable region comprising a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65; and a light chain variable region comprising a CDR 1 comprising the amino acid sequence set forth in SEQ ID NO: 66. a CDR2 comprising the amino acid sequence set forth in SEQ ID NO: 67, and a CDR3 comprising the amino acid sequence set forth in SEQ ID NO: 68; (i) heavy chain variable region comprising a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; and a light chain variable region comprising a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77; (j) heavy chain variable region comprising a CDR1 comprising the amino acid sequence set forth in SEQ ID NO: 45. a CDR2 comprising the amino acid sequence set forth in SEQ ID NO: 82, and a CDR3 comprising the amino acid sequence set forth in SEQ ID NO: 83; and a light chain variable region comprising a CDR1 comprising the amino acid sequence set forth in SEQ ID NO: 84. a CDR2 comprising the amino acid sequence set forth in SEQ ID NO: 67. and a CDR3 comprising the amino acid sequence set forth in SEQ ID NO: 85; (k) heavy chain variable region comprising a CDR1 comprising the amino acid sequence set forth in SEQ ID NO: 90. a CDR2 comprising the amino acid sequence set forth in SEQ ID NO: 91, and a CDR3 comprising the amino acid sequence set forth in SEQ ID NO: 92; and a light chain variable region comprising a CDR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94; or (l) heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.

A16. The foregoing antibody or antigen-binding fragment thereof of any one of A-A15, wherein the antibody or antigen-binding fragment thereof binds to a uPAR comprising the amino acid sequence set forth in SEQ ID NO: 116 or a fragment thereof.

A17. The foregoing antibody or antigen-binding fragment thereof of any one of A-A16, wherein the sequence of the antibody is in a light-heavy variable chain orientation (V_{L}-V_{H}).

A18. The foregoing antibody or antigen-binding fragment thereof of any one of A-A17, wherein the antibody comprises a human variable region framework region.

A19. The foregoing antibody or antigen-binding fragment thereof of any one of A-A17, which is a fully human or an antigen-binding fragment thereof.

A20. The foregoing antibody or antigen-binding fragment thereof of any one of A-A17, which is a chimeric antibody or an antigen-binding fragment thereof.

A21. The foregoing antibody or antigen-binding fragment thereof of any one of A-A17, which is a humanized antibody or an antigen-binding fragment thereof.

A22. The foregoing antibody or antigen-binding fragment thereof of any one of A-A21, wherein the antigen-binding fragment is a Fab, Fab', F(ab')₂, variable fragment (Fv), or single chain variable region (scFv).

A23. The foregoing antibody or antigen-binding fragment thereof of A22, wherein the antigen antigen-binding fragment is an scFv.

A24. The foregoing antibody or an antigen-binding fragment thereof of A23, wherein the scFv comprises the amino acid sequence set forth in SEQ ID NO: 9, SEQ ID NO: 17, SEQ ID NO: 25, SEQ ID NO: 33, SEQ ID NO: 43, SEQ ID NO: 52, SEQ ID NO: 61. SEQ ID NO: 71, SEQ ID NO: 80, SEQ ID NO: 88, SEQ ID NO: 97, or SEQ ID NO: 103.

A25. In certain non-limiting embodiments, the presently disclosed subject matter provides an antibody or an antigen-binding fragment thereof, which cross-competes for binding to uPAR with an antibody or an antigen-binding fragment thereof of any one of A-A24.

A26. In certain non-limiting embodiments, the presently disclosed subject matter provides an antibody or an antigen-binding fragment thereof, which binds to the same epitope region on uPAR with an antibody or an antigen-binding fragment thereof of any one of A-A24.

B. In certain non-limiting embodiments, the presently disclosed subject matter provides a composition comprising the antibody or antigen-binding fragment thereof of any one of A-A26.

B1. The foregoing composition of B, which is a pharmaceutical composition that further comprises a pharmaceutically acceptable carrier.

C. In certain non-limiting embodiments, the presently disclosed subject matter provides an immunoconjugate comprising the antibody or antigen-binding fragment thereof of any one of A-A26, linked to a therapeutic agent.

C1. The foregoing immunoconjugate of C, wherein the therapeutic agent is a drug, a cytotoxin, or a radioactive isotope.

D. In certain non-limiting embodiments, the presently disclosed subject matter provides a composition comprising the immunoconjugate of C or C1.

D1. The foregoing composition of D, which is a pharmaceutical composition that further comprises a pharmaceutically acceptable carrier.

E. In certain non-limiting embodiments, the presently disclosed subject matter provides a multi-specific molecule comprising the antibody or antigen-binding fragment thereof of any one of A-A26, linked to one or more functional moieties.

E1. The foregoing bispecific molecule of E, wherein the one or more functional moieties have a different binding specificity than the antibody or antigen binding fragment thereof.

F. In certain non-limiting embodiments, the presently disclosed subject matter provides a composition comprising the multi-specific molecule of claim E or E1.

F1. The foregoing composition of F, which is a pharmaceutical composition that further comprises a pharmaceutically acceptable carrier.

G. In certain non-limiting embodiments, the presently disclosed subject matter provides a nucleic acid that encodes an antibody or antigen-binding fragment thereof of any one of A-A26.

H. In certain non-limiting embodiments, the presently disclosed subject matter provides a vector comprising the nucleic acid molecule of G.

I. In certain non-limiting embodiments, the presently disclosed subject matter provides a host cell comprising the vector of H.

J. In certain non-limiting embodiments, the presently disclosed subject matter provides a method for detecting uPAR in a whole cell, a tissue, or a blood sample, comprising: contacting a cell, tissue or blood sample with the antibody or antigen-binding fragment thereof of any one of claims 1-27, wherein the antibody or antigen-binding fragment thereof comprises a detectable label; and determining the amount of the labeled antibody or antigen-binding fragment thereof bound to the cell, tissue or blood sample by measuring the amount of detectable label associated with said cell or tissue, wherein the amount of bound antibody or antigen-binding fragment thereof indicates the amount of uPAR in the cell, tissue or blood sample.

K. In certain non-limiting embodiments, the presently disclosed subject matter provides a method of treating or ameliorating a disease or disorder in a subject, comprising administering to the subject the antibody or antigen-binding fragment thereof of any one of A-A26, the immunoconjugate of C or C1, the multi-specific molecule of E or E1, or the composition of any one of B, B1, D, D1, F, and F1.

K1. The foregoing method of K, wherein the disease or disorder is selected from the group consisting of tumors, senescence-associated pathologies, and tissue decline associated with aging.

K2. The foregoing method of K1, wherein the disease or disorder is a senescence-associated pathology.

K3. The foregoing method of K2. wherein the senescence-associated pathology is selected from the group consisting of lung fibrosis, atherosclerosis, Alzheimer's disease, diabetes, osteoarthritis, liver fibrosis, chronic kidney disease, cardiac fibrosis, and Parkinson's disease.

K4. The foregoing method of K1, wherein the disease or disorder is a tumor.

K5. The foregoing method of K4, wherein the tumor is selected from the group consisting of breast cancer, endometrial cancer, ovarian cancer, colon cancer, rectal cancer, lung cancer, stomach cancer, prostate cancer, gastric cancer, renal cancer, pancreatic cancer, blood cancer, cervical cancer, head and neck cancer, liver cancer, urotherial cancer, melanoma, and brain cancer.

K6. The foregoing method of K5. wherein the blood cancer is selected from the group consisting of acute lymphoblastic leukemia (ALL), chronic lymphocytic leukemia (CLL), acute myeloid leukemia (AML), myelofibrosis, polycythemia vera, myelodysplastic syndrome, and erythroleukemia.

K7. The foregoing method of K4-K6, wherein the tumor is cancer.

L. In certain non-limiting embodiments, the presently disclosed subject matter provides a method of increasing production of an immune-activating cytokine in response to a tumor cell in a subject, comprising administering to the subject the antibody or antigen-binding fragment thereof of any one of A-A26, the immunoconjugate of C or C1, the multi-specific molecule of E or E1, or the composition of any one of B, B1, D, D1, F, and F1.

L1. The foregoing method of L, wherein the immune-activating cytokine is selected from the group consisting of granulocyte macrophage colony stimulating factor (GM-CSF), IFN-α, IFN-β, IFN-γ, TNF-α, IL-1 IL-2, IL-3, IL-6, IL-11, IL-7, IL-8, IL-12, IL-15, IL-21, interferon regulatory factor 7 (IRF7), CCL1, CCL2, CCL3, CCL5, CCL7, CCL8, CCL13, CCL16, CXCL1, CXCL3, (CXCL5, CXCL9, CXCL10, and combinations thereof.

L2. The foregoing method of any one of J, K1-K7, and L-L1, wherein the subject is a human.

M. In certain non-limiting embodiments, the presently disclosed subject matter provides a kit for treating or ameliorating a disease or disorder in a subject, and/or increasing production of an immune-activating cytokine in response to a tumor cell in a subject, comprising the antibody or antigen-binding fragment thereof of any one of A-A26, the immunoconjugate of C or C1, the multi-specific molecule of E or E1, or the composition of any one of B, B1, D, D1, F, and F1.

M1. The foregoing kit of M, wherein the kit further comprises written instructions for using the antibody or antigen-binding fragment thereof, immunoconjugate, multi-specific molecule, or composition for treating or ameliorating a disease or disorder in a subject, and/or increasing production of an immune-activating cytokine in response to a tumor cell in a subject.

### 5. EXAMPLES

The following examples are put forth so as to provide those of ordinary skill in the art with a complete disclosure and description of how to make and use the antibodies, multi-specific antibodies, compositions comprising thereof, screening, and therapeutic methods of the presently disclosed subject matter, and are not intended to limit the scope of what the inventors regard as their presently disclosed subject matter. It is understood that various other embodiments may be practiced, given the general description provided above.

### Example 1 - Generation of anti-uPAR antibodies and scFvs

A portion of uPAR corresponding to the extracellular domain and amino acids His 65-Ala 265, was recombinantly produced as a soluble protein with a polyhistidine tag for purification. The extracellular domain of murine uPAR (Thr 67-Arg 267) was also produced with a polyhistidine tag to screen antibodies for cross-species reactivity.

A proprietary naïve, semi-synthetic scFv phage display library was screened for antibodies that bind to the uPAR protein by using standard solid phase phage display panning techniques. Briefly, recombinant uPAR was immobilized on a polystyrene surface followed by blocking with about 5% milk and incubation with the phage library. Subsequent washing, elution and phage amplification steps were performed to complete each round of biopanning. Three rounds of panning were completed using amplified uPAR binder-enriched phage pools from the previous round of panning as input for subsequent rounds. In order to identify clones that showed high specificity for uPAR, single clones from the third round of panning were analyzed for binding to human uPAR, murine uPAR, and BSA (as a non-specific control) by enzyme-linked immunosorbent assay (ELISA) using an anti-M13 phage antibody. Only those supernatants that showed uPAR-specific binding were selected for sequencing, resulting in the identification of thirteen clones with unique sequences (3-C3-A, 3-D8-A, 3-G1-A, 3-H4-A, 4-F5-A, 4-F12-A, 4-A5-B, 05G9, 05A6, 05B2, 05F5, 05G5, and 07B3). Some clones showed binding to both human and mouse homologs of uPAR, e.g.. 3-C3-A. 3-D8-A, 40FSA.

To test whether antibodies recovered from the phage panning campaign were able to bind to uPAR in its native conformation on the cell surface, monoclonal phage preps were also screened by flow cytometry on NALM6 cells transfected with uPAR and wild type NALM6 cells.

### Embodiments of the presently disclosed subject matter

From the foregoing description, it will be apparent that variations and modifications may be made to the presently disclosed subject matter to adopt it to various usages and conditions. Such embodiments are also within the scope of the following claims.

The recitation of a listing of elements in any definition of a variable herein includes definitions of that variable as any single element or combination (or sub-combination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.
Aspects or embodiments of the invention may also be provided according to the following paragraphs:
1. An anti-uPAR antibody or an antigen-binding fragment thereof, comprising a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108.
2. An anti-uPAR antibody or an antigen-binding fragment thereof, comprising a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.
3. An anti-uPAR antibody or an antigen-binding fragment thereof, comprising
   (a) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108; and
   (b) a light chain variable region comprising an amino acid sequence that is at least about 80%, at least about 85%, at least about 90%, at least about 95%, at least about 96%, at least about 97%, at least about 98%, at least about 99% at least about 100% homologous or identical to the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.
4. An anti-uPAR antibody or an antigen-binding fragment thereof, comprising a heavy chain variable region and a light chain variable region, wherein the heavy chain variable region and the light chain variable region are selected from the group consisting of:
   (a) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO:7, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 8;
   (b) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 15, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 16;
   (c) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 23, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 24;
   (d) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 31, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 32;
   (e) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 41, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 42;
   (f) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 50, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 51;
   (g) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 59, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 60;
   (h) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 69, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 70;
   (i) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 78, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 79;
   (j) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 86, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 87;
   (k) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 95, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 96; and
   (l) a heavy chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 101, and a light chain variable region comprising an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 102.
5. An anti-uPAR antibody or an antigen-binding fragment thereof, comprising a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108.
6. An anti-uPAR antibody or an antigen-binding fragment thereof, comprising a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.
7. An anti-uPAR antibody or an antigen-binding fragment thereof, comprising
   (a) a heavy chain variable region comprising the amino acid sequence set forth in SEQ ID NO: 7, SEQ ID NO: 15, SEQ ID NO: 23, SEQ ID NO: 31, SEQ ID NO: 41, SEQ ID NO: 50, SEQ ID NO: 59, SEQ ID NO: 69, SEQ ID NO: 78, SEQ ID NO: 86, SEQ ID NO: 95, SEQ ID NO: 101, or SEQ ID NO: 108; and
   (b) a light chain variable region comprising the amino acid sequence set forth in SEQ ID NO: 8, SEQ ID NO: 16, SEQ ID NO: 24, SEQ ID NO: 32, SEQ ID NO: 42, SEQ ID NO: 51, SEQ ID NO: 60, SEQ ID NO: 70, SEQ ID NO: 79, SEQ ID NO: 87, SEQ ID NO: 96, or SEQ ID NO: 102.
8. The antibody or antigen-binding fragment thereof of any one of paragraphs 1-7, wherein
   (a) the heavy chain variable region comprises the amino acid sequence set forth in SEQ ID NO: 7, and the light chain variable region comprises the amino acid sequence set forth in SEQ ID NO: 8;
   (b) the heavy chain variable region comprises the amino acid sequence set forth in SEQ ID NO: 15, and the light chain variable region comprises the amino acid sequence set forth in SEQ ID NO: 16;
   (c) the heavy chain variable region comprises the amino acid sequence set forth in SEQ ID NO: 23, and the light chain variable region comprises the amino acid sequence set forth in SEQ ID NO: 24;
   (d) the heavy chain variable region comprises the amino acid sequence set forth in SEQ ID NO: 31, and the light chain variable region comprises the amino acid sequence set forth in SEQ ID NO: 32;
   (e) the heavy chain variable region comprises the amino acid sequence set forth in SEQ ID NO: 41, and the light chain variable region comprises the amino acid sequence set forth in SEQ ID NO: 42;
   (f) the heavy chain variable region comprises the amino acid sequence set forth in SEQ ID NO: 50, and the light chain variable region comprises the amino acid sequence set forth in SEQ ID NO: 51;
   (g) the heavy chain variable region comprises the amino acid sequence set forth in SEQ ID NO: 59, and the light chain variable region comprises the amino acid sequence set forth in SEQ ID NO: 60;
   (h) the heavy chain variable region comprises the amino acid sequence set forth in SEQ ID NO: 69, and the light chain variable region comprises the amino acid sequence set forth in SEQ ID NO: 70;
   (i) the heavy chain variable region comprises the amino acid sequence set forth in SEQ ID NO: 78, and the light chain variable region comprises the amino acid sequence set forth in SEQ ID NO: 79;
   (j) the heavy chain variable region comprises the amino acid sequence set forth in SEQ ID NO: 86, and the light chain variable region comprises the amino acid sequence set forth in SEQ ID NO: 87;
   (k) the heavy chain variable region comprises the amino acid sequence set forth in SEQ ID NO: 95, and the light chain variable region comprises the amino acid sequence set forth in SEQ ID NO: 96; and
   (l) the heavy chain variable region comprises the amino acid sequence set forth in SEQ ID NO: 101, and the light chain variable region comprises the amino acid sequence set forth in SEQ ID NO: 102.
9. An anti-uPAR antibody or an antigen-binding fragment thereof, comprising a heavy chain variable region that comprises CDR1, CDR2, and CDR3 domains; and a light chain variable region that comprises CDR1, CDR2, and CDR3 domains, wherein the heavy chain variable region and light chain variable region CDR3 domains are selected from:
   (a) a heavy chain variable region CDR3 comprising the amino acid sequence set forth in SEQ ID NO: 3 and a conservative modification thereof; and a light chain variable region CDR3 comprising the amino acid sequence set forth in SEQ ID NO: 6 and a conservative modification thereof;
   (b) a heavy chain variable region CDR3 comprising the amino acid sequence set forth in SEQ ID NO: 12 and a conservative modification thereof; and a light chain variable region CDR3 comprising the amino acid sequence set forth in SEQ ID NO: 14 and a conservative modification thereof;
   (c) a heavy chain variable region CDR3 comprising the amino acid sequence set forth in SEQ ID NO: 20 and a conservative modification thereof; and a light chain variable region CDR3 comprising the amino acid sequence set forth in SEQ ID NO: 22 and a conservative modification thereof;
   (d) a heavy chain variable region CDR3 comprising the amino acid sequence set forth in SEQ ID NO: 28 and a conservative modification thereof; and a light chain variable region CDR3 comprising the amino acid sequence set forth in SEQ ID NO: 30 and a conservative modification thereof;
   (e) a heavy chain variable region CDR3 comprising the amino acid sequence set forth in SEQ ID NO: 37 and a conservative modification thereof; and a light chain variable region CDR3 comprising the amino acid sequence set forth in SEQ ID NO: 40 and a conservative modification thereof;
   (f) a heavy chain variable region CDR3 comprising the amino acid sequence set forth in SEQ ID NO: 47 and a conservative modification thereof; and a light chain variable region CDR3 comprising the amino acid sequence set forth in SEQ ID NO: 49 and a conservative modification thereof;
   (g) a heavy chain variable region CDR3 comprising the amino acid sequence set forth in SEQ ID NO: 56 and a conservative modification thereof; and a light chain variable region CDR3 comprising the amino acid sequence set forth in SEQ ID NO: 58 and a conservative modification thereof;
   (h) a heavy chain variable region CDR3 comprising the amino acid sequence set forth in SEQ ID NO: 65 and a conservative modification thereof; and a light chain variable region CDR3 comprising the amino acid sequence set forth in SEQ ID NO: 68 and a conservative modification thereof;
   (i) a heavy chain variable region CDR3 comprising the amino acid sequence set forth in SEQ ID NO: 75 and a conservative modification thereof; and a light chain variable region CDR3 comprising the amino acid sequence set forth in SEQ ID NO: 77 and a conservative modification thereof;
   (j) a heavy chain variable region CDR3 comprising the amino acid sequence set forth in SEQ ID NO: 83 and a conservative modification thereof; and a light chain variable region CDR3 comprising the amino acid sequence set forth in SEQ ID NO: 85 and a conservative modification thereof;
   (k) a heavy chain variable region CDR3 comprising the amino acid sequence set forth in SEQ ID NO: 92 and a conservative modification thereof; and a light chain variable region CDR3 comprising the amino acid sequence set forth in SEQ ID NO: 94 and a conservative modification thereof; and
   (l) a heavy chain variable region CDR3 comprising the amino acid sequence set forth in SEQ ID NO: 99 and a conservative modification thereof; and a light chain variable region CDR3 comprising the amino acid sequence set forth in SEQ ID NO: 100 and a conservative modification thereof.
10. The antibody or antigen-binding fragment thereof of paragraph 9, wherein the heavy chain variable region and light chain variable region CDR2 domains are selected from:
   (a) a heavy chain variable region CDR2 comprising the amino acid sequence set forth in SEQ ID NO: 2 and a conservative modification thereof; and a light chain variable region CDR2 comprising the amino acid sequence set forth in SEQ ID NO: 5 and a conservative modification thereof;
   (b) a heavy chain variable region CDR2 comprising the amino acid sequence set forth in SEQ ID NO: 27 and a conservative modification thereof; and a light chain variable region CDR2 comprising the amino acid sequence set forth in SEQ ID NO: 5 and a conservative modification thereof;
   (c) a heavy chain variable region CDR2 comprising the amino acid sequence set forth in SEQ ID NO: 36 and a conservative modification thereof; and a light chain variable region CDR2 comprising the amino acid sequence set forth in SEQ ID NO: 39 and a conservative modification thereof;
   (d) a heavy chain variable region CDR2 comprising the amino acid sequence set forth in SEQ ID NO: 46 and a conservative modification thereof; and a light chain variable region CDR2 comprising the amino acid sequence set forth in SEQ ID NO: 5 and a conservative modification thereof;
   (e) a heavy chain variable region CDR2 comprising the amino acid sequence set forth in SEQ ID NO: 55 and a conservative modification thereof; and a light chain variable region CDR2 comprising the amino acid sequence set forth in SEQ ID NO: 5 and a conservative modification thereof;
   (f) a heavy chain variable region CDR2 comprising the amino acid sequence set forth in SEQ ID NO: 64 and a conservative modification thereof; and a light chain variable region CDR2 comprising the amino acid sequence set forth in SEQ ID NO: 67 and a conservative modification thereof;
   (g) a heavy chain variable region CDR2 comprising the amino acid sequence set forth in SEQ ID NO: 74 and a conservative modification thereof; and a light chain variable region CDR2 comprising the amino acid sequence set forth in SEQ ID NO: 67 and a conservative modification thereof;
   (h) a heavy chain variable region CDR2 comprising the amino acid sequence set forth in SEQ ID NO: 82 and a conservative modification thereof; and a light chain variable region CDR2 comprising the amino acid sequence set forth in SEQ ID NO: 67 and a conservative modification thereof;
   (i) a heavy chain variable region CDR2 comprising the amino acid sequence set forth in SEQ ID NO: 91 and a conservative modification thereof; and a light chain variable region CDR2 comprising the amino acid sequence set forth in SEQ ID NO: 67 and a conservative modification thereof; and
   (j) a heavy chain variable region CDR2 comprising the amino acid sequence set forth in SEQ ID NO: 46 and a conservative modification thereof; and a light chain variable region CDR2 comprising the amino acid sequence set forth in SEQ ID NO: 67 and a conservative modification thereof.
11. The antibody or antigen-binding fragment thereof of paragraph 9 or 10, wherein the heavy chain variable region and light chain variable region CDR1 domains are selected from:
   (a) a heavy chain variable region CDR1 comprising the amino acid sequence set forth in SEQ ID NO: 1 and a conservative modification thereof; and a light chain variable region CDR1 comprising the amino acid sequence set forth in SEQ ID NO: 4 and a conservative modification thereof;
   (b) a heavy chain variable region CDR1 comprising the amino acid sequence set forth in SEQ ID NO: 11 and a conservative modification thereof; and a light chain variable region CDR1 comprising the amino acid sequence set forth in SEQ ID NO: 13 and a conservative modification thereof;
   (c) a heavy chain variable region CDR1 comprising the amino acid sequence set forth in SEQ ID NO: 19 and a conservative modification thereof; and a light chain variable region CDR1 comprising the amino acid sequence set forth in SEQ ID NO: 21 and a conservative modification thereof;
   (d) a heavy chain variable region CDR1 comprising the amino acid sequence set forth in SEQ ID NO: 19 and a conservative modification thereof; and a light chain variable region CDR1 comprising the amino acid sequence set forth in SEQ ID NO: 29 and a conservative modification thereof;
   (e) a heavy chain variable region CDR1 comprising the amino acid sequence set forth in SEQ ID NO: 35 and a conservative modification thereof; and a light chain variable region CDR1 comprising the amino acid sequence set forth in SEQ ID NO: 38 and a conservative modification thereof;
   (f) a heavy chain variable region CDR1 comprising the amino acid sequence set forth in SEQ ID NO: 45 and a conservative modification thereof; and a light chain variable region CDR1 comprising the amino acid sequence set forth in SEQ ID NO: 48 and a conservative modification thereof;
   (g) a heavy chain variable region CDR1 comprising the amino acid sequence set forth in SEQ ID NO: 54 and a conservative modification thereof; and a light chain variable region CDR1 comprising the amino acid sequence set forth in SEQ ID NO: 57 and a conservative modification thereof;
   (h) a heavy chain variable region CDR1 comprising the amino acid sequence set forth in SEQ ID NO: 63 and a conservative modification thereof; and a light chain variable region CDR1 comprising the amino acid sequence set forth in SEQ ID NO: 66 and a conservative modification thereof;
   (i) a heavy chain variable region CDR1 comprising the amino acid sequence set forth in SEQ ID NO: 73 and a conservative modification thereof; and a light chain variable region CDR1 comprising the amino acid sequence set forth in SEQ ID NO: 76 and a conservative modification thereof;
   (j) a heavy chain variable region CDR1 comprising the amino acid sequence set forth in SEQ ID NO: 45 and a conservative modification thereof; and a light chain variable region CDR1 comprising the amino acid sequence set forth in SEQ ID NO: 84 and a conservative modification thereof;
   (k) a heavy chain variable region CDR1 comprising the amino acid sequence set forth in SEQ ID NO: 90 and a conservative modification thereof; and a light chain variable region CDR1 comprising the amino acid sequence set forth in SEQ ID NO: 93 and a conservative modification thereof; and
   (l) a heavy chain variable region CDR1 comprising the amino acid sequence set forth in SEQ ID NO: 45 and a conservative modification thereof; and a light chain variable region CDR1 comprising the amino acid sequence set forth in SEQ ID NO: 66 and a conservative modification thereof.
12. The antibody or antigen-binding fragment thereof of any one of paragraphs 9-11, wherein one or more of the CDR sequences have up to about 5 amino acid substitutions.
13. The antibody or antigen-binding fragment thereof of any one of paragraphs 9-11, wherein one or more of the CDR sequences have up to about 3 amino acid substitutions.
14. An anti-uPAR antibody or an antigen-binding fragment thereof, comprising a heavy chain variable region comprising:
   (a) a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3;
   (b) a CDR1 comprising the amino acid sequence set forth in SEQ ID NO: 11, a CDR2 comprising the amino acid sequence set forth in SEQ ID NO: 2, and a CDR3 comprising the amino acid sequence set forth in SEQ ID NO: 12;
   (c) a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20;
   (d) a CDR1 comprising the amino acid sequence set forth in SEQ ID NO: 19; a heavy chain variable region CDR2 comprising the amino acid sequence set forth in SEQ ID NO: 27; and a heavy chain variable region CDR3 comprising the amino acid sequence set forth in SEQ ID NO: 28;
   (e) a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37;
   (f) a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47;
   (g) a CDR1 comprising the amino acid sequence set forth in SEQ ID NO: 54, a CDR2 comprising the amino acid sequence set forth in SEQ ID NO: 55, and a CDR3 comprising the amino acid sequence set forth in SEQ ID NO: 56;
   (h) a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65;
   (i) a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75;
   (j) a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83;
   (k) a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92;
   (l) a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; or
   (m) a CDR1 comprising the amino acid sequence set forth in SEQ ID NO: 105, a CDR2 comprising the amino acid sequence set forth in SEQ ID NO: 106, and a CDR3 comprising the amino acid sequence set forth in SEQ ID NO: 107.
15. An anti-uPAR antibody or an antigen-binding fragment thereof, comprising a light chain variable region comprising:
   (a) a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6;
   (b) a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14;
   (c) a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22;
   (d) a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30;
   (e) a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40;
   (f) a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49;
   (g) a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58;
   (h) a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68;
   (i) a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77;
   (j) a CDR1 comprising the amino acid sequence set forth in SEQ ID NO: 84, a CDR2 comprising the amino acid sequence set forth in SEQ ID NO: 67, and a CDR3 comprising the amino acid sequence set forth in SEQ ID NO: 85;
   (k) a CDR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94; or
   (l) a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.
16. An anti-uPAR antibody or an antigen-binding fragment thereof, comprising:
   (a) a heavy chain variable region comprising a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; and a light chain variable region comprising a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6;
   (b) a heavy chain variable region comprising a CDR1 comprising the amino acid sequence set forth in SEQ ID NO: 11, a CDR2 comprising an amino acid sequence set forth in SEQ ID NO: 2, and a CDR3 comprising the amino acid sequence set forth in SEQ ID NO: 12; and a light chain variable region comprising a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14;
   (c) a heavy chain variable region comprising a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; and a light chain variable region comprising a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22;
   (d) a heavy chain variable region comprising a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28; and a light chain variable region comprising a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30;
   (e) a heavy chain variable region comprising a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; and a light chain variable region comprising a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40;
   (f) heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; and a light chain variable region comprising a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49;
   (g) heavy chain variable region comprising a CDR1 comprising the amino acid sequence set forth in SEQ ID NO: 54, a comprising the amino acid sequence set forth in SEQ ID NO: 55, and a CDR3 comprising the amino acid sequence set forth in SEQ ID NO: 56; and a light chain variable region comprising a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58;.
   (h) heavy chain variable region comprising a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68;
   (i) heavy chain variable region comprising a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; and a light chain variable region comprising a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77;
   (j) heavy chain variable region comprising a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83; and a light chain variable region comprising a CDR1 comprising the amino acid sequence set forth in SEQ ID NO: 84, a CDR2 comprising the amino acid sequence set forth in SEQ ID NO: 67, and a CDR3 comprising the amino acid sequence set forth in SEQ ID NO: 85;
   (k) heavy chain variable region comprising a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92; and a light chain variable region comprising a CDR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94; or
   (l) heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100.
17. The antibody or antigen-binding fragment thereof of any one of paragraphs 1-16, wherein the antibody or antigen-binding fragment thereof binds to a uPAR comprising the amino acid sequence set forth in SEQ ID NO: 116 or a fragment thereof.
18. The antibody or antigen-binding fragment thereof of any one of paragraphs 1-17, wherein the sequence of the antibody is in a light-heavy variable chain orientation (V_{L}-V_{H}).
19. The antibody or antigen-binding fragment thereof of any one of paragraphs 1-18, wherein the antibody comprises a human variable region framework region.
20. The antibody or antigen-binding fragment thereof of any one of paragraphs 1-19, which is a fully human or an antigen-binding fragment thereof.
21. The antibody or antigen-binding fragment thereof of any one of paragraphs 1-18, which is a chimeric antibody or an antigen-binding fragment thereof.
22. The antibody or antigen-binding fragment thereof of any one of paragraphs 1-18, which is a humanized antibody or an antigen-binding fragment thereof.
23. The antibody or antigen-binding fragment thereof of any one of paragraphs 1-22, wherein the antigen-binding fragment is a Fab, Fab', F(ab')₂, variable fragment (Fv), or single chain variable region (scFv).
24. The antibody or antigen-binding fragment thereof of paragraph 23, wherein the antigen antigen-binding fragment is an scFv.
25. The antibody or an antigen-binding fragment thereof of paragraph 24, wherein the scFv comprises the amino acid sequence set forth in SEQ ID NO: 9, SEQ ID NO: 17, SEQ ID NO: 25, SEQ ID NO: 33, SEQ ID NO: 43, SEQ ID NO: 52, SEQ ID NO: 61, SEQ ID NO: 71, SEQ ID NO: 80, SEQ ID NO: 88, SEQ ID NO: 97, or SEQ ID NO: 103.
26. An antibody or an antigen-binding fragment thereof, which cross-competes for binding to uPAR with an antibody or an antigen-binding fragment thereof of any one of paragraphs 1-25.
27. An antibody or an antigen-binding fragment thereof, which binds to the same epitope region on uPAR with an antibody or an antigen-binding fragment thereof of any one of paragraphs 1-25.
28. A composition comprising the antibody or antigen-binding fragment thereof of any one of paragraphs 1-27.
29. The composition of paragraph 28, which is a pharmaceutical composition that further comprises a pharmaceutically acceptable carrier.
30. An immunoconjugate comprising the antibody or antigen-binding fragment thereof of any one of paragraphs 1-27, linked to a therapeutic agent.
31. The immunoconjugate of paragraph 30, wherein the therapeutic agent is a drug, a cytotoxin, or a radioactive isotope.
32. A composition comprising the immunoconjugate of paragraph 30 or 31.
33. The composition of paragraph 32, which is a pharmaceutical composition that further comprises a pharmaceutically acceptable carrier.
34. A multi-specific molecule comprising the antibody or antigen-binding fragment thereof of any one of paragraphs 1-27, linked to one or more functional moieties.
35. The bispecific molecule of paragraph 34, wherein the one or more functional moieties have a different binding specificity than the antibody or antigen binding fragment thereof.
36. A composition comprising the multi-specific molecule of paragraph 34 or 35.
37. The composition of paragraph 36, which is a pharmaceutical composition that further comprises a pharmaceutically acceptable carrier.
38. A nucleic acid that encodes an antibody or antigen-binding fragment thereof of any one of paragraphs 1-27.
39. A vector comprising the nucleic acid molecule of paragraph 38.
40. A host cell comprising the vector of paragraph 39.
41. A method for detecting uPAR in a whole cell, a tissue, or a blood sample, comprising:
   contacting a cell, tissue or blood sample with the antibody or antigen-binding fragment thereof of any one of paragraphs 1-27, wherein the antibody or antigen-binding fragment thereof comprises a detectable label; and
   determining the amount of the labeled antibody or antigen-binding fragment thereof bound to the cell, tissue or blood sample by measuring the amount of detectable label associated with said cell or tissue, wherein the amount of bound antibody or antigen-binding fragment thereof indicates the amount of uPAR in the cell, tissue or blood sample.
42. A method of treating or ameliorating a disease or disorder in a subject, comprising administering to the subject the antibody or antigen-binding fragment thereof of any one of paragraphs 1-27, the immunoconjugate of paragraph 30 or 31, the multi-specific molecule of paragraph 34 or 35, or the composition of any one of paragraphs 28, 29, 32, 33, 36, and 37.
43. The method of paragraph 42, wherein the disease or disorder is selected from the group consisting of tumors, senescence-associated pathologies, and tissue decline associated with aging.
44. The method of paragraph 43, wherein the disease or disorder is a senescence-associated pathology.
45. The method of paragraph 44, wherein the senescence-associated pathology is selected from the group consisting of lung fibrosis, atherosclerosis, Alzheimer's disease, diabetes, osteoarthritis, liver fibrosis, chronic kidney disease, cardiac fibrosis, and Parkinson's disease.
46. The method of paragraph 42, wherein the disease or disorder is a tumor.
47. The method of paragraph 46, wherein the tumor is selected from the group consisting of breast cancer, endometrial cancer, ovarian cancer, colon cancer, rectal cancer, lung cancer, stomach cancer, prostate cancer, gastric cancer, renal cancer, pancreatic cancer, blood cancer, cervical cancer, head and neck cancer, liver cancer, urotherial cancer, melanoma, and brain cancer.
48. The method of paragraph 47, wherein the blood cancer is selected from the group consisting of acute lymphoblastic leukemia (ALL), chronic lymphocytic leukemia (CLL), acute myeloid leukemia (AML), myelofibrosis, polycythemia vera, myelodysplastic syndrome, and erythroleukemia.
49. The method of any one of paragraphs 46-48, wherein the tumor is cancer.
50. A method of increasing production of an immune-activating cytokine in response to a tumor cell in a subject, comprising administering to the subject the antibody or antigen-binding fragment thereof of any one of paragraphs 1-27, the immunoconjugate of paragraph 30 or 31, the multi-specific molecule of paragraph 34 or 35, or the composition of any one of paragraphs 28, 29, 32, 33, 36, and 37.
51. The method of paragraph 50, wherein the immune-activating cytokine is selected from the group consisting of granulocyte macrophage colony stimulating factor (GM-CSF), IFN-α, IFN-β, IFN- γ, TNF-α, IL-1 IL-2, IL-3, IL-6, IL-11, IL-7, IL-8, IL-12, IL-15, IL-21, interferon regulatory factor 7 (IRF7), CCL1, CCL2, CCL3, CCL5, CCL7, CCL8, CCL13, CCL16, CXCL1, CXCL3, CXCL5, CXCL9, CXCL10, and combinations thereof.
52. The method of any one of paragraphs 42-51, wherein the subject is a human.
53. A kit for treating or ameliorating a disease or disorder in a subject, and/or increasing production of an immune-activating cytokine in response to a tumor cell in a subject, comprising the antibody or antigen-binding fragment thereof of any one of paragraphs 1-27, the immunoconjugate of paragraph 30 or 31, the multi-specific molecule of paragraph 34 or 35, or the composition of any one of paragraphs 28, 29, 32, 33, 36, and 37.
54. The kit of paragraph 53, wherein the kit further comprises written instructions for using the antibody or antigen-binding fragment thereof, immunoconjugate, multi-specific molecule, or composition for treating or ameliorating a disease or disorder in a subject, and/or increasing production of an immune-activating cytokine in response to a tumor cell in a subject.

## Claims

1. An anti-uPAR antibody or an antigen-binding fragment thereof, comprising:
(a) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 99; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 100;
(b) a heavy chain variable region comprising a CDR1 comprising the amino acid sequence set forth in SEQ ID NO: 11, a CDR2 comprising an amino acid sequence set forth in SEQ ID NO: 2, and a CDR3 comprising the amino acid sequence set forth in SEQ ID NO: 12; and a light chain variable region comprising a CDR1 comprising the amino acid sequence set forth in SEQ ID NO: 13, a CDR2 comprising the amino acid sequence set forth in SEQ ID NO: 5, and a CDR3 comprising the amino acid sequence set forth in SEQ ID NO: 14;
(c) a heavy chain variable region comprising a CDR1 comprising the amino acid sequence set forth in SEQ ID NO: 19, a CDR2 comprising the amino acid sequence set forth in SEQ ID NO: 2, and a CDR3 comprising the amino acid sequence set forth in SEQ ID NO: 20; and a light chain variable region comprising a CDR1 comprising the amino acid sequence set forth in SEQ ID NO: 21, a CDR2 comprising the amino acid sequence set forth in SEQ ID NO: 5, and a CDR3 comprising the amino acid sequence set forth in SEQ ID NO: 22;
(d) a heavy chain variable region comprising a CDR1 comprising the amino acid sequence set forth in SEQ ID NO: 19, a CDR2 comprising the amino acid sequence set forth in SEQ ID NO: 27, and a CDR3 comprising the amino acid sequence set forth in SEQ ID NO: 28; and a light chain variable region comprising a CDR1 comprising the amino acid sequence set forth in SEQ ID NO: 29, a CDR2 comprising the amino acid sequence set forth in SEQ ID NO: 5, and a CDR3 comprising the amino acid sequence set forth in SEQ ID NO: 30;
(e) a heavy chain variable region comprising a CDR1 comprising the amino acid sequence set forth in SEQ ID NO: 35, a CDR2 comprising the amino acid sequence set forth in SEQ ID NO: 36, and a CDR3 comprising the amino acid sequence set forth in SEQ ID NO: 37; and a light chain variable region comprising a CDR1 comprising the amino acid sequence set forth in SEQ ID NO: 38, a CDR2 comprising the amino acid sequence set forth in SEQ ID NO: 39, and a CDR3 comprising the amino acid sequence set forth in SEQ ID NO: 40;
(f) a heavy chain variable region comprising a CDR1 comprising the amino acid sequence set forth in SEQ ID NO: 45, a CDR2 comprising the amino acid sequence set forth in SEQ ID NO: 46, and a CDR3 comprising the amino acid sequence set forth in SEQ ID NO: 47; and a light chain variable region comprising a CDR1 comprising the amino acid sequence set forth in SEQ ID NO: 48, a CDR2 comprising the amino acid sequence set forth in SEQ ID NO: 5, and a CDR3 comprising the amino acid sequence set forth in SEQ ID NO: 49;
(g) a heavy chain variable region comprising a CDR1 comprising the amino acid sequence set forth in SEQ ID NO: 54, a comprising the amino acid sequence set forth in SEQ ID NO: 55, and a CDR3 comprising the amino acid sequence set forth in SEQ ID NO: 56; and a light chain variable region comprising a CDR1 comprising the amino acid sequence set forth in SEQ ID NO: 57, a CDR2 comprising the amino acid sequence set forth in SEQ ID NO: 5, and a CDR3 comprising the amino acid sequence set forth in SEQ ID NO: 58;.
(h) a heavy chain variable region comprising a CDR1 comprising the amino acid sequence set forth in SEQ ID NO: 63, a CDR2 comprising the amino acid sequence set forth in SEQ ID NO: 64, and a CDR3 comprising the amino acid sequence set forth in SEQ ID NO: 65; and a light chain variable region comprising a CDR1 comprising the amino acid sequence set forth in SEQ ID NO: 66, a CDR2 comprising the amino acid sequence set forth in SEQ ID NO: 67, and a CDR3 comprising the amino acid sequence set forth in SEQ ID NO: 68;
(i) a heavy chain variable region comprising a CDR1 comprising the amino acid sequence set forth in SEQ ID NO: 73, a CDR2 comprising the amino acid sequence set forth in SEQ ID NO: 74, and a CDR3 comprising the amino acid sequence set forth in SEQ ID NO: 75; and a light chain variable region comprising a CDR1 comprising the amino acid sequence set forth in SEQ ID NO: 76, a CDR2 comprising the amino acid sequence set forth in SEQ ID NO: 67, and a CDR3 comprising the amino acid sequence set forth in SEQ ID NO: 77;
(j) a heavy chain variable region comprising a CDR1 comprising the amino acid sequence set forth in SEQ ID NO: 45, a CDR2 comprising the amino acid sequence set forth in SEQ ID NO: 82, and a CDR3 comprising the amino acid sequence set forth in SEQ ID NO: 83; and a light chain variable region comprising a CDR1 comprising the amino acid sequence set forth in SEQ ID NO: 84, a CDR2 comprising the amino acid sequence set forth in SEQ ID NO: 67, and a CDR3 comprising the amino acid sequence set forth in SEQ ID NO: 85;
(k) a heavy chain variable region comprising a CDR1 comprising the amino acid sequence set forth in SEQ ID NO: 90, a CDR2 comprising the amino acid sequence set forth in SEQ ID NO: 91, and a CDR3 comprising the amino acid sequence set forth in SEQ ID NO: 92; and a light chain variable region comprising a CDR1 comprising the amino acid sequence set forth in SEQ ID NO: 93, a CDR2 comprising the amino acid sequence set forth in SEQ ID NO: 67, and a CDR3 comprising the amino acid sequence set forth in SEQ ID NO: 94; or
(l) a heavy chain variable region comprising a CDR1 comprising the amino acid sequence set forth in SEQ ID NO: 1, a CDR2 comprising the amino acid sequence set forth in SEQ ID NO: 2, and a CDR3 comprising the amino acid sequence set forth in SEQ ID NO: 3; and a light chain variable region comprising a CDR1 comprising the amino acid sequence set forth in SEQ ID NO: 4, a CDR2 comprising the amino acid sequence set forth in SEQ ID NO: 5, and a CDR3 comprising the amino acid sequence set forth in SEQ ID NO: 6;.

2. The antibody or antigen-binding fragment thereof of claim 1, wherein:
(a) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 101, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 102;
(b) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 15, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 16;
(c) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 23, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 24;
(d) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 31, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 32;
(e) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 41, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 42;
(f) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 50, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 51;
(g) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 59, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 60;
(h) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 69, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 70;
(i) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 78, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 79;
(j) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 86, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 87;
(k) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 95, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 96; or
(l) the heavy chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO:7, and the light chain variable region comprises an amino acid sequence that is at least about 80%, at least about 85%, at least about 90%, at least about 95%, at least about 96%, at least about 97%, at least about 98%, at least about 99%, at least about 100% homologous or identical to the amino acid sequence set forth in SEQ ID NO: 8.

3. The antibody or antigen-binding fragment thereof of claim 1 or claim 2, wherein
(a) the heavy chain variable region comprises the amino acid sequence set forth in SEQ ID NO: 101, and the light chain variable region comprises the amino acid sequence set forth in SEQ ID NO: 102;
(b) the heavy chain variable region comprises the amino acid sequence set forth in SEQ ID NO: 15, and the light chain variable region comprises the amino acid sequence set forth in SEQ ID NO: 16;
(c) the heavy chain variable region comprises the amino acid sequence set forth in SEQ ID NO: 23, and the light chain variable region comprises the amino acid sequence set forth in SEQ ID NO: 24;
(d) the heavy chain variable region comprises the amino acid sequence set forth in SEQ ID NO: 31, and the light chain variable region comprises the amino acid sequence set forth in SEQ ID NO: 32;
(e) the heavy chain variable region comprises the amino acid sequence set forth in SEQ ID NO: 41, and the light chain variable region comprises the amino acid sequence set forth in SEQ ID NO: 42;
(f) the heavy chain variable region comprises the amino acid sequence set forth in SEQ ID NO: 50, and the light chain variable region comprises the amino acid sequence set forth in SEQ ID NO: 51;
(g) the heavy chain variable region comprises the amino acid sequence set forth in SEQ ID NO: 59, and the light chain variable region comprises the amino acid sequence set forth in SEQ ID NO: 60;
(h) the heavy chain variable region comprises the amino acid sequence set forth in SEQ ID NO: 69, and the light chain variable region comprises the amino acid sequence set forth in SEQ ID NO: 70;
(i) the heavy chain variable region comprises the amino acid sequence set forth in SEQ ID NO: 78, and the light chain variable region comprises the amino acid sequence set forth in SEQ ID NO: 79;
(j) the heavy chain variable region comprises the amino acid sequence set forth in SEQ ID NO: 86, and the light chain variable region comprises the amino acid sequence set forth in SEQ ID NO: 87;
(k) the heavy chain variable region comprises the amino acid sequence set forth in SEQ ID NO: 95, and the light chain variable region comprises the amino acid sequence set forth in SEQ ID NO: 96; or
(l) heavy chain variable region comprises the amino acid sequence set forth in SEQ ID NO: 7, and the light chain variable region comprises the amino acid sequence set forth in SEQ ID NO: 8.

4. The antibody or antigen-binding fragment thereof of any one of claims 1-3, wherein:
(a) the sequence of the antibody is in a light-heavy variable chain orientation (VL-VH); and/or
(b) the antibody comprises a human variable region framework region.

5. The antibody or antigen-binding fragment thereof of any one of claims 1-4, which is a fully human antibody, a humanized antibody, or a chimeric antibody, or an antigen-binding fragment thereof;
optionally wherein, the antigen-binding fragment is a Fab, Fab', F(ab')2, variable fragment (Fv), or single chain variable region (scFv); and
optionally wherein, the scFv comprises the amino acid sequence set forth in SEQ ID NO:103, SEQ ID NO: 17, SEQ ID NO: 25, SEQ ID NO: 33, SEQ ID NO: 43, SEQ ID NO: 52, SEQ ID NO: 61, SEQ ID NO: 71, SEQ ID NO: 80, SEQ ID NO: 88, SEQ ID NO: 97, or SEQ ID NO: 9.

6. An antibody or an antigen-binding fragment thereof, which:
(a) cross-competes for binding to uPAR with an antibody or an antigen-binding fragment thereof of any one of claims 1-5; and/or
(b) binds to the same epitope region on uPAR with an antibody or an antigen-binding fragment thereof of any one of claims 1-5.

7. An immunoconjugate comprising the antibody or antigen-binding fragment thereof of any one of claims 1-6, linked to a therapeutic agent, optionally wherein the therapeutic agent is a drug, a cytotoxin, or a radioactive isotope.

8. A multi-specific molecule comprising the antibody or antigen-binding fragment thereof of any one of claims 1-6, linked to one or more functional moieties; optionally wherein, the one or more functional moieties have a different binding specificity than the antibody or antigen binding fragment thereof.

9. A composition comprising:
(a) the antibody or antigen-binding fragment thereof of any one of claims 1-6;
(b) the immunoconjugate of claim 7; or
(c) the multi-specific molecule of claim 8;
optionally wherein the composition (a)-(c) is a pharmaceutical composition that further comprises a pharmaceutically acceptable carrier.

10. A host cell comprising:
(a) a nucleic acid encoding an antibody or antigen-binding fragment thereof of any one of claims 1-6; or
(b) a vector comprising a nucleic acid encoding an antibody or antigen-binding fragment thereof of any one of claims 1-6.

11. A method for detecting uPAR in a whole cell, a tissue, or a blood sample, comprising:
contacting a cell, tissue or blood sample with the antibody or antigen-binding fragment thereof of any one of claims 1-6, wherein the antibody or antigen-binding fragment thereof comprises a detectable label; and
determining the amount of the labeled antibody or antigen-binding fragment thereof bound to the cell, tissue, or blood sample by measuring the amount of detectable label associated with said cell or tissue, wherein the amount of bound antibody or antigen-binding fragment thereof indicates the amount of uPAR in the cell, tissue, or blood sample.

12. A method of treating or ameliorating a disease or disorder in a subject, comprising administering to the subject the antibody or antigen-binding fragment thereof of any one of claims 1-6, the immunoconjugate of claim 7, the multi-specific molecule of claim 8, or the composition of claim 9; optionally wherein the disease or disorder is selected from the group consisting of tumors, senescence-associated pathologies, and tissue decline associated with aging.

13. The method of claim 12, wherein:
(a) the disease or disorder is a senescence-associated pathology; optionally wherein, the senescence-associated pathology is selected from the group consisting of lung fibrosis, atherosclerosis, Alzheimer's disease, diabetes, osteoarthritis, liver fibrosis, chronic kidney disease, cardiac fibrosis, and Parkinson's disease;
(b) the disease or disorder is a tumor; optionally wherein, the tumor is selected from the group consisting of breast cancer, endometrial cancer, ovarian cancer, colon cancer, rectal cancer, lung cancer, stomach cancer, prostate cancer, gastric cancer, renal cancer, pancreatic cancer, blood cancer, cervical cancer, head and neck cancer, liver cancer, urothelial cancer, melanoma, and brain cancer; or
(c) the disease or disorder is a blood cancer; optionally wherein, the blood cancer is selected from the group consisting of acute lymphoblastic leukemia (ALL), chronic lymphocytic leukemia (CLL), acute myeloid leukemia (AML), myelofibrosis, polycythemia vera, myelodysplastic syndrome, and erythroleukemia.

14. A method of increasing production of an immune-activating cytokine in response to a tumor cell in a subject, comprising administering to the subject the antibody or antigen-binding fragment thereof of any one of claims 1-6, the immunoconjugate of claim 7, the multi-specific molecule of claim 8, or the composition of claim 9; optionally wherein the immune-activating cytokine is selected from the group consisting of granulocyte macrophage colony stimulating factor GM-CSF), IFN-α, IFN-β, IFN-γ, TNF-α, IL-1 IL-2, IL-3, IL-6, IL-11, IL-7, IL-8, IL-12, IL-15, IL-21, interferon regulatory factor 7 (IRF7), CCL1, CCL2, CCL3, CCL5, CCL7, CCL8, CCL13, CCL16, CXCL1, CXCL3, CXCL5, CXCL9, CXCL10, and combinations thereof.

15. A kit for treating or ameliorating a disease or disorder in a subject, and/or increasing production of an immune-activating cytokine in response to a tumor cell in a subject, comprising the antibody or antigen-binding fragment thereof of any one of claims 1-6, the immunoconjugate of claim 7, the multi-specific molecule of claim 8, or the composition of claim 9; optionally wherein the kit further comprises written instructions for using the antibody or antigen-binding fragment thereof, immunoconjugate, multi-specific molecule, or composition for treating or ameliorating a disease or disorder in a subject, and/or increasing production of an immune-activating cytokine in response to a tumor cell in a subject.
